# EUROPEAN PATENT SPECIFICATION

(11) **EP 0 397 753 B1**
(45) Date of publication and mention of the grant of the patent: **12.06.1996**
(21) Application number: 89902001.0
(22) Date of filing: 27.01.1989
(51) Int. Cl.: C12Q 1/68, C12N 15/00, C07H 21/02, C07H 21/04, C07G 17/00

(54) **DETERMINATION OF GENETIC SEX IN RUMINANTS USING Y-CHROMOSOME-SPECIFIC POLYNUCLEOTIDES**
BESTIMMUNG DES GESCHLECHTS BEI WIEDERKÄUERN UNTER VERWENDUNG VON GAMMA-CHROMOSOM-SPEZIFISCHEN POLYNUKLEOTIDEN
DETERMINATION DU SEXE GENETIQUE CHEZ LES RUMINANTS AU MOYEN DE POLYNUCLEOTIDES SPECIFIQUES AUX CHROMOSOMES Y

(30) Priority: 29.01.1988 AU 6476/88
(43) Date of publication of application: 22.11.1990
(73) Proprietor: ADVANCED RIVERINA HOLDINGS LIMITED, Table Top, NSW 2640 (AU)
(72) Inventor: REED, Kenneth, Clifford, Monash, ACT 2904 (AU); LORD, Eric, Arthur, North Lyneham, ACT 2602 (AU); MATTHAEI, Klaus, Ingo, Scullin, ACT 2614 (AU); MANN, David, Andrew, Higgins, ACT 2615 (AU); BEATON, Sandra, MacGregor, ACT 2615 (AU); HERR, Charles, Marvin, Tabletop, NSW 2640 (AU); MATTHEWS, Margaret, Ellen, Reid, ACT 2601 (AU)
(74) Representative: Woods, Geoffrey Corlett
(86) International application number: AU8900029
(87) International publication number: WO8907154

(56) References cited:
- WO-A-86/00342
- WO-A-87/05027
- WO-A-88/01300
- AU-A- 8 432 150
- AU-A- 8 659 561
- AU-A- 8 769 203
- AU-A- 8 774 532
- AU-A- 8 777 886
- US-A- 4 511 661

## Description

The present invention relates to determination of the primary (i.e. genetic) sex of individuals and of samples of cells removed from individuals, and is particularly concerned with ruminant sex determination using polynucleotides that are associated specifically with the Y chromosome.

(Note: References cited herein are collected at the end of the specification.)

The capacity to determine the sex of an embryo or a foetus is becoming increasingly advantageous, particularly in light of advances in the area of reproductive biology such as embryo transfer. In the dairy and beef cattle industry alone, some 50,000 embryo transplants were reported to have been carried out in 1985. Given the predisposition of the dairy industry to female progeny, it would be most advantageous if embryos could be routinely sexed in vitro prior to transfer into a maternal host. The availability of sexed embryos would allow dairy producers to select replacement progeny for their stock from embryos which possessed desirable traits, such as increased milk production and mothering ability. Similarly, in the sheep and goat industries, the availability of sexed embryos would enable producers to select the most desirable progeny for their stock.

The ability to determine the sex of an embryo or foetus in vivo is also important. In "conventional" pregnancies which do not involve embryo transfer, but rather arise via artificial insemination or natural insemination, the early determination of sex of an embryo or foetus would allow a producer to terminate a pregnancy if an embryo or foetus of the desired sex was not obtained.

The primary sex of a mammal is determined by the presence or absence of the entire Y-chromosome or a functional portion thereof. Gene(s) present on the Y-chromosome are responsible for the formation of the testis and the development of the male phenotype. The primary sex of an individual mammal is therefore dependent upon whether or not its genome contains certain DNA sequences, specifically those sequences comprising that part of the Y-chromosome which encode gene(s) responsible for testis determination.

The sex or presumptive sex of an individual mammal can therefore be determined by analysis for Y-chromosome specific genes in the DNA of the animal. Alternatively, sex can be determined by analysis for unrelated but genetically linked sequences that are associated specifically with the Y-chromosome. In order to minimise possible errors due to infrequent genetic recombination events, such analyses are best made for sequences which are closely linked to the testis-determining gene(s).

A number of investigators have identified DNA sequences which hybridize preferentially or exclusively to male DNA (1,2). These DNA sequences, however, have not been functionally characterized. Furthermore, it is unknown whether these sequences are capable of hybridization to non-human species.

International Patent Publication WO-A-86/07095 and Australian Patent Application No. 59561/86 disclose bovine DNA probes which hybridize preferentially to male DNA. These DNA sequences are stated to be useful as hybridization probes for sexing in embryos and foetuses. A particular disadvantage associated with the DNA sequences described in these patent specifications is that they are species' specific, i.e. bovine specific, and do not hybridize to DNA from other ruminant animals such as sheep or goats. The species specificity of these sequences therefore limits their usefulness as a general reagent for determining the sex of foetuses or embryos of ruminant animals.

Australian specification AU-B-69203/87 discloses a 7 kilobase EcoRI sequence which also is bovine specific.

International Patent Application No. PCT/AU87/00254 filed August 11th, 1987 and published as WO88/01300, with a claim to priority from original applications filed on August 12th, 1986, discloses a 307 base paid nucleic acid sequence designated BRY.1 comprising Y-chromosome-specific DNA which is capable of hybridizing, i.e. forming stable heterologous hybrids, with male bovine- and ovine-derived DNA but not with DNA isolated from female animals.

The DNA sequences of the present invention were discovered through their association with the BRY.1 sequence in the DNA of male ruminants. Certain of these sequences are more efficacious than BRY.1 for determining the genetic sex of ruminant cells because they exist in much higher copy number, are present in multiple copies in males (up to 500 copies per cell), but not the females, of all domestic ruminant species, i.e. cattle, sheep and goats, and show stronger sequence similarity between individual elements that have been sequenced.

The present invention arises from the discovery of specific Y-chromosomal DNA sequences, all or part of which, are found in all mammals so far examined. In particular, all or a large part of these sequences are conserved among ruminants and have been found to be repeated in the Y-chromosome of all ruminant animals studied to date. The conservation of these DNA sequences throughout the Order Mammalia, the demonstration that one of these sequences (called OY11.1) encodes all or part of a structural gene that is expressed in ruminant testes, and the exclusive association of repeating units of these sequences with the Y-chromosome of various ruminant species (which constitutes evidence for suppression of genetic recombination between these sequence elements and a gene or genes associated with testis-determination and/or male fertility) implies their close linkage on the Y-chromosome to DNA sequences essential to the differentiation and development of fertile males.

According to one aspect of the present invention, there is provided nucleic acid isolates capable of hybridizing only to Y-chromosome specific DNA species of cattle, sheep, goats, and other ruminants. The nucleic acid isolates correspond to all or part of a DNA sequence found on the Y-chromosome of at least one of bovine, ovine and caprine animals.

In particular, there are provided and defined a number of DNA isolates from male sheep that are capable of hybridizing only to samples of nucleic acid of cattle, sheep, goats which contain Y-chromosomal DNA sequences in the form of one or both strands of the ovine sequences referred to herein as OY1.1, OY4.1, OY4.2, OY9.1, OY9.2, OY9.5 and OY11.1; of the bovine sequences referred to herein as BRY.4a, BRY.4b, BRY.4c, BRY.4d, BRY.4e, BRY4a(d) and BRY.4c(e); the goat sequences referred to herein as GRY.1a, GRY.1a(a), GRY.1b, and GRY.1b(a), and the bovine sequence referred to herein as BCY.11a.

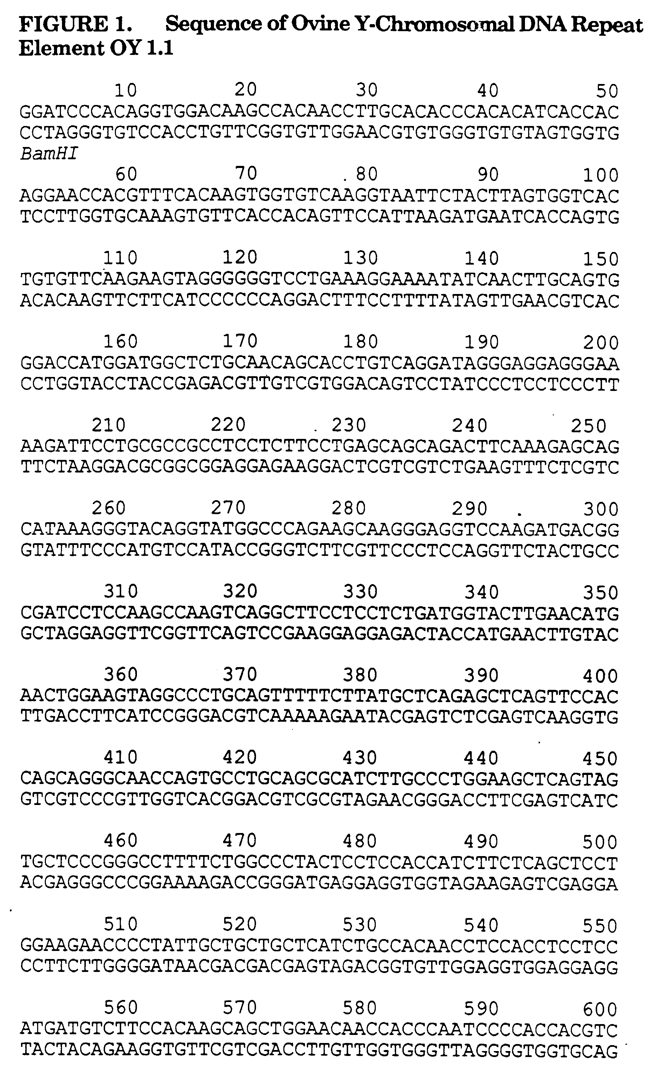

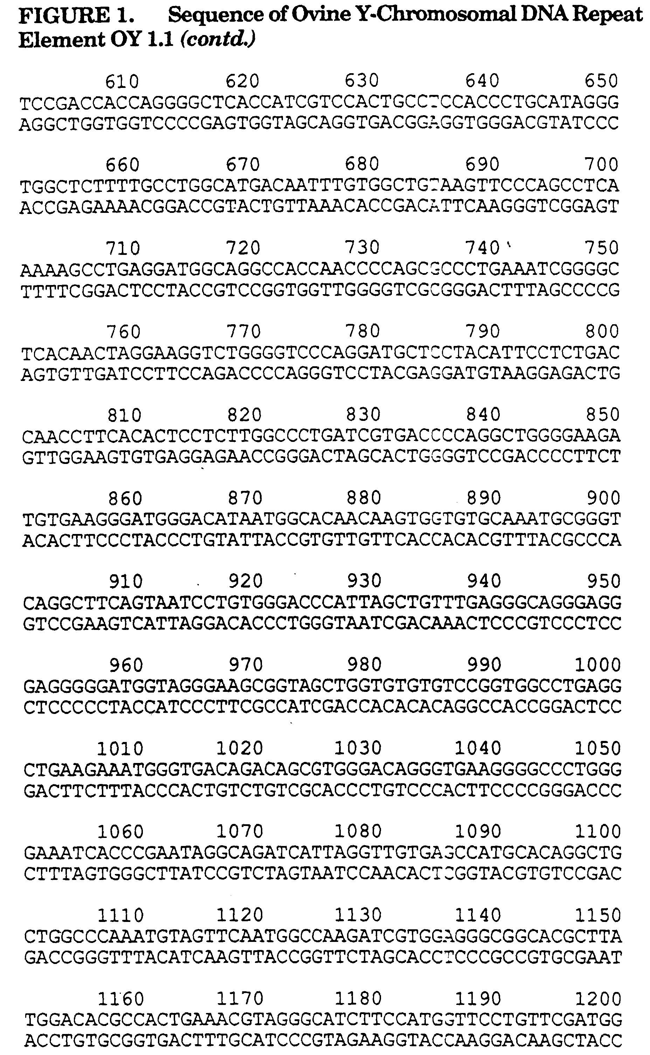

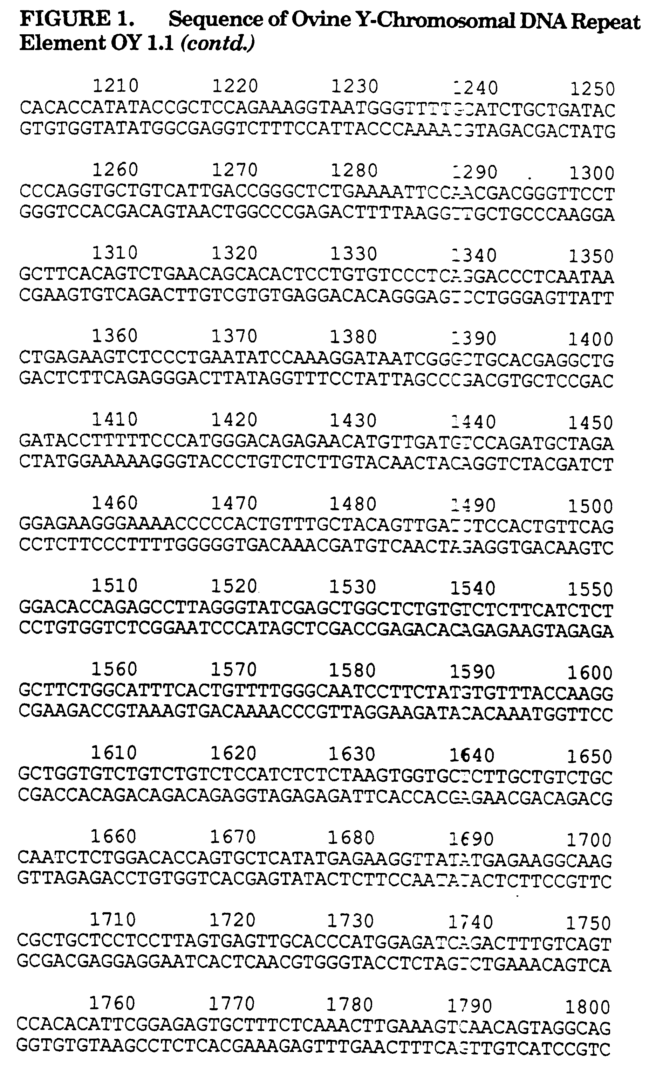

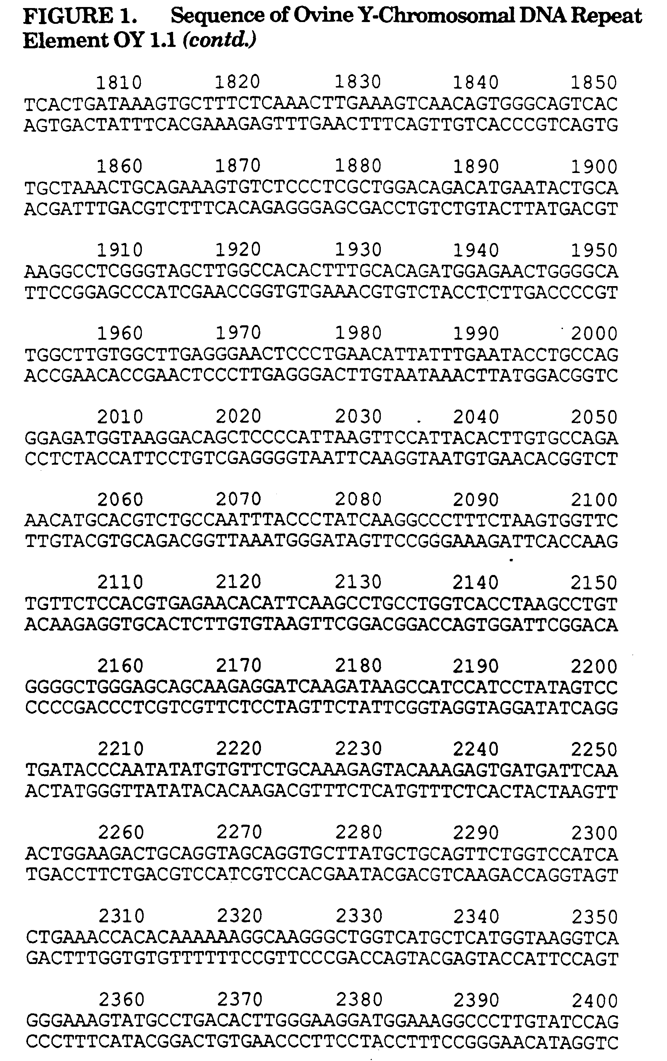

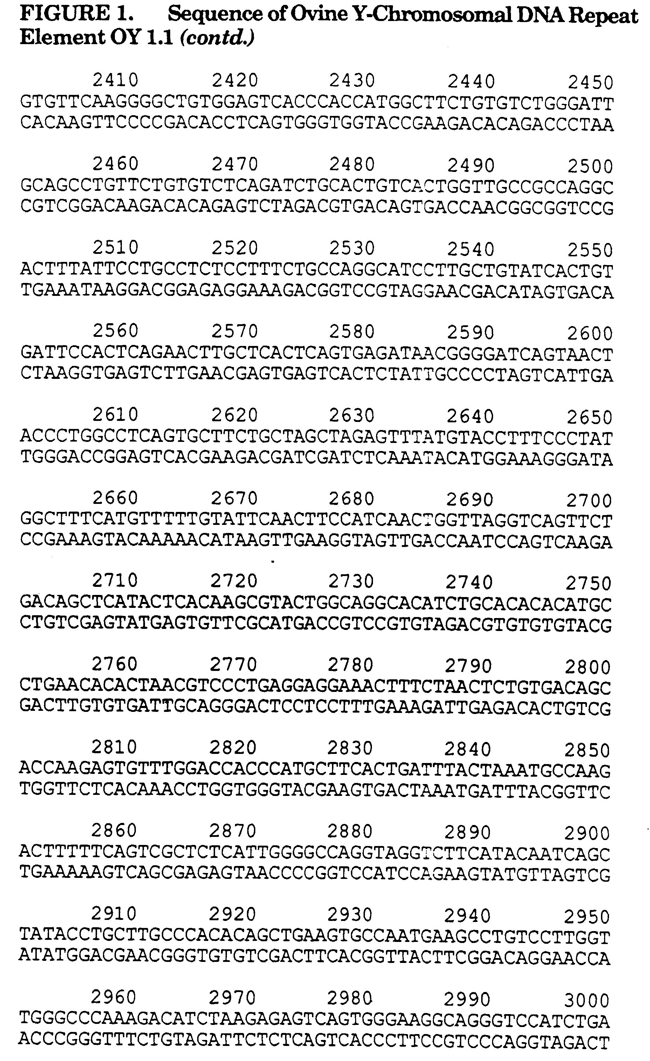

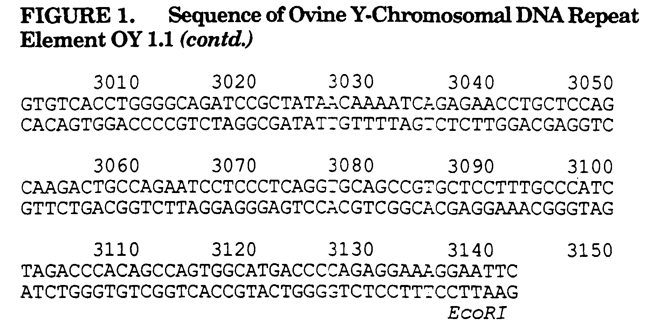

The OY1.1 DNA sequence is shown in Figure 1. This sequence comprises 3,142 base pairs of nucleotides, with those numbered 2,161-2,475 inclusive being similar to the 307 base pairs of Y-specific DNA sequence BRY.1 to the extent that it can form stable heterologous hybrids with BRY.1 under standard conditions of high-stringency DNA/DNA hybridization and its determined sequence of bases is similar to that of BRY.1. OY1.1 is a 3,142 base pair BamHI-EcoRI restriction fragment that was cloned into plasmids pTZ18u and pTZ20u (trademark Bio-Rad) from a recombinant bacteriophage (λOGY1) containing approximately 17200 base pairs of ram genomic DNA. Bacteriophage λOGY1 was identified and isolated from an unamplified library of ram genomic DNA by virtue of its hybridization to a 3,500 base pair (approx.) fragment of bovine genomic DNA containing BRY.1 DNA, designated BRY.1 (BRY.S is a BamHI fragment contained within a recombinant bacteriophage isolated from an amplified library of bull genomic DNA: the specific recombinant was isolated by virtue of its hybridisation with BRY.1, as was the BRY.S fragment itself). The restriction fragment OY1.1 was sub-cloned in consequence of its being found to hybridize with BRY.1. It was subsequently shown by hybridization to Southern blots of genomic DNA from individual male and female mammals to be generally conserved, male-specific and repeated in ruminants.

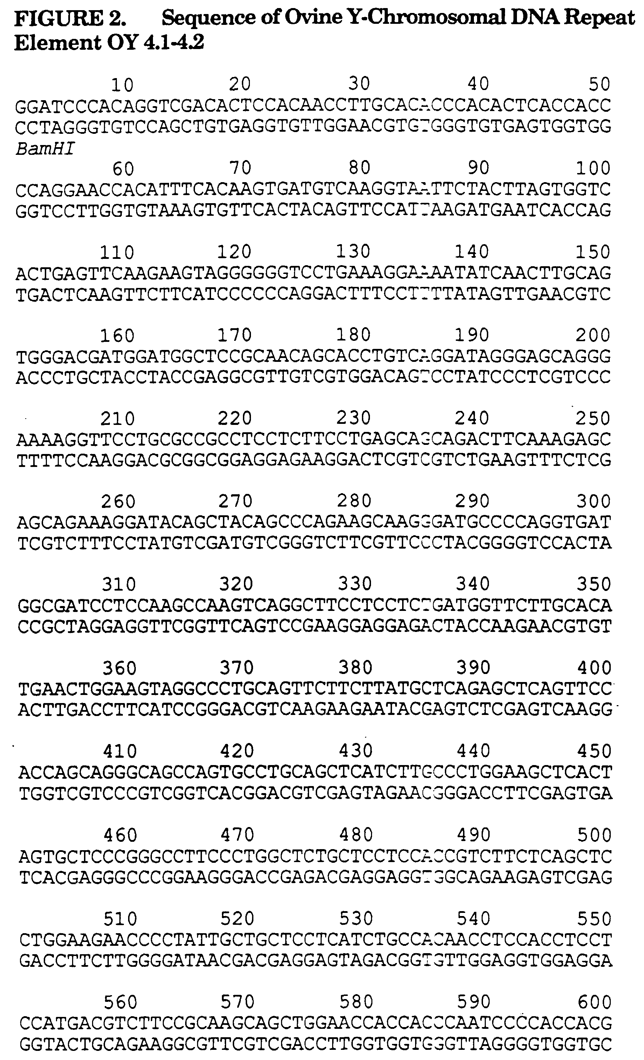

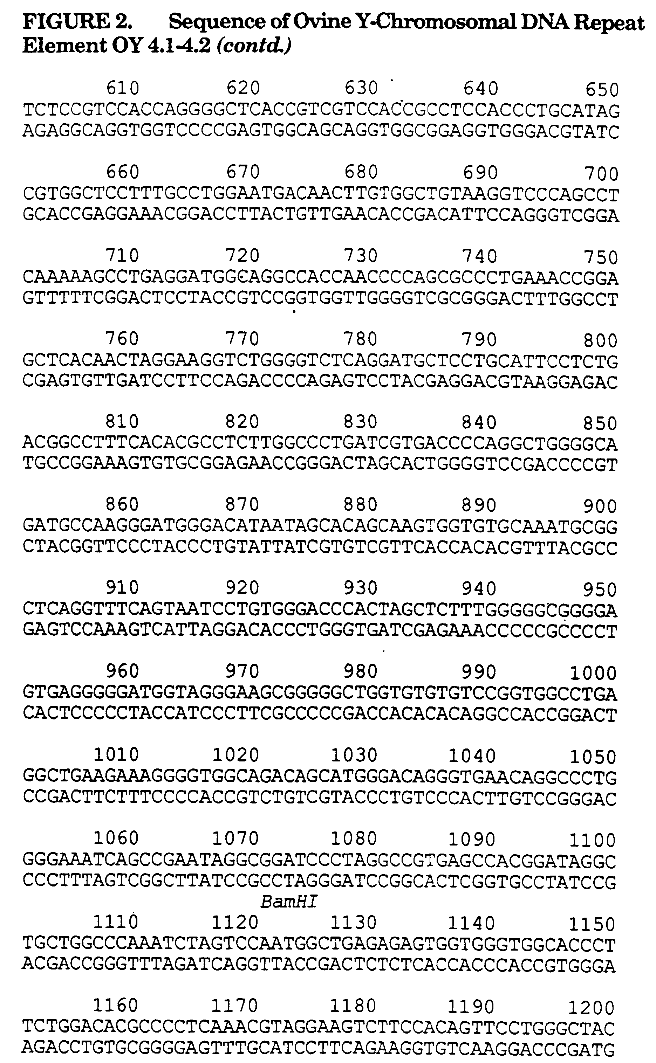

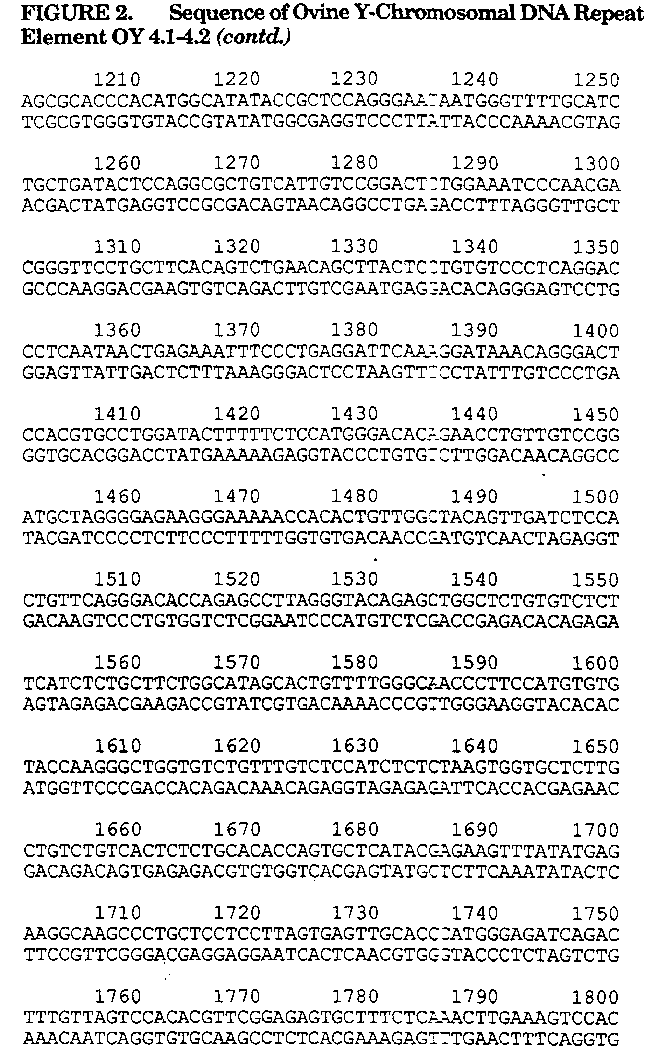

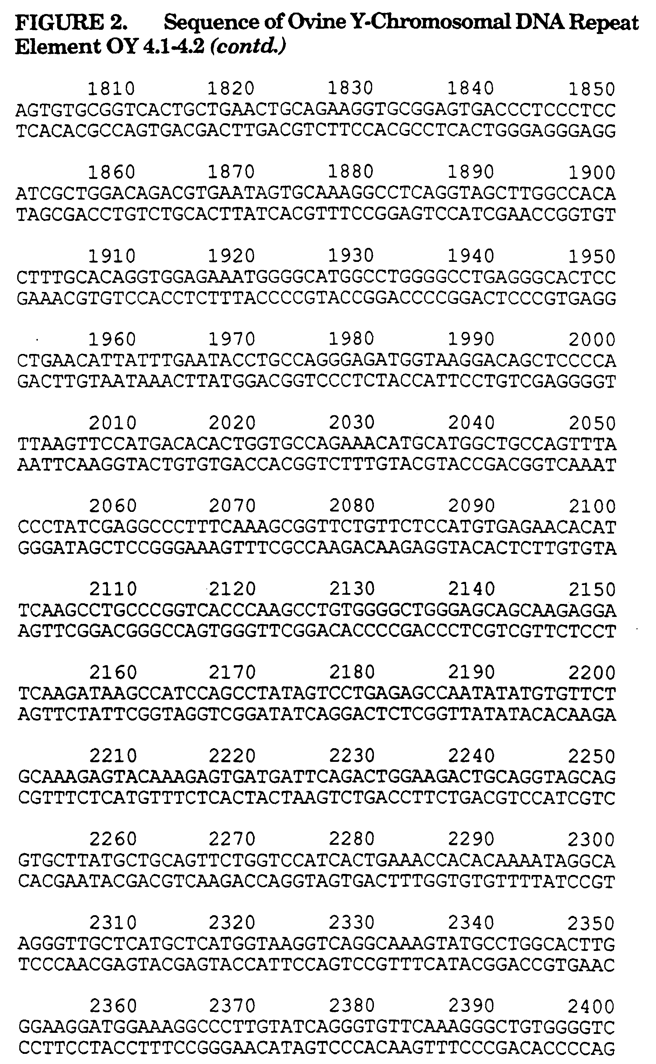

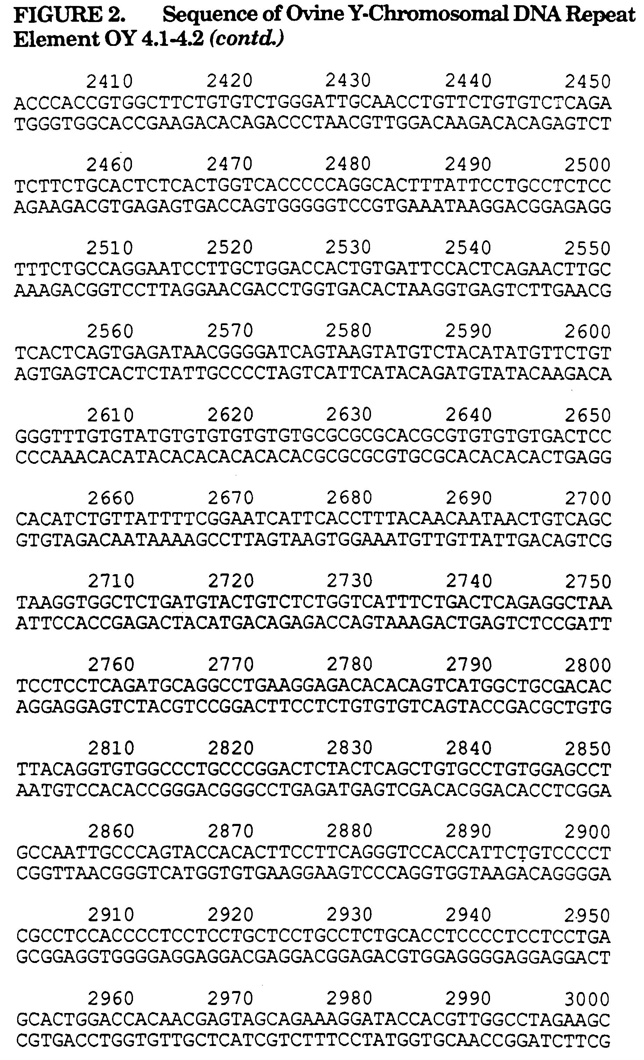

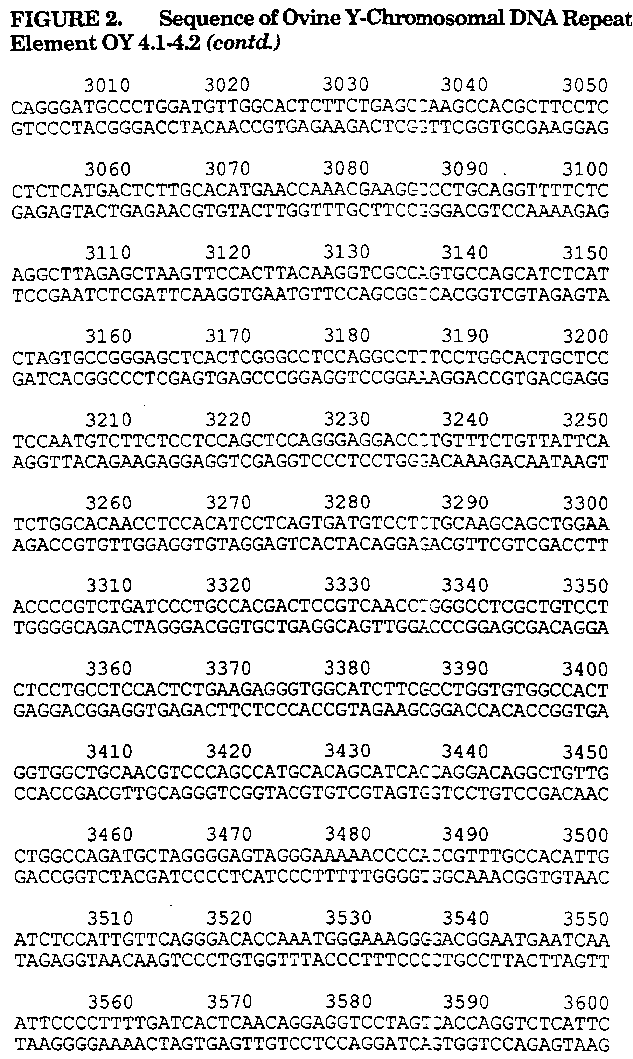

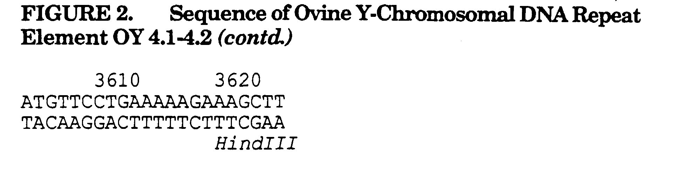

OY4.1 (Figure 2) is a 2,552 base pair HindIII-BamHI restriction fragment that was cloned from a recombinant bacteriophage (λOGY4) containing approximately 12,900 base pairs of ram genomic DNA. Bacteriophage λOGY4 was identified and isolated from the unamplified library of ram genomic DNA by virtue of its hybridization to BRY.S. The restriction fragment OY4.1 was sub-cloned in consequence of its being found to hybridize with BRY.1. It was subsequently shown by hybridization to Southern blots of genomic DNA from individual male and female mammals to be generally conserved, male-specific and repeated in ruminants. The restriction fragment was blunt-ended with the large (Klenow) fragment of E. coli DNA polymerase I and cloned into the SmaI site of plasmid pTZ18u.

OY4.2 (Figure 2) is a 1,076 base pair BamHI-BamHI restriction fragment that was cloned from the recombinant bacteriophage λOGY4. The restriction fragment OY4.2 was sub-cloned in consequence of its being adjacent to OY4.1 and being found to hybridize with BRY.S but not with BRY.1. The restriction fragment was blunt-ended with the large (Klenow) fragment of E. coli DNA polymerase I and cloned into the SmaI site of plasmid pTZ18u.

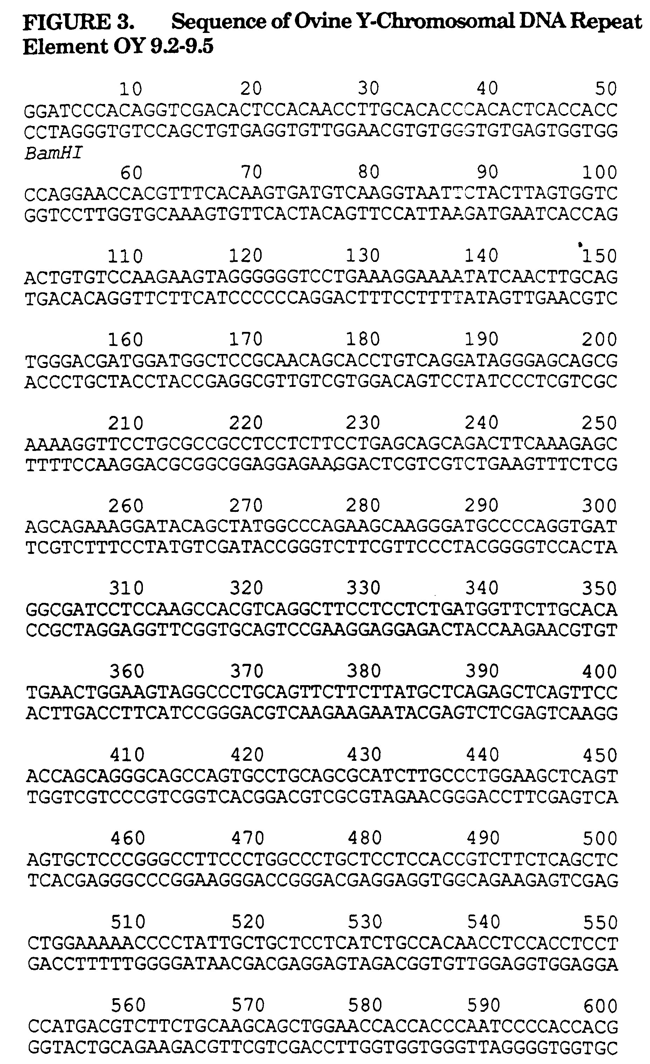

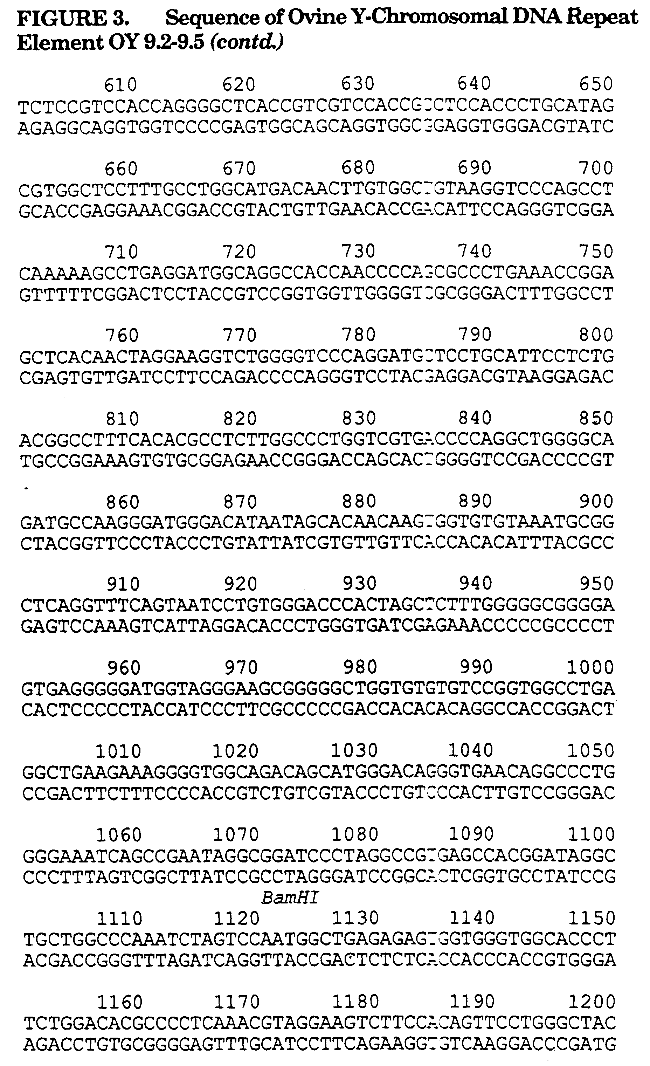

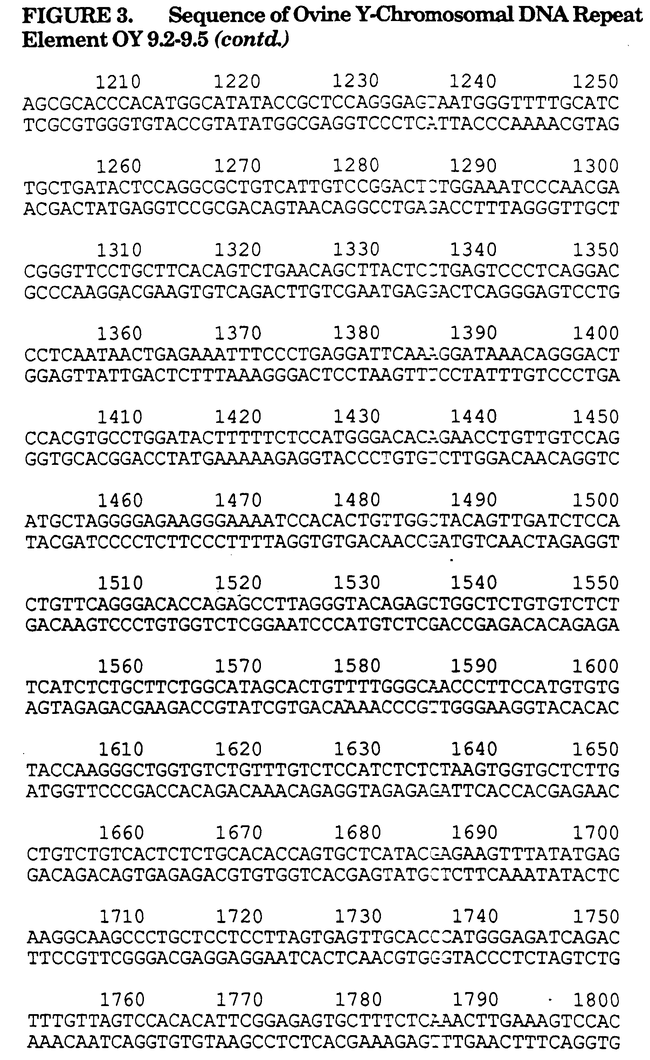

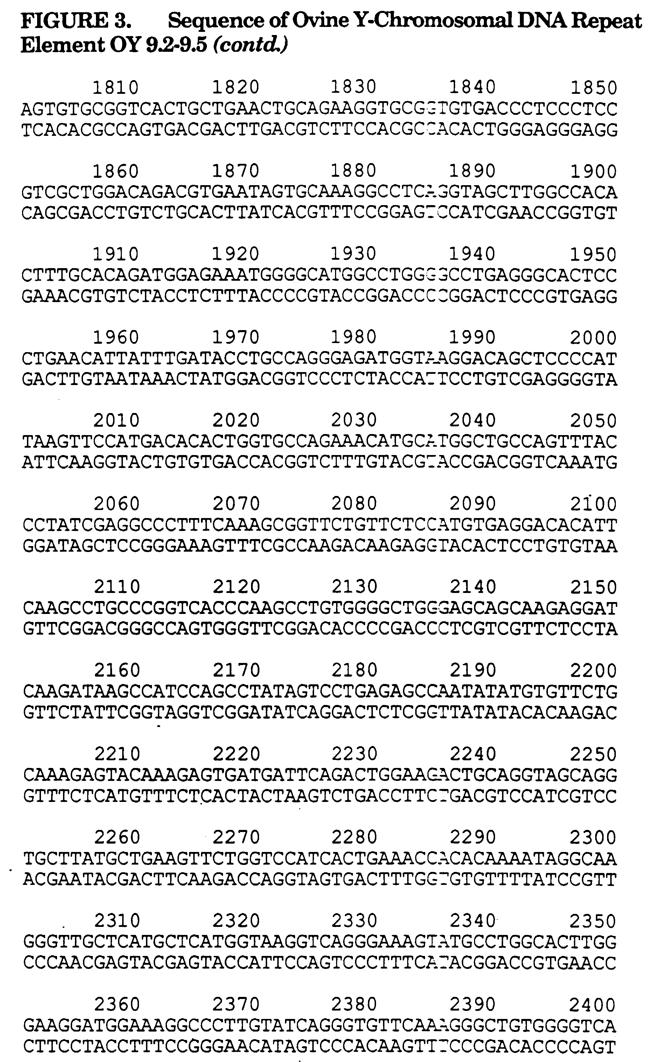

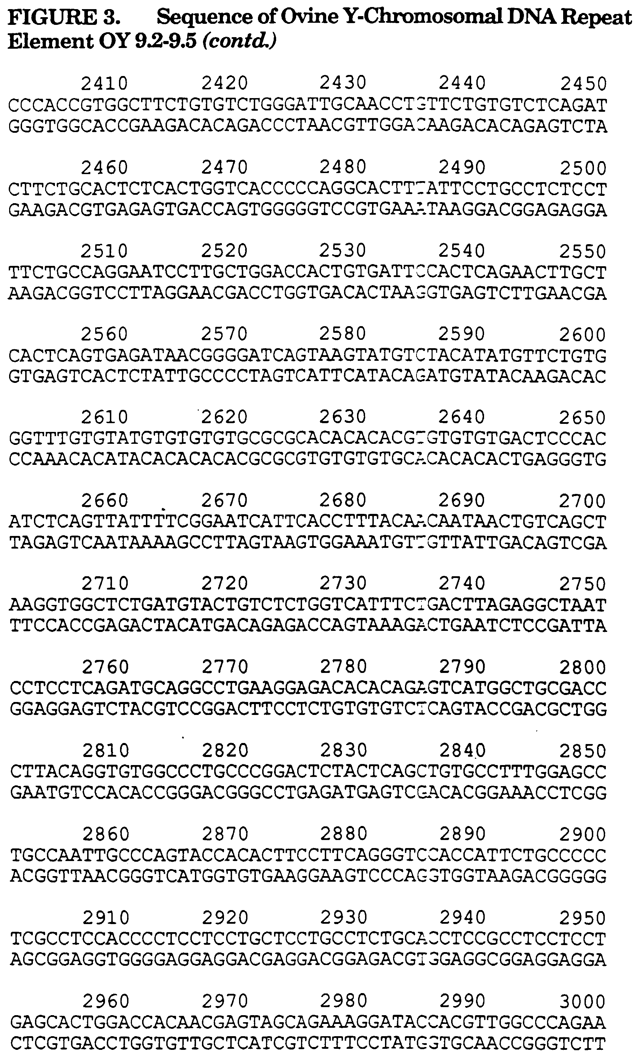

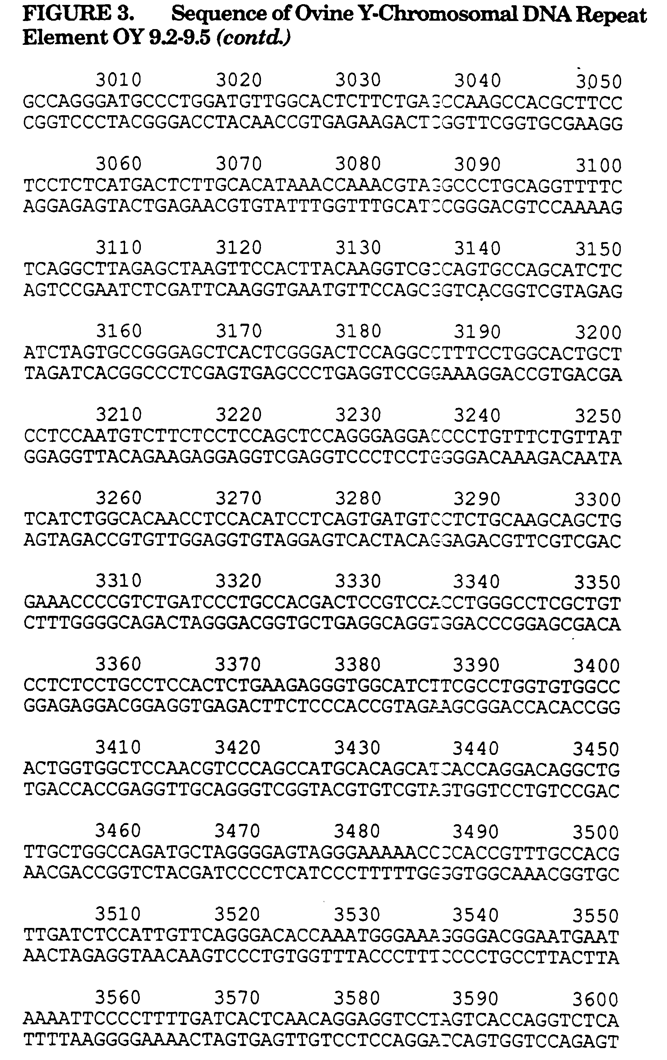

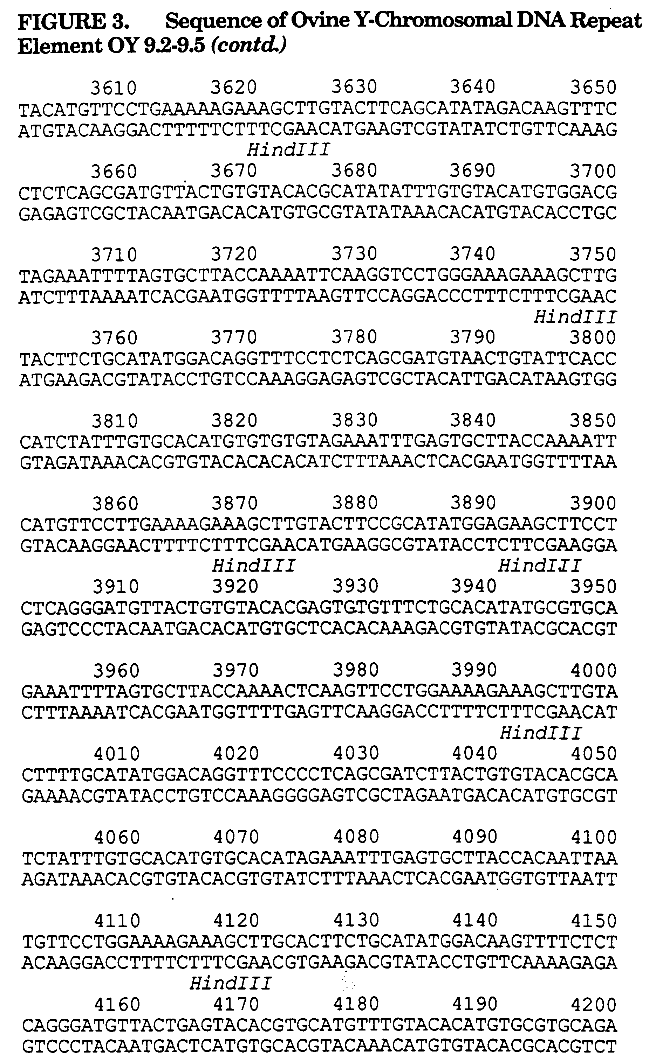

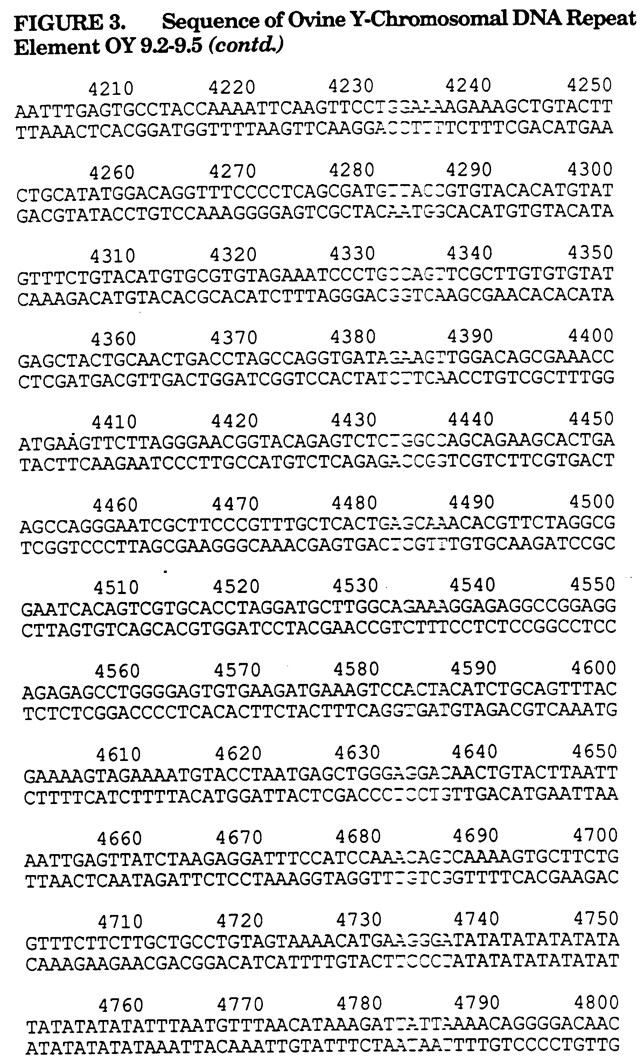

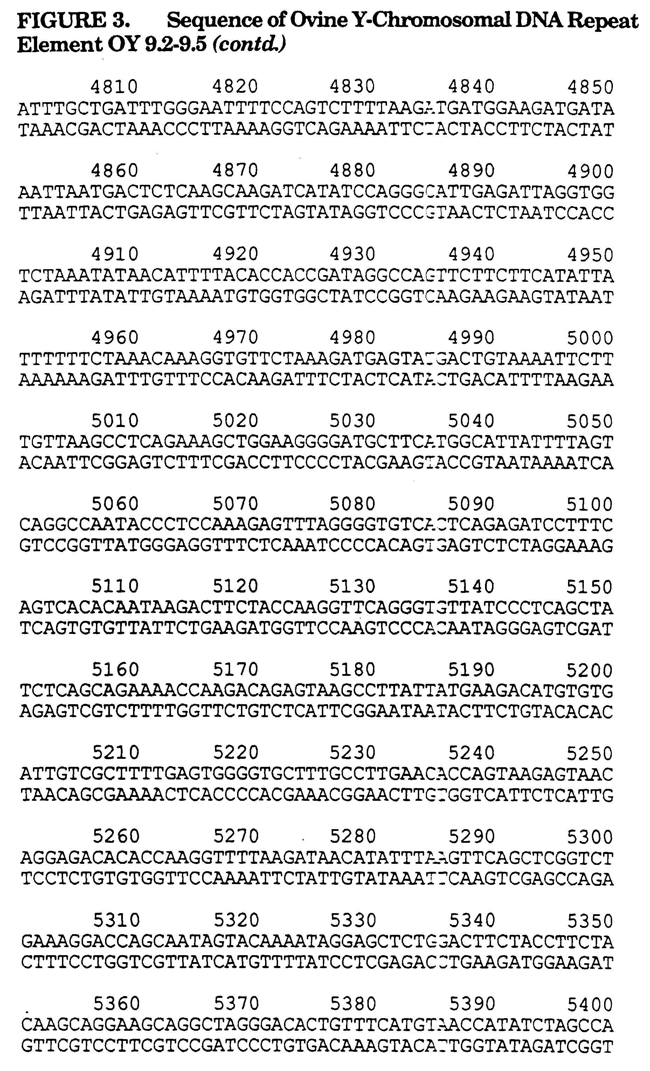

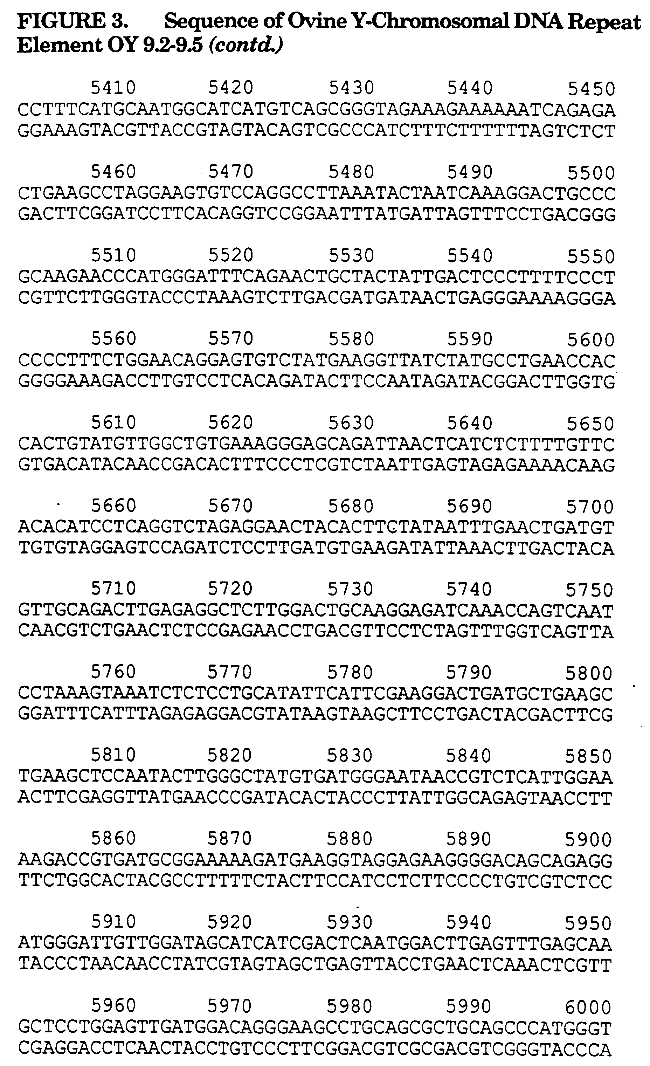

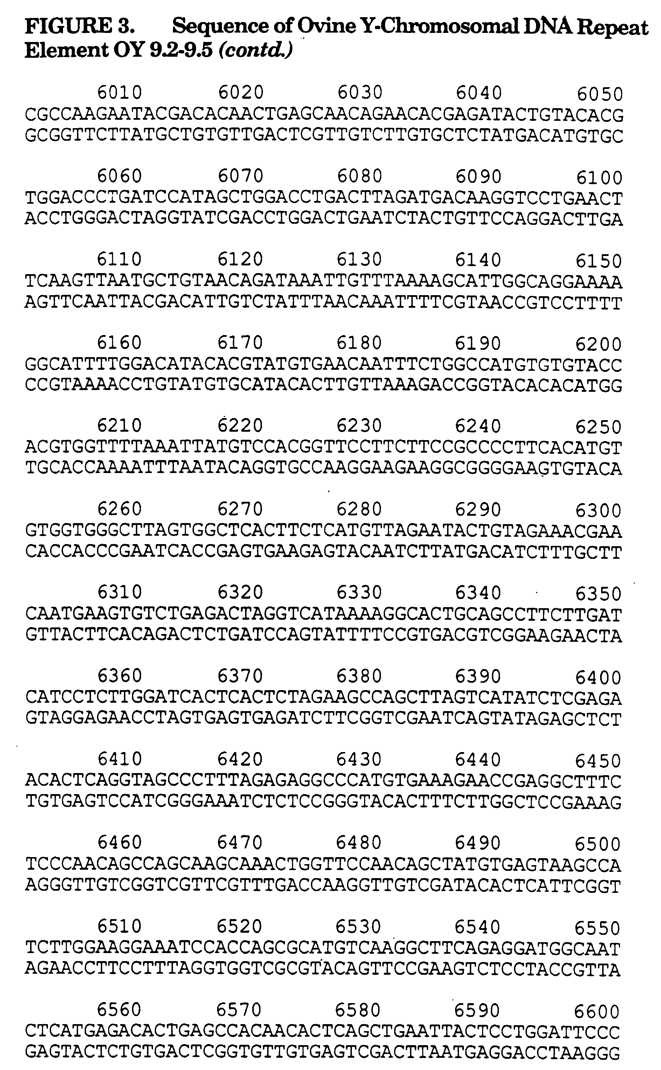

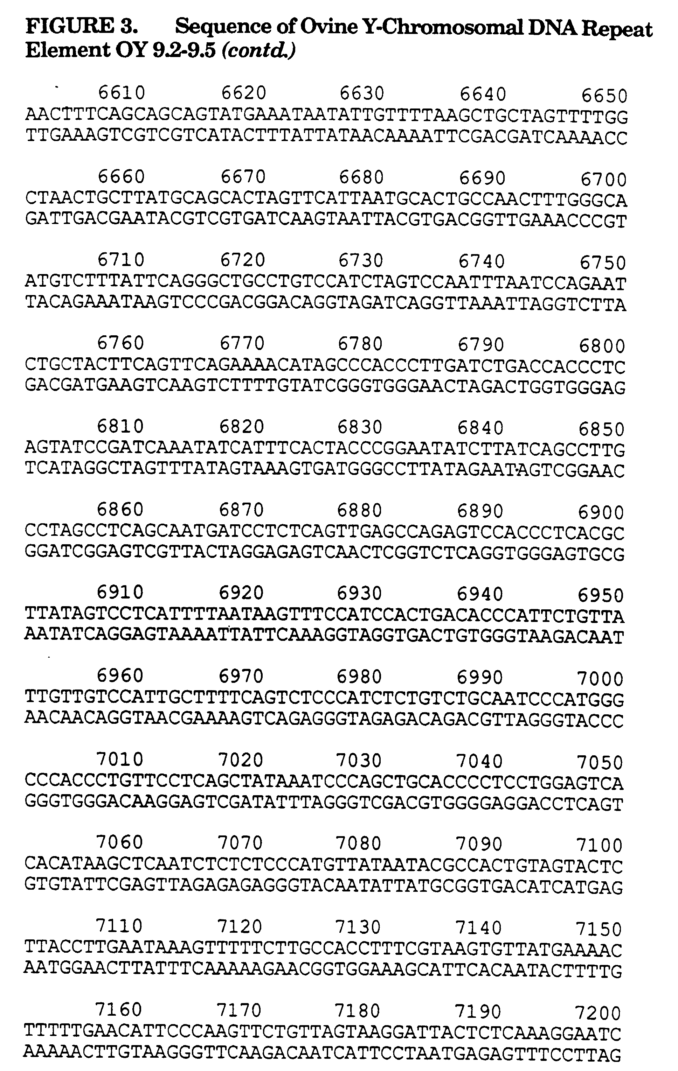

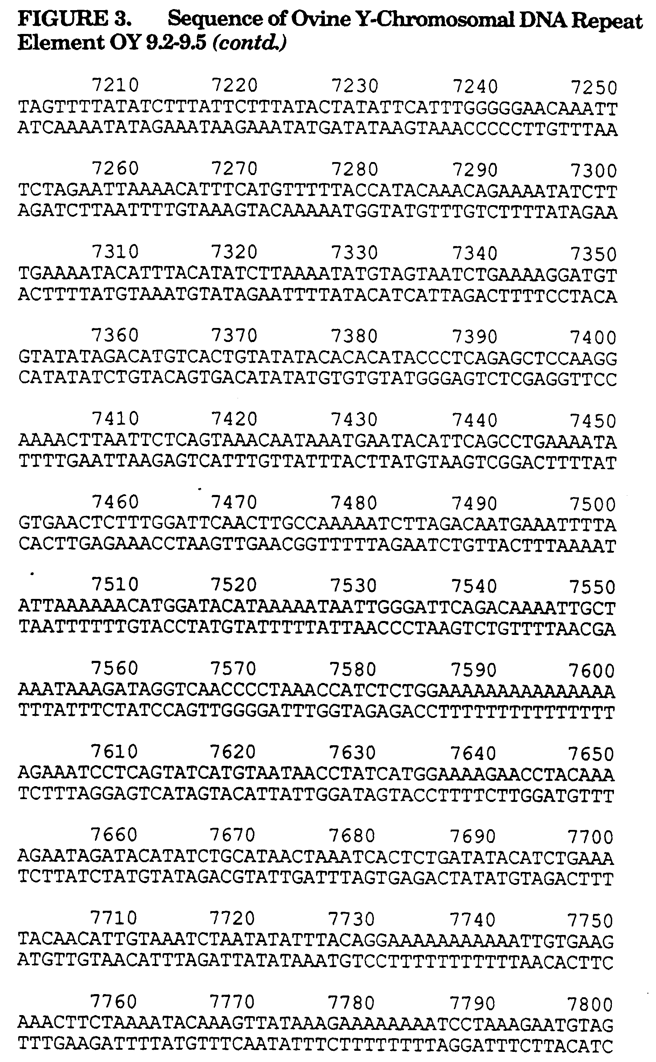

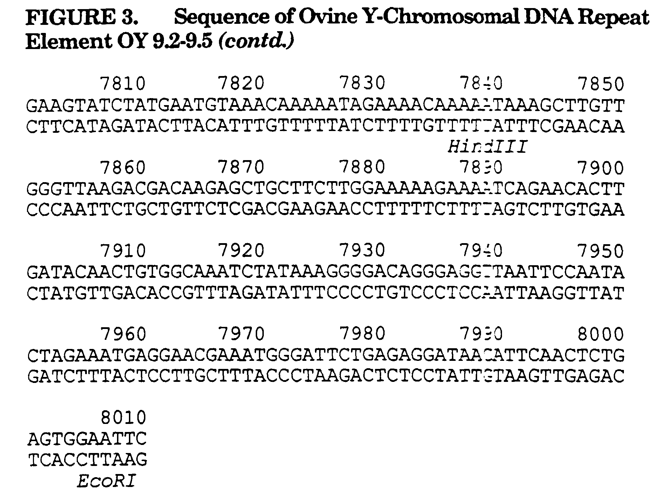

OY9.1 is a 2,555 base pair HindIII-BamHI restriction fragment (corresponding to base pairs 1071 and 3625 of Figure 3) that was cloned from a recombinant bacteriophage (λOGY9) containing approximately 13,100 base pairs of ram genomic DNA. Bacteriophage λOGY9 was identified and isolated from the unamplified library of ram genomic DNA by virtue of its hybridization to BRY.S. The restriction fragment OY9.1 was sub-cloned in consequence of its being found to hybridize with BRY.1. It was subsequently shown by hybridization to Southern blots of genomic DNA from individual male and female mammals to be generally conserved, male-specific and repeated in ruminants. The restriction fragment was blunt-ended with the large (Klenow) fragment of E. coli DNA polymerase I and cloned into the SmaI site of plasmid pTZ18u.

OY9.2 (Figure 3) is a 1,076 base pair BamHI-BamHI restriction fragment that was cloned from the recombinant bacteriophage λOGY9. The restriction fragment OY9.2 was sub-cloned in consequence of its being adjacent to OY9.1 and being found to hybridize with BRY.S but not with BRY.1. It was subsequently shown by hybridization to Southern blots of genomic DNA from individual male and female mammals to be generally conserved, male-specific and repeated in ruminants. The restriction fragment was blunt-ended with the large (Klenow) fragment of E. coli DNA polymerase I and cloned into the SmaI site of plasmid pTZ18u.

OY9.5 (Figure 3) is a 6,933 base pair EcoRI-BamHI restriction fragment that was cloned into plasmid pTZ18u from the recombinant bacteriophage λOGY9. The restriction fragment OY9.5 was sub-cloned in consequence of its subtending OY9.1, with which it shares the BamHI terminus, and a number of 124 base pair tandem repeats adjacent to the HindIII terminus of OY9.1.

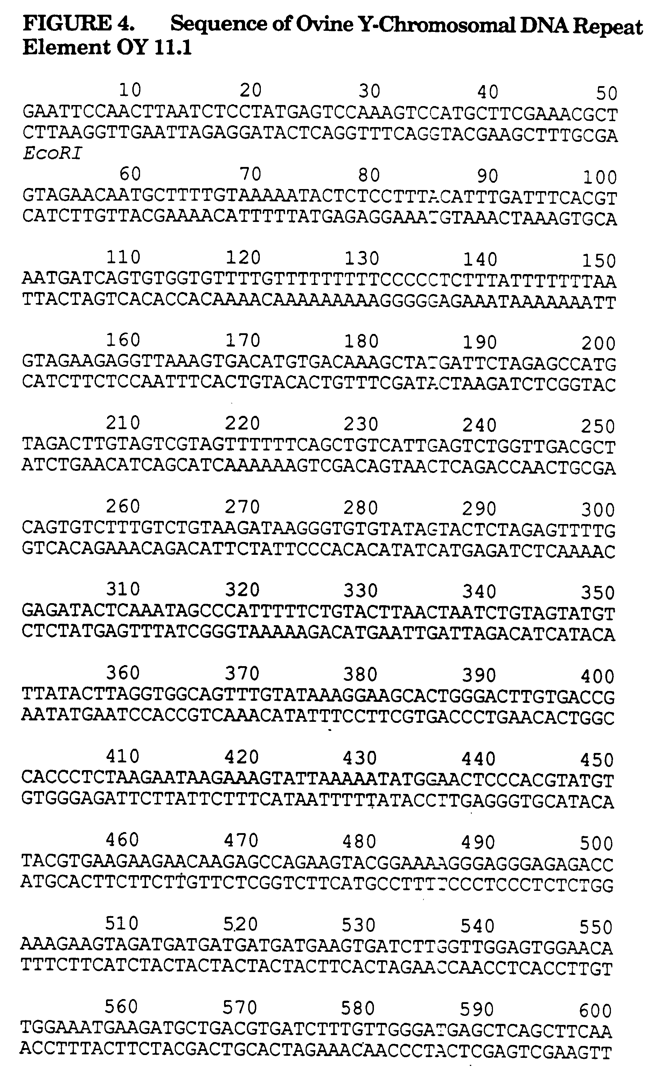

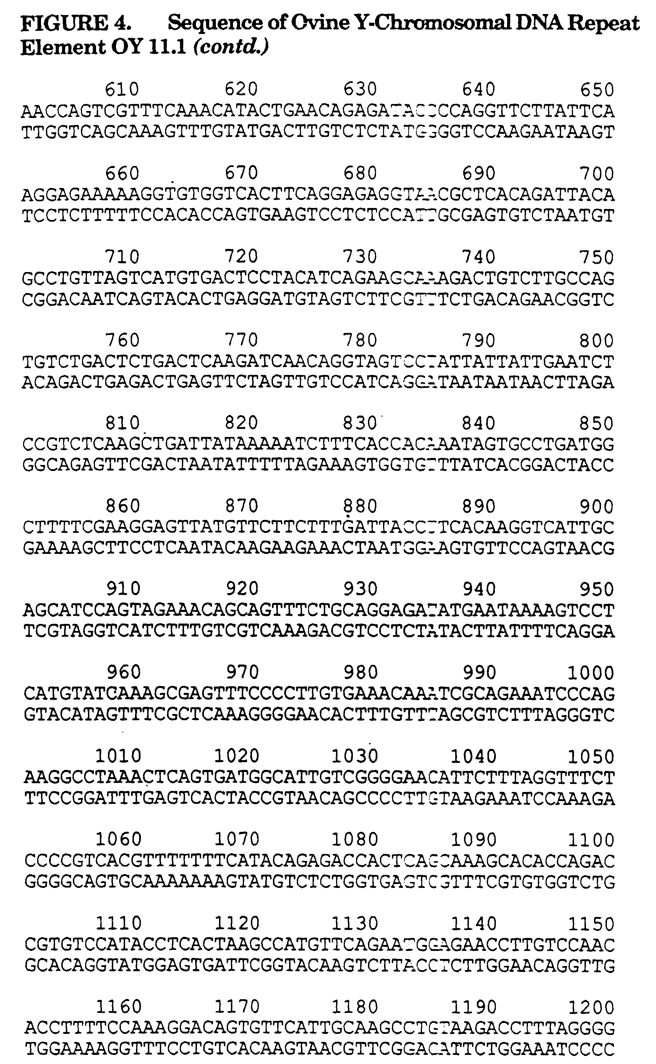

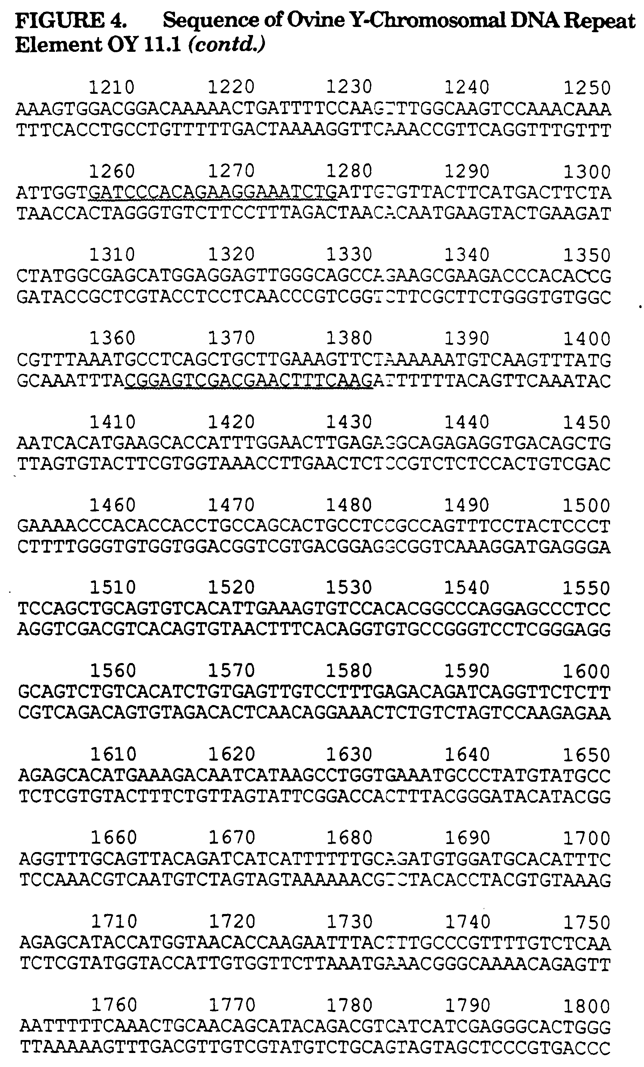

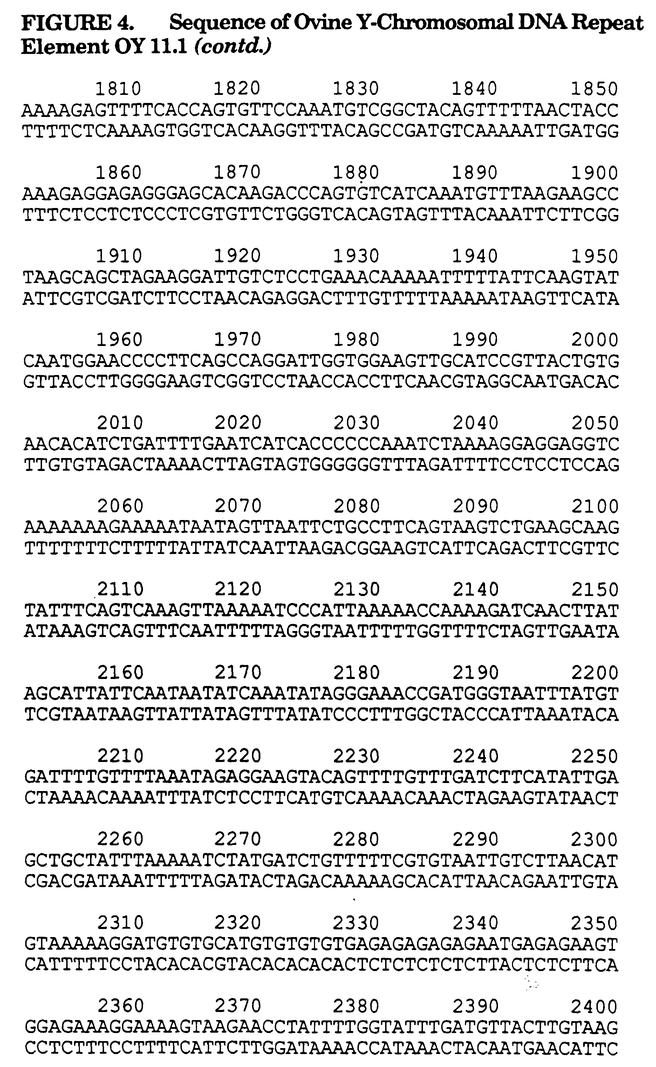

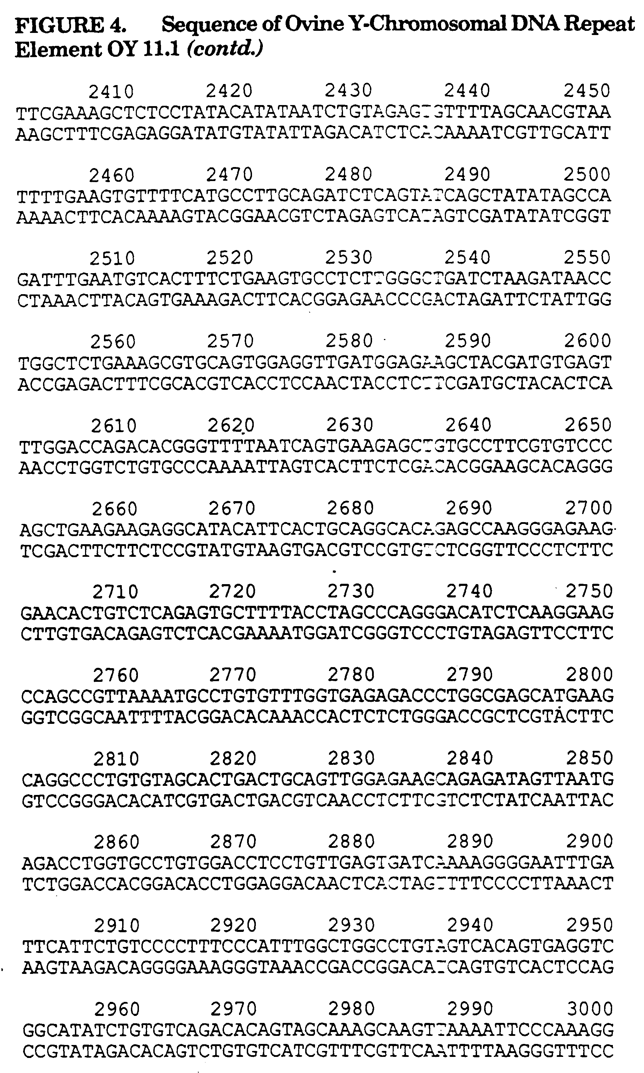

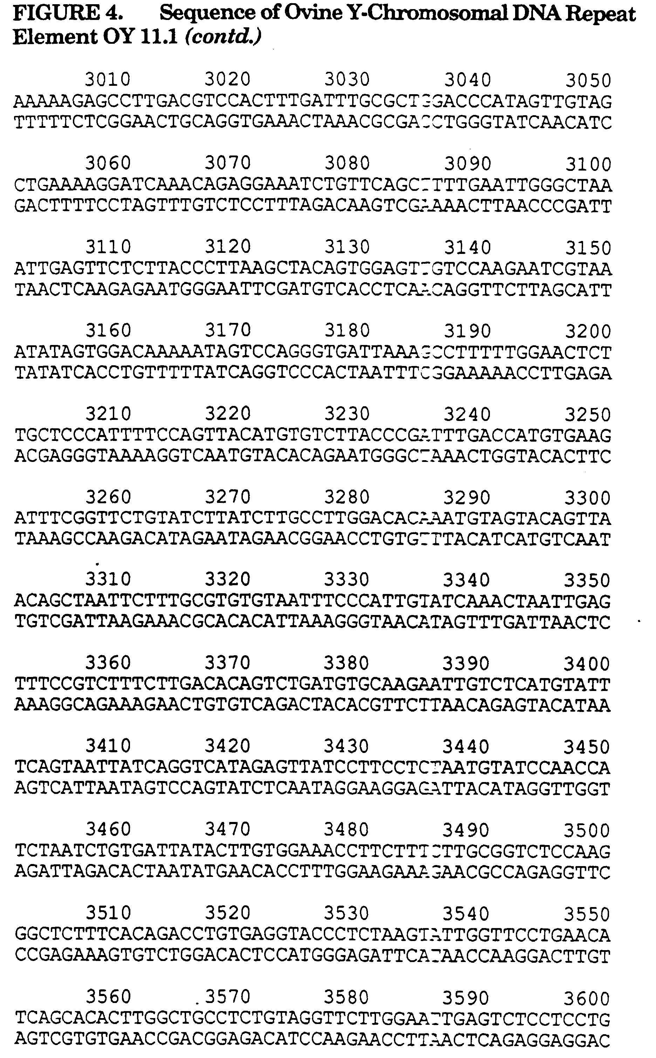

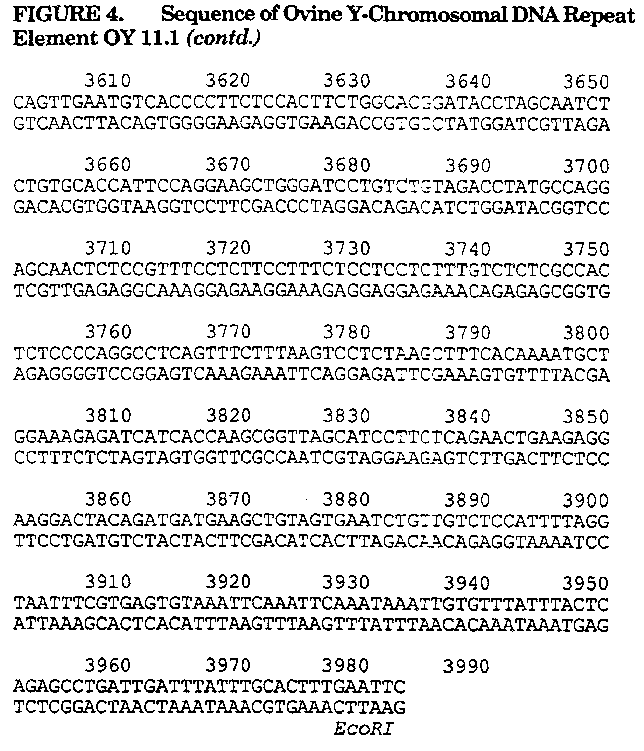

The OY11.1 DNA sequence comprising 3,983 nucleotide pairs is shown in Figure 4. OY11.1 is an EcoRI-EcoRI restriction fragment of a recombinant bacteriophage (λOGY11) containing approximately 18,100 base pairs of ram genomic DNA. Bacteriophage λOGY11 was identified and isolated from an unamplified library of ram genomic DNA by virtue of its hybridization to BRY.S. The restriction fragment was blunt-ended with the large (Klenow) fragment of E. coli DNA polymerase I and cloned into the SmaI site of plasmid pTZ18u.

The restriction fragment OY11.1 was cloned in consequence of its being found to hybridize with a discrete species of polyadenylated RNA in the testes of foetal cattle, and was subsequently shown by hybridization to Southern blots of genomic DNA from individual male and female mammals to be generally conserved, male-specific and repeated in ruminants. It was also found to hybridize to Southern blots of genomic DNA of all mammals examined, but in a manner indicative of one or a small number of copies/genome.

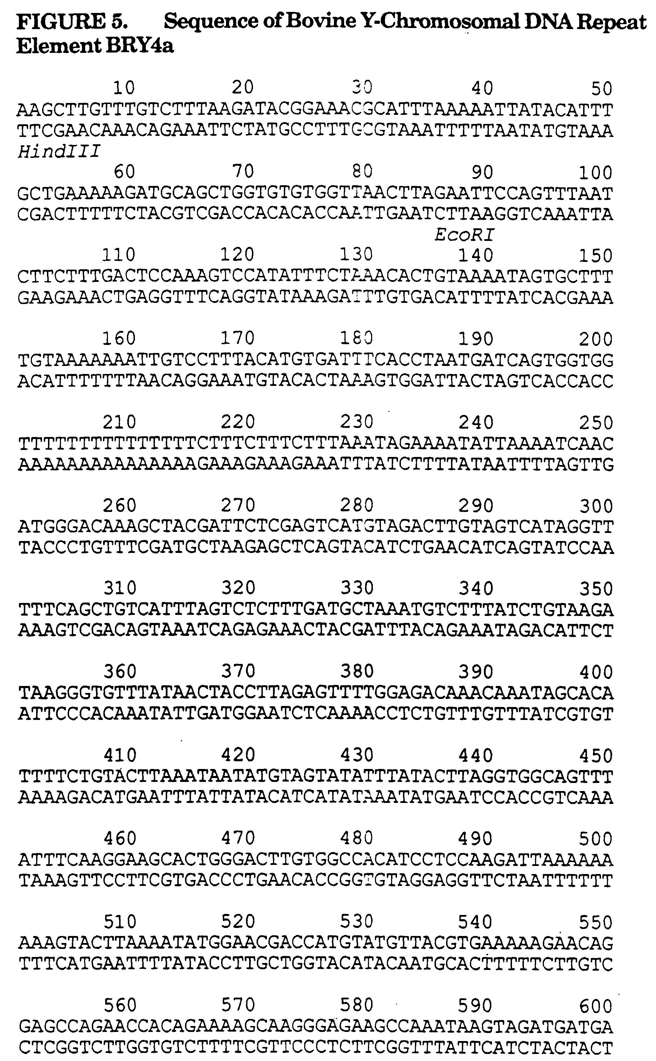

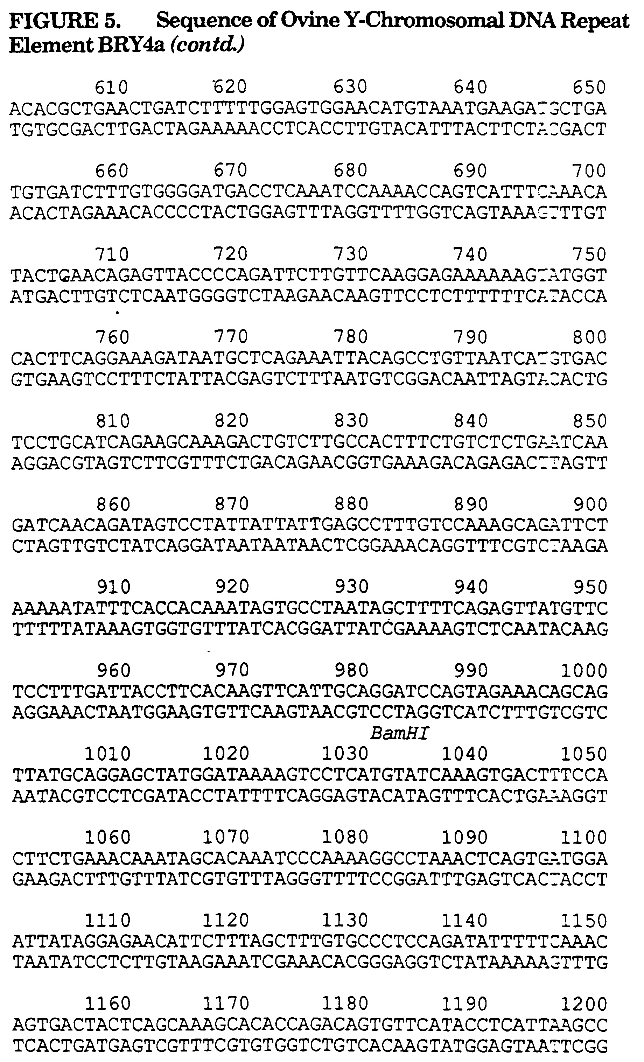

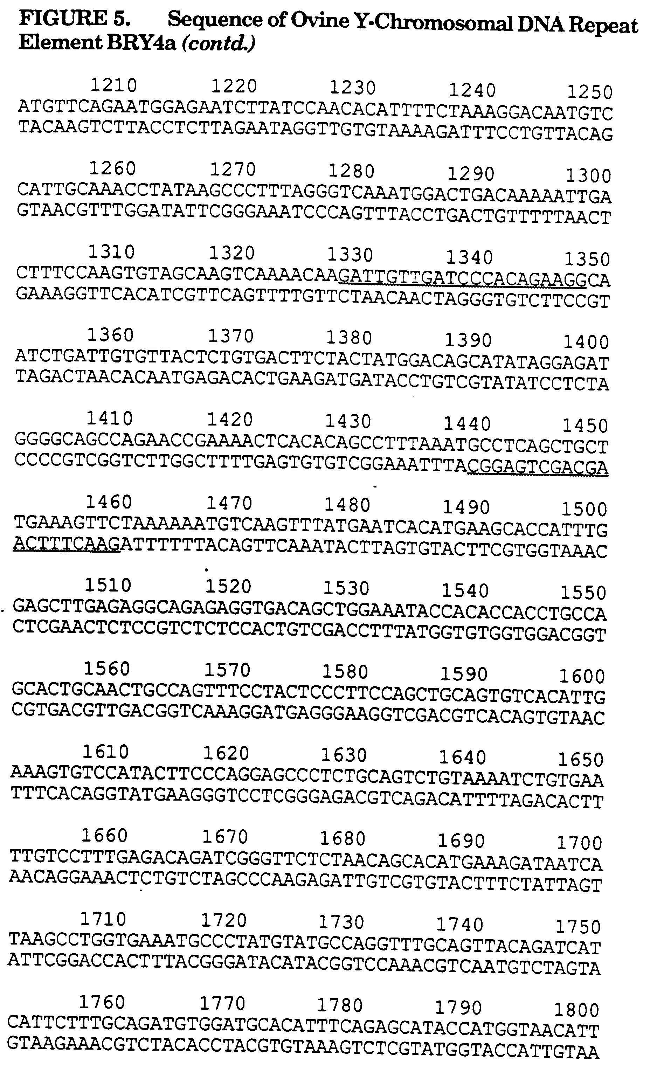

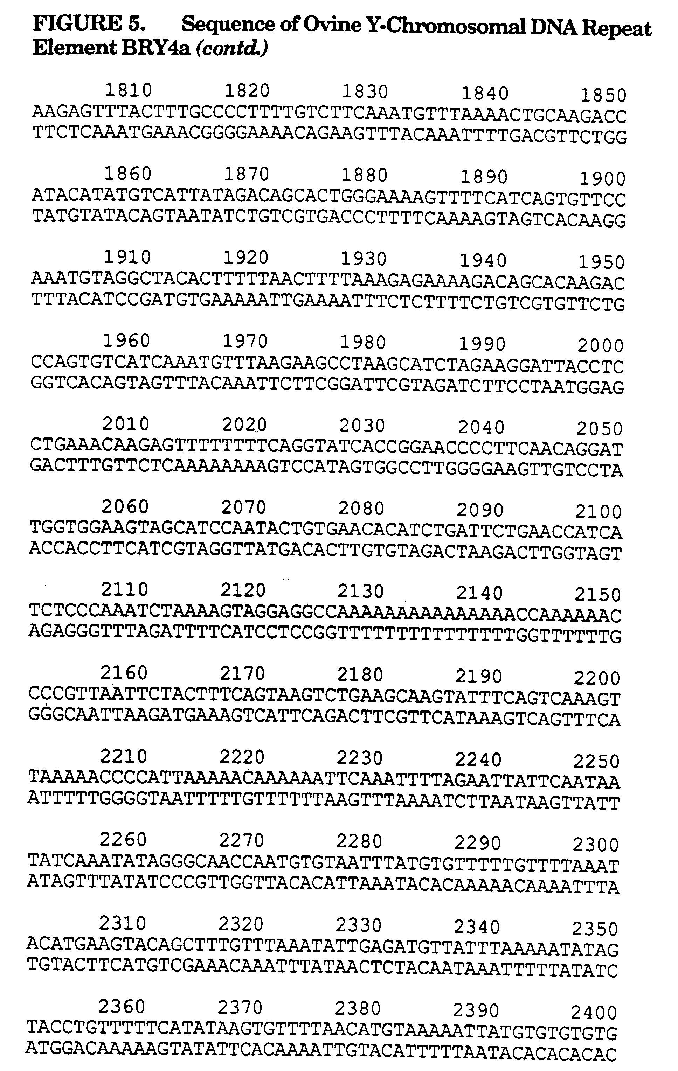

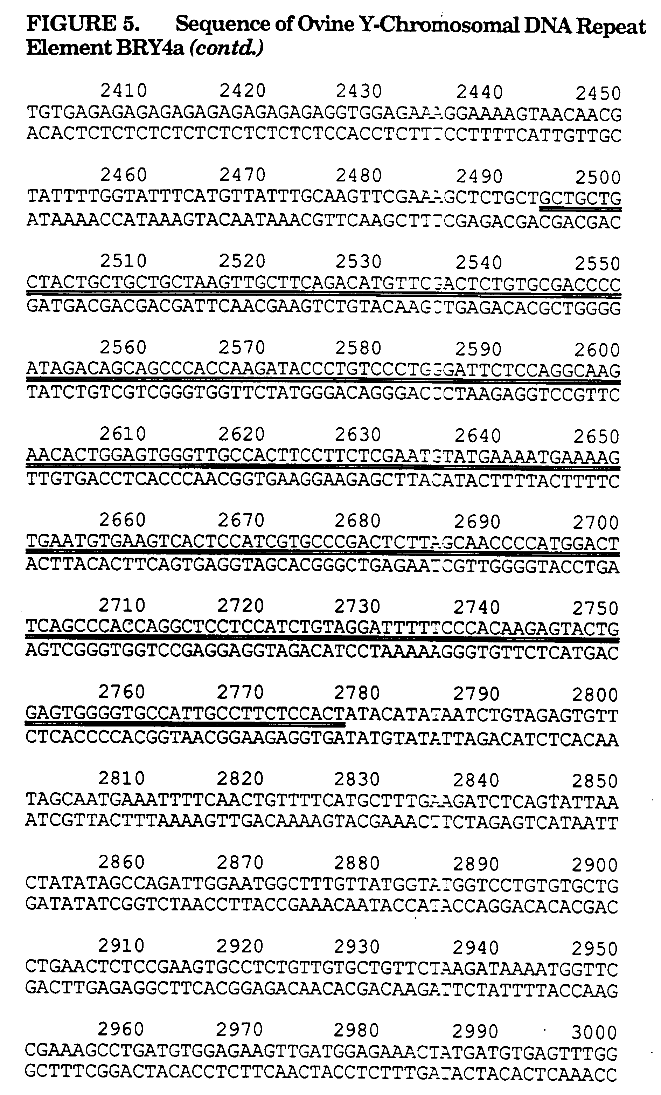

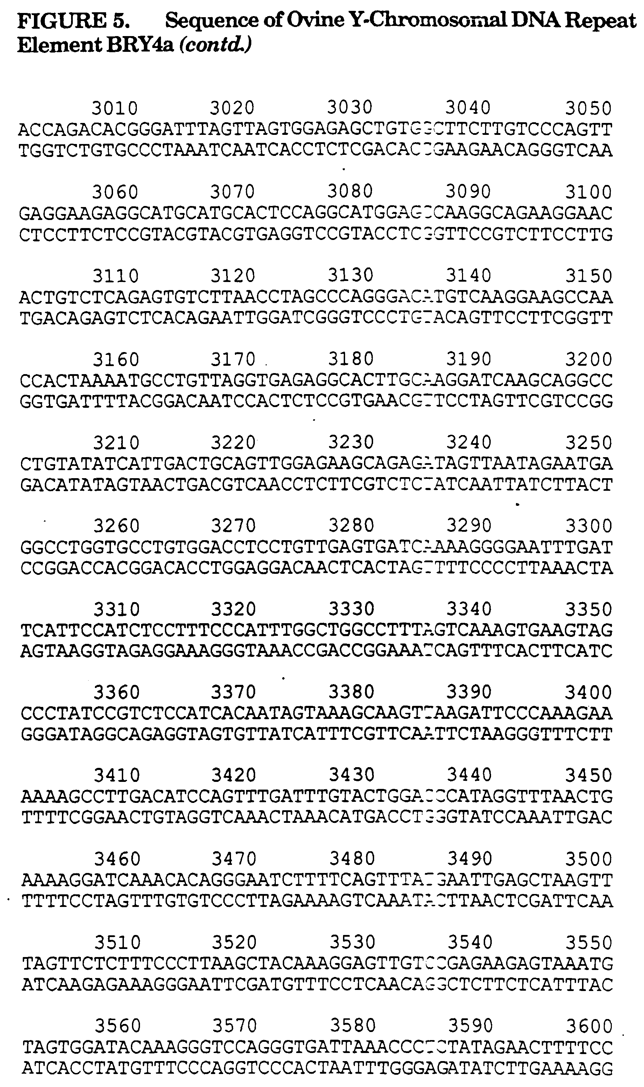

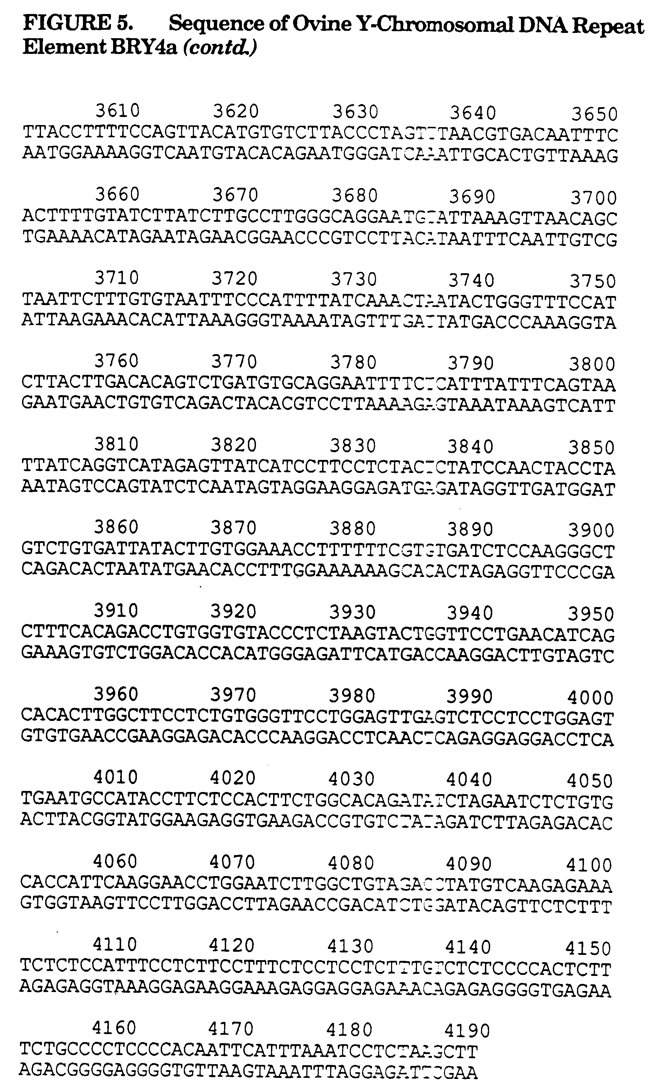

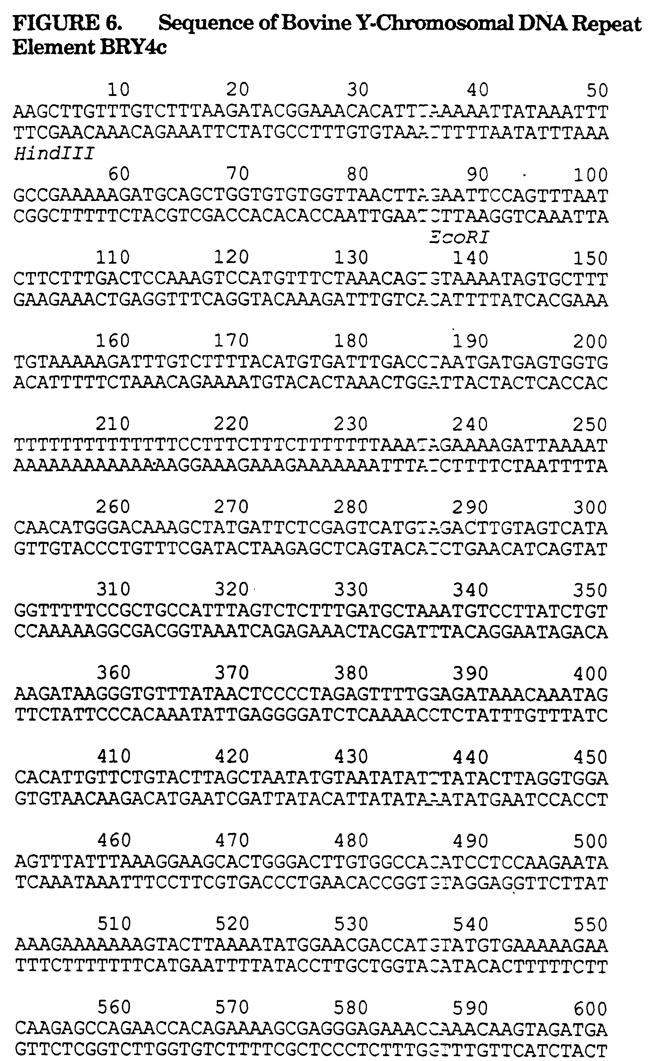

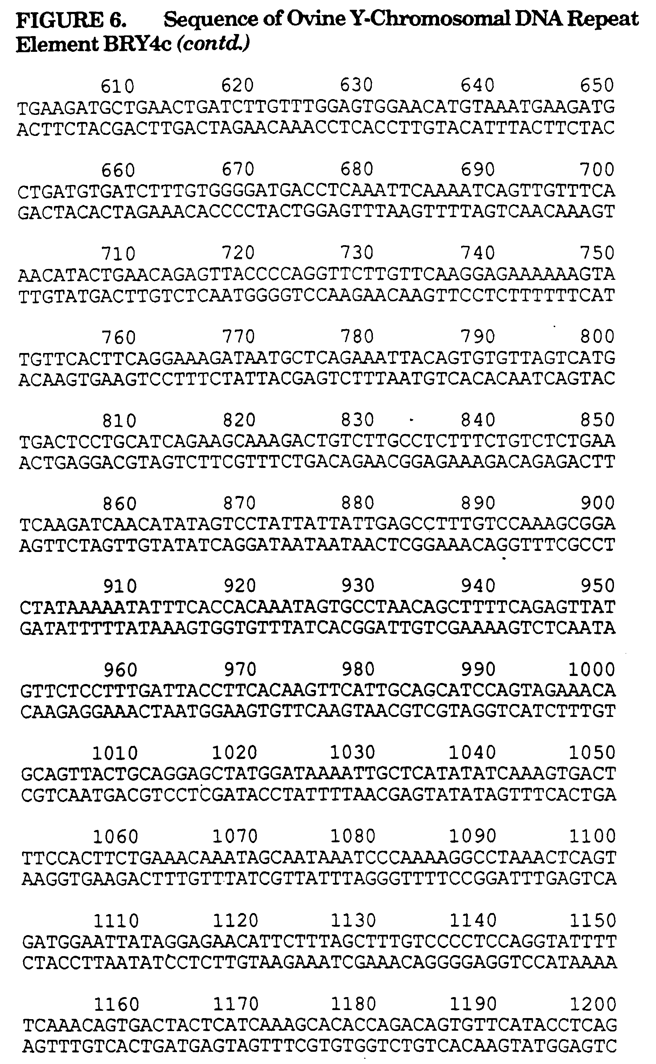

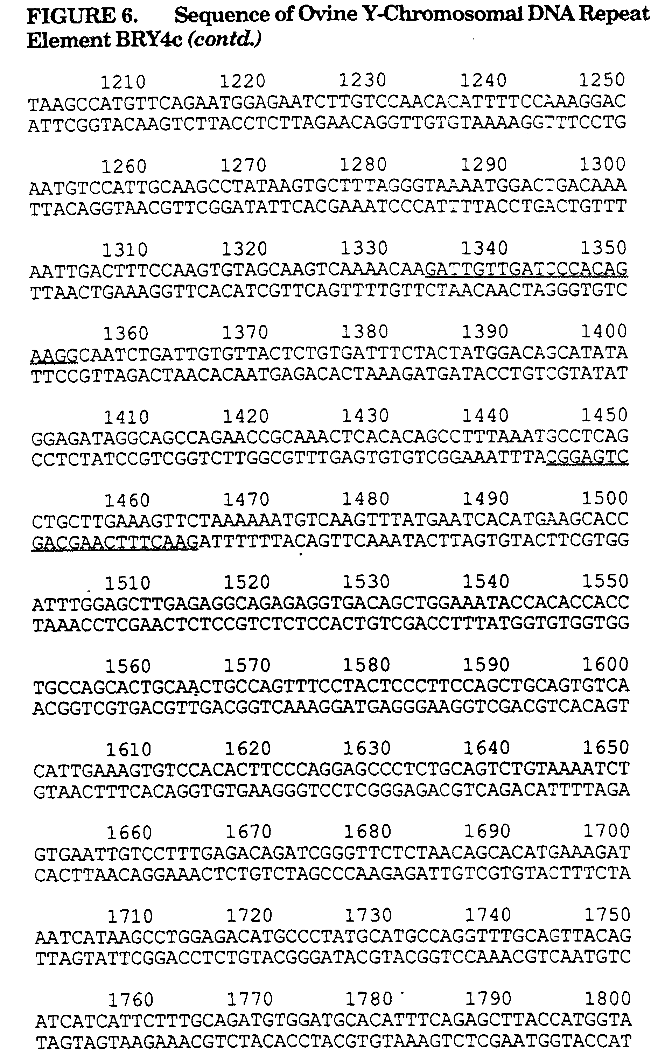

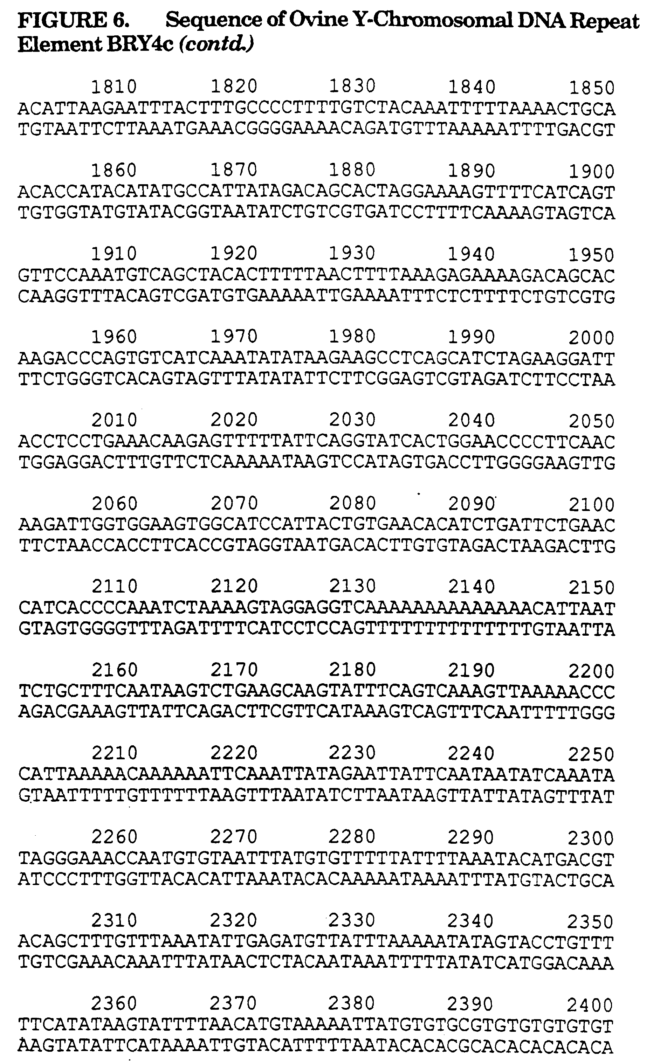

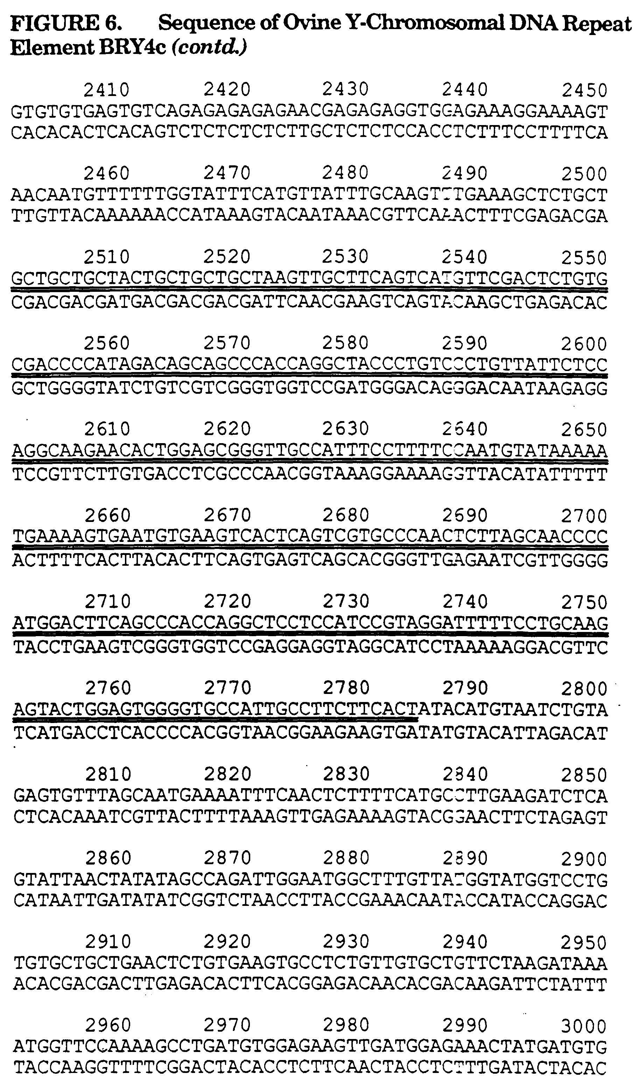

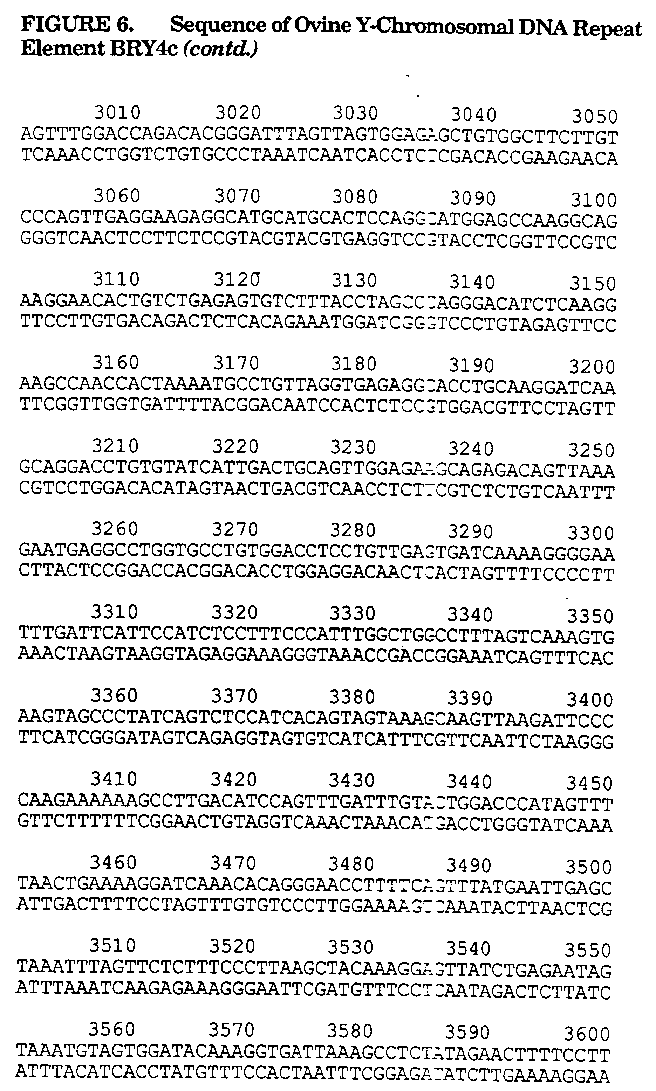

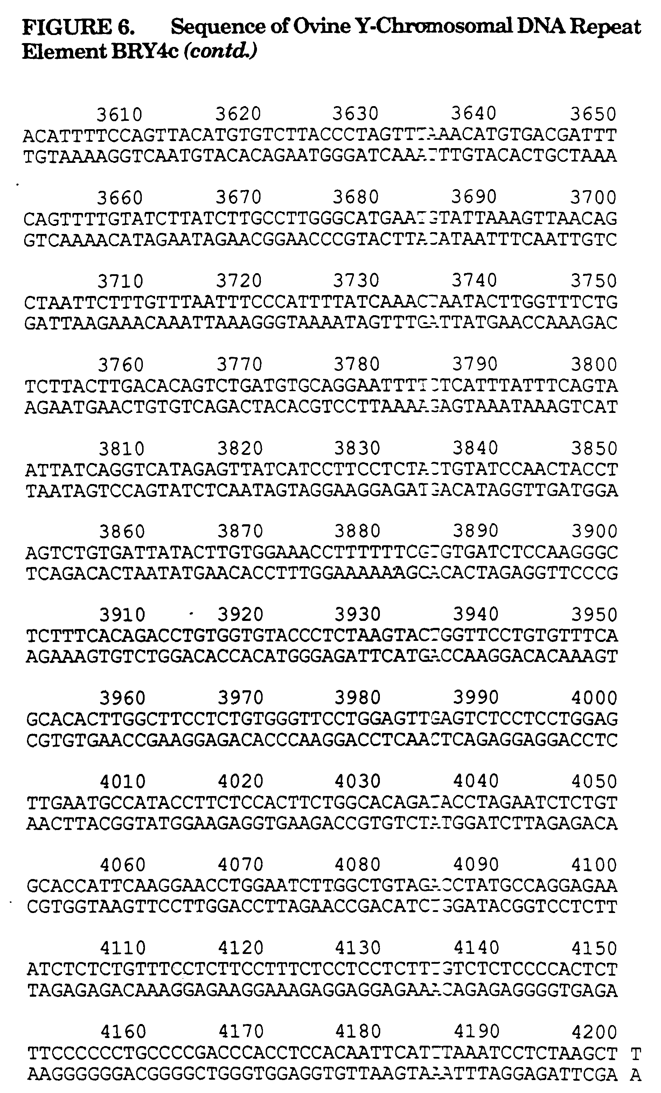

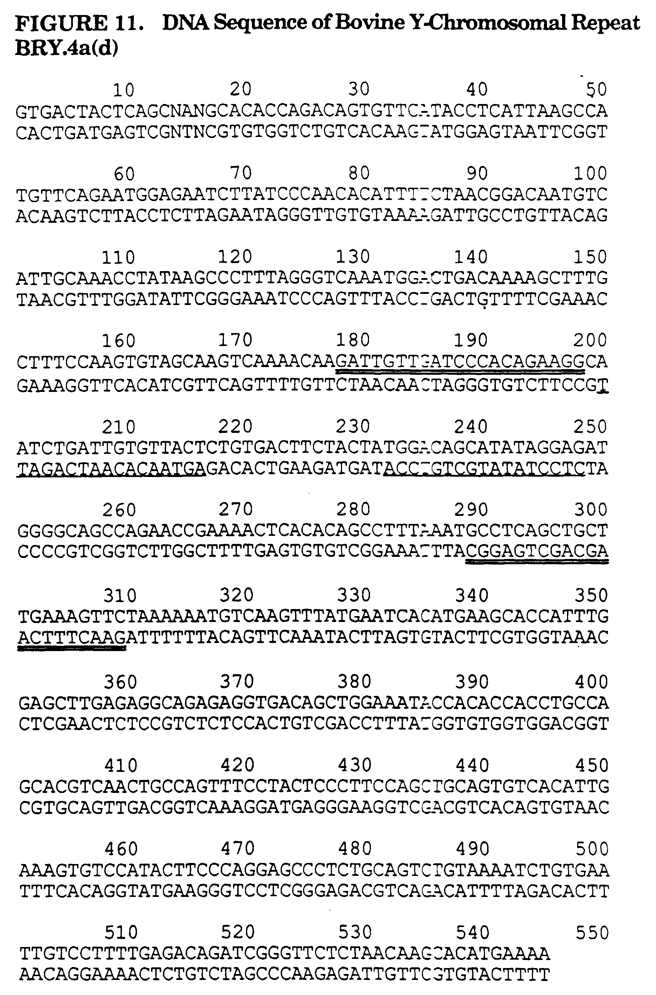

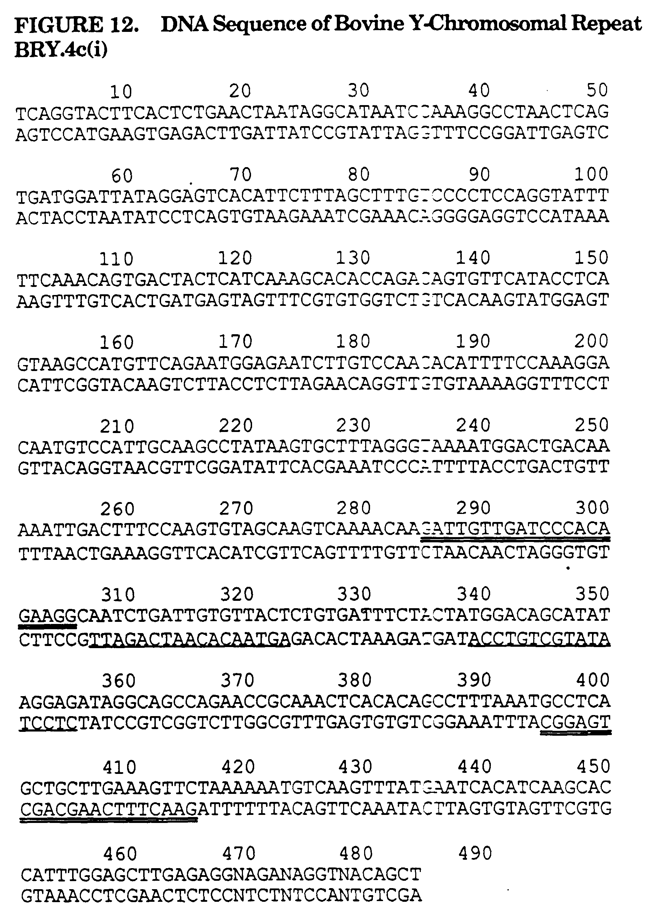

Of the nucleic acid isolates from the Y-chromosome of bovine animals, BRY.4a(d) (Figure 11) is 545 base pairs and is similar to bases numbered 1,074-1,613 in OY11.1 and BRY.4c(i) (Figure 12) is 484 base pairs and is similar to bases numbered 950-1,449 in OY11.1 BRY.4a(d) and BRY.4c(i) are portions of two independent 4,000 base pair (approx.) HindIII restriction fragments (BRY.4a and BRY.4c, respectively) that were resected into plasmid pTZ19u from recombinant bacteriophage (λBGY1 and λBGY2 respectively) which contained approximately 15,000 base pairs of bull genomic DNA. The DNA sequences of BRY.4a and BRY.4c are shown in Figures 5 and 6, respectively. The complete sequences of BRY.4b, 4d and 4e (not shown) are greater than 90% homologous with BRY.4a and BRY.4c.

Bacteriophage λBGY1 and λBGY2 were identified and isolated from an unamplified library of bull genomic DNA by virtue of their hybridization with OY11.1. The restriction fragments BRY.4a and BRY.4c were sub-cloned in consequence of both their hybridization to OY11.1 and their apparent identity in size to similarly hybridizing HindIII fragments of three additional independent recombinant bacteriophage (λBGY3, λBGY4 and λBGY5, respectively) isolated similarly from the bull library.

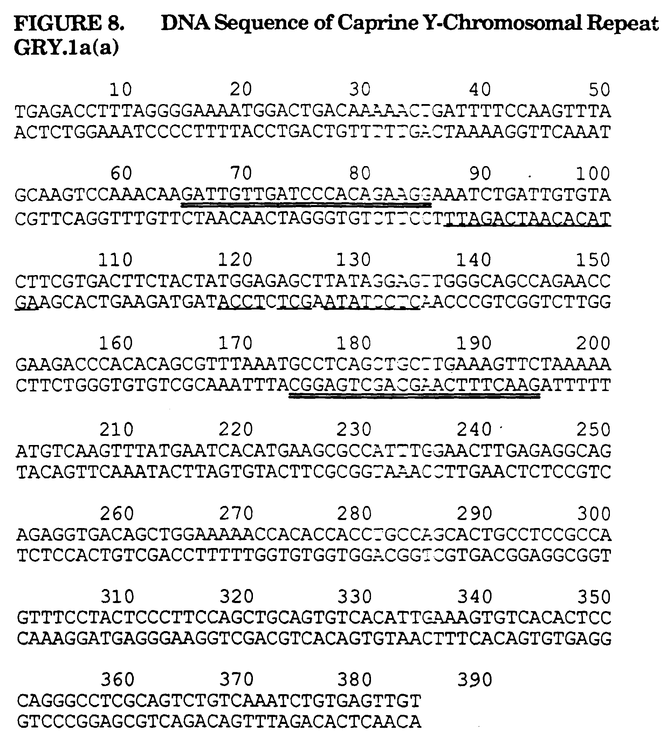

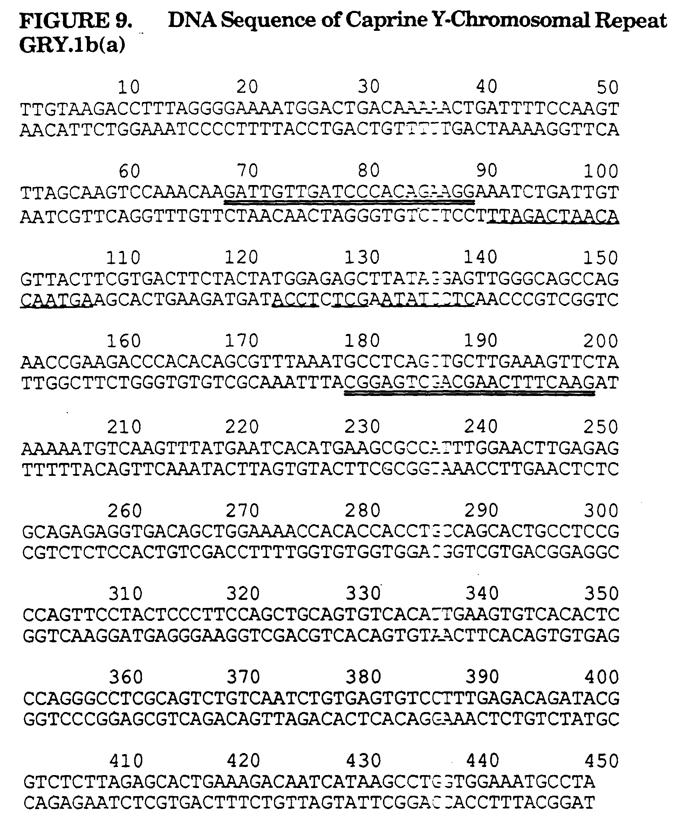

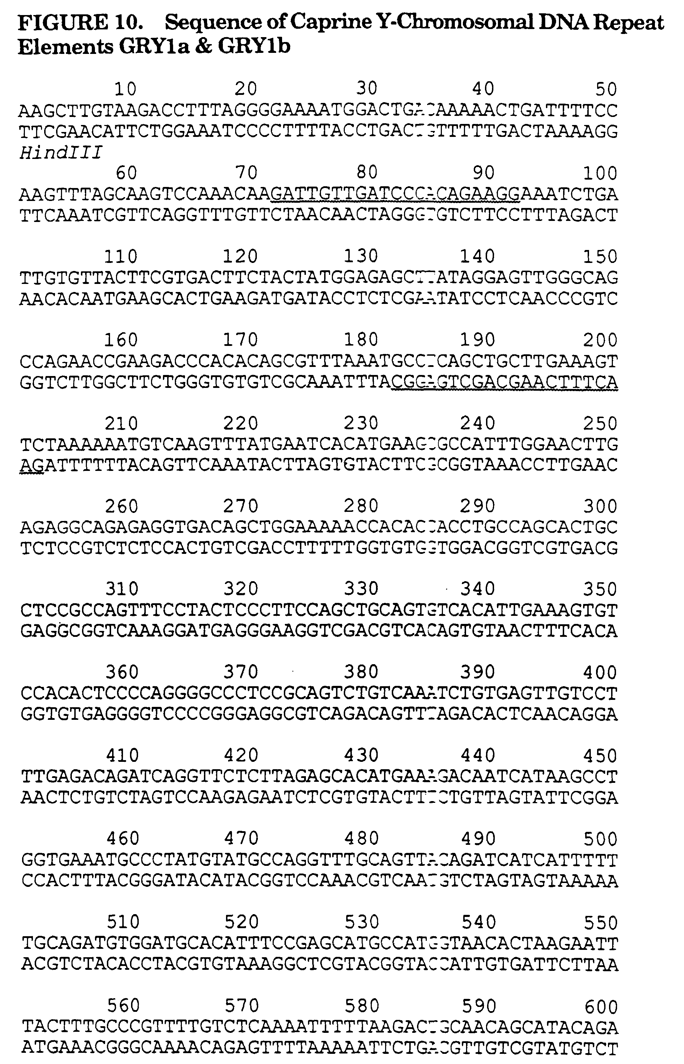

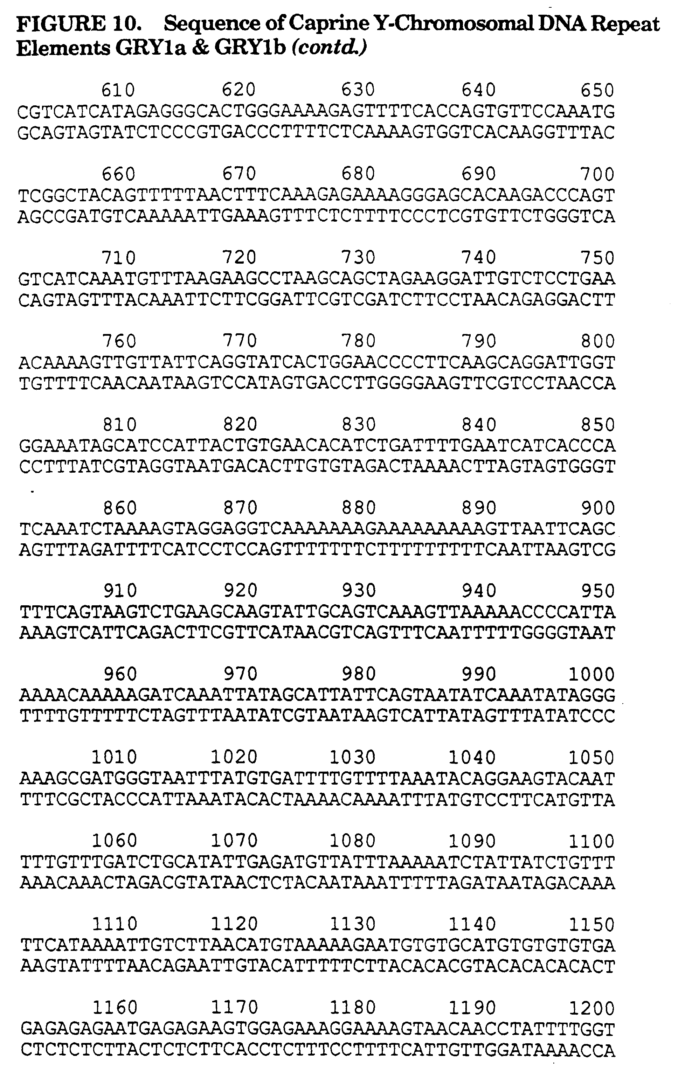

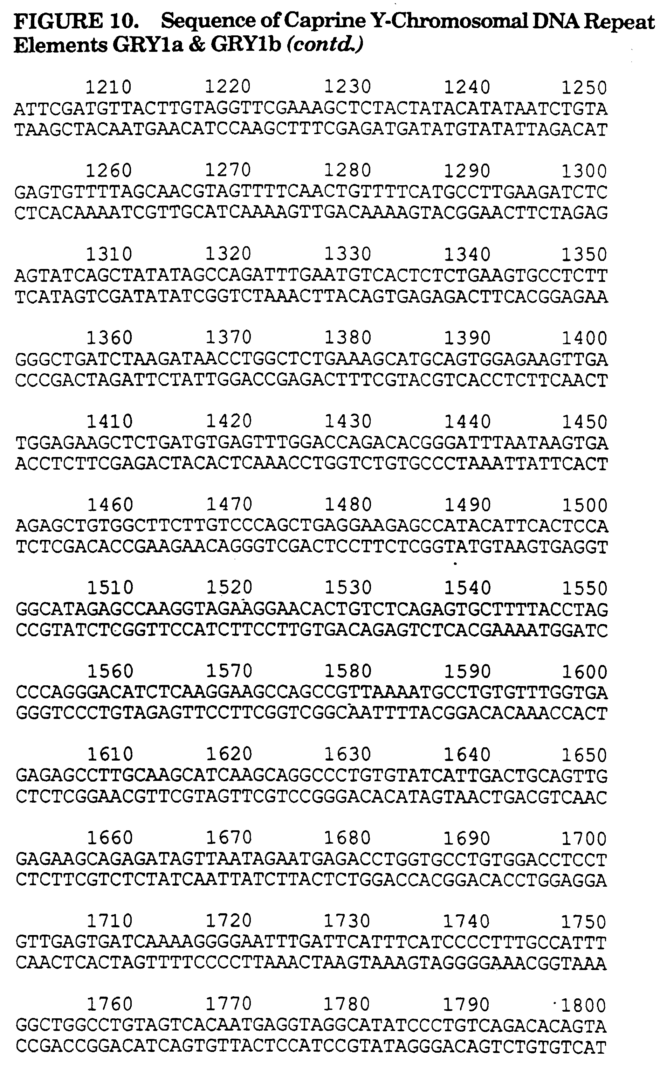

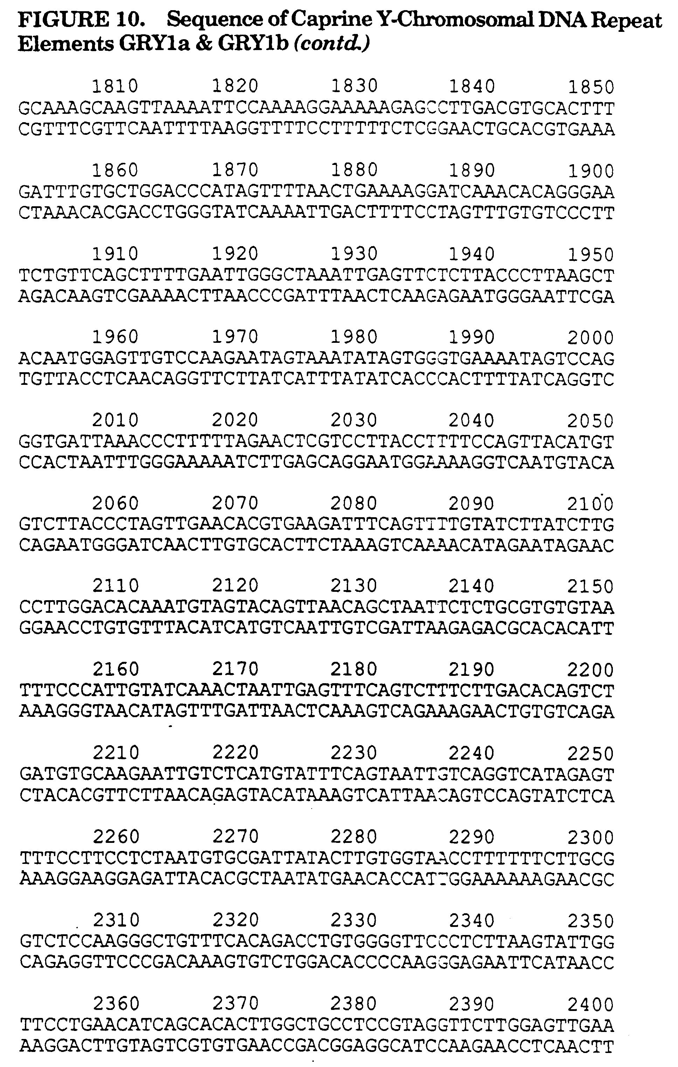

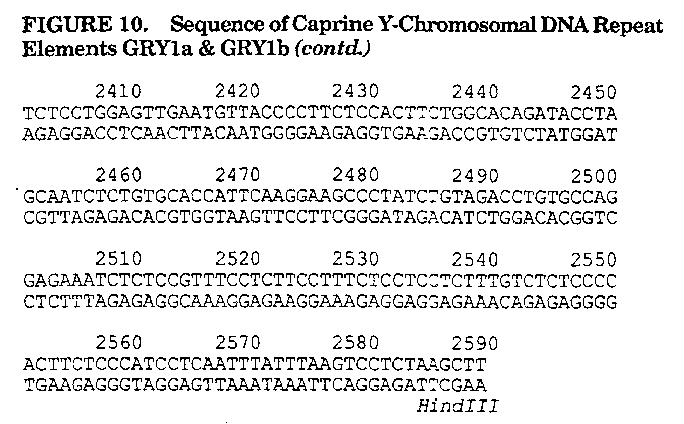

The DNA sequences of GRY.1a and GRY.1b are shown in Figure 10. Contained within these sequences are two smaller fragments designated GRY.1a(a) (384 base pairs, similar to bases numbered 1,186-1,575 in OY11.1) and GRY.1b(a) (448 base pairs, similar to bases numbered 1,183-1,642 in OY11.1) and the sequences of which are shown in Figures 8 and 9, respectively. GRY.1a(a) and GRY.1b(a) are HindIII restriction fragments of GRY.1a and GRY.1b, respectively, that were resected into plasmid pTZ19u from recombinant bacteriophage (λCGY1 and λCGY2, respectively) which contained approx. 15,000 base pairs of male goat genomic DNA. Bacteriophage λCGY1 and λCGY2 were identified and isolated from an unamplified library of male goat genomic DNA by virtue of their hybridization with OY11.1. The restriction fragments GRY.1a and GRY.1b were sub-cloned in consequence of both their hybridization to OY11.1 and their apparent identity in size to similarly hybridizing HindIII fragments of one additional independent recombinant bacteriophage (λCGY3) isolated similarly from the male goat library.

The DNA sequences described herein (Figure 1-12) were determined by standard DNA sequencing techniques (17), in which recombinant plasmids were transformed into E. coli strain JPA101 and infected with "helper" bacteriophage M13K07 to produce single-stranded recombinant plasmid molecules. The DNA sequences of these template molecules were determined by the Sanger chain termination procedure. The complete sequences were determined for both strands from a nested series of deletion recombinants generated by sequential erasure at one end of the insert by the combined actions of exonuclease III and S1 nuclease followed by re-circularization and transformation.

The terms "OY1.1", "OY4.1", "OY4.2", "OY9.2", "OY9.5", "OY11.1", "BRY.4a", "BRY.4b", "BRY.4c", "BRY.4,d", "BRY.4e", "GRY,1a", and "GRY,1b" refer to, where provided, the specific sequences set forth in Figures 1-12. These terms also include variants where nucleotides have been substituted, added to or deleted from the relevant sequences shown in Figures 1-12, as long as the variants hybridize with all or part of any of the sequences given in Figures 1-12 and diverge by no more than 25% from any of those sequences.

Such variants may be naturally occurring allelic and/or cis variants which may arise within a population of individuals by virtue of point mutation(s), deletion(s) or insertion(s) of DNA sequences, by recombination or by rearrangement, and are not restricted to ruminant species. Alternatively, such variants may be artificially produced, for example by site-directed mutagenesis, by deletion of fragments of DNA using exonuclease(s) and/or endonuclease(s) including restriction endonuclease(s), or by the addition of DNA sequences by ligating portions of DNA together, or by the addition of DNA sequences by templat-dependent and/or template-independent DNA polymerase(s).

The various DNA sequences described herein show a high degree of sequence conservation. For example, DNA sequences OY1.1; OY4.1; OY4.2; OY9.1; OY9.2; OY9.5 and OY11.1 share over 85% homology. DNA sequences OY11.1; BRY.4a; BRY.4b; BRY.4c; BRY.4d; BRY.4e; GRY.1a. and GRY.1b are similarly homologous.

The present invention also extends to any contiguous portion of 12 or more nucleotides of any and all of the sequences illustrated, hereinafter referred to as "oligonucleotides". Such oligonucleotides may be used as hybridization probes to detect any of the illustrated sequences or similar sequences in ruminant and non-ruminant species. Such oligonucleotides may be constructed synthetically using commercially available DNA synthesizers such as the Applied Biosystems 380A DNA Synthesizer, using standard methods. Oligonucleotides which comprise less than 12 nucleotides have limited effectiveness as hybridization probes.

According to a further aspect of the present invention, there is provided nucleic acid isolates comprising any contiguous portion of 12 or more nucleotides of any and all of the sequences claimed herein.

The present invention also includes recombinant DNA molecules constructed from all or part of the claimed sequences as defined above and a vector capable of propagation in host prokaryotic or eukaryotic cells for the purpose of cloning, amplification and/or expression of the claimed sequences.

Although such vectors are exemplified herein to be molecules such as pTZ18u or pTZ19u, the skilled artisan could use any number of a wide range of vector molecules depending on the intended use of the said nucleic acid sequences. Examples of other vector molecules can be found in reference (9), and include both eukaryotic and prokaryotic vectors, plasmids or bacteriophage molecules and shuttle vectors.

The present invention also extends to RNA molecules which correspond to part(s) or the total of any or all of the sequences claimed (where thymidine of the corresponding DNA sequence(s) is replaced by Uracil), including any contiguous portion of 12 or more nucleotides of such RNA sequence(s) corresponding to either or both of he complementary strands of the corresponding DNA sequences(s).

The nucleic acids of the invention may be utilized as hybridization probes, and may be labelled with radioactive markers such as ³²P, ³⁵S, ¹²⁵I, ¹⁴C, ³H, or with non-radioactive markers such as biotinylated (deoxy)nucleotide(s), bromodeoxyuridine (replacing deoxythymidine), or sulphonated (deoxy)nucleotide(s) by known methods (3-8). Claimed oligonucleotides may also be used as hybridization probes, and as primers for the determination of Y-specific DNA sequence in intact genomic DNA or in fragments of Y-specific DNA using known methods. These oligonucleotides or derivatives thereof may also be useful in oligonucleotide mutagenesis of Y-chromosomal genes.

RNA corresponding to all or part of the sequences claimed (as described above) may be produced using in vitro transcription systems, utilizing for example the RNA polymerases of bacteriophage SP6, T3 or T7 (7,8).

The nucleic acid isolates of the present invention may be used as hybridization probes to detect Y-chromosome-specific DNA and RNA sequences and, hence, to determine the genetic sex of, for example, an embryo or foetus of ruminant species. Similarly, the nucleic acid isolates may be used to detect variations in amounts and/or minor variations in sequence of corresponding sequences in individual animals. Such analyses are useful in paternity testing.

The DNA of fractionated sperm may also be tested with the nucleic acid isolates of the present invention. Particularly, fractionation procedures that in theory or in fact may enrich for sperm bearing a Y-chromosome can be assessed using the nucleic acid isolates of the present invention.

Where the sex of an embryo, foetus or individual ruminant animal or the sex chromosome content of individual sperm or a population of sperm is to be determined, a sample of cells is removed for assay. DNA and/or RNA may be extracted therefrom using known methods (9). The isolated DNA and/or RNA may then be applied and fixed directly to a membrane such as nitrocellulose, Nylon-66 (trademark DuPont) or a derivative thereof such as Hybond-N (trademark The Radiochemical Centre), including charge-modified derivative(s) such as Zeta-Probe membrane (trademark Bio-Rad). Alternatively, the DNA and/or RNA may be electrophoresed through a gel matrix and then transferred and fixed to a similar membrane.

The nucleic acids bound to the membrane are then hybridized with any or all of the nucleic acid isolates of the invention which is/are labelled with a detectable marker as hereinbefore described. Labelled isolate which binds to nucleic acid on the membrane is detected appropriately, for example by autoradiography (10). If the labelled isolate(s) hybridize(s) to similar sequences in the target sample, sex can be unequivocally designated male. In the above-described method, the target DNA of the embryo, foetus, and the like may be amplified according to the procedures of Saiki et al. (11,12).

According to a further aspect of the invention, nucleic acids are not extracted from the sample of cells removed for assay. The cells are heated in alkaline solution and the resultant solution is filtered directly onto a charge-modified nylon membrane such as Zeta-Probe membrane. DNA fixed to the membrane is hybridized with nucleic acid isolate(s) of the invention as described above.

According to a further aspect of the invention, there is provided a method for the determination of the sex chromosome constitution of a tissue or cell sample comprising, isolating DNA from said tissue of cell sample, immobilizing the isolated DNA onto a support matrix, hybridizing the immobilized DNA with a nucleic acid isolate capable of binding only to Y-chromosome specific DNA sequences, washing unbound nucleic acid isolate from the support matrix and subsequently detecting binding of said nucleic acid isolate to DNA bound to the support matrix.

According to another aspect of the present invention there is provided a method for determining the presence or absence of a Y-chromosome in fixed cells or interphase or metaphase chromosome spreads comprising, hybridizing said fixed cells or interphase or metaphase chromosomes with a nucleic acid isolate capable of hybridizing only to Y-chromosome specific DNA sequences of ruminants, under conditions enabling the nucleic acid isolate to bind to complementary DNA sequences, washing away unbound nucleic acid isolate, and detecting binding of the nucleic acid isolate standard techniques of in situ hybridization are used (13).

According to another aspect of the present invention there is provided a method for determining the sex chromosome constitution of a tissue or cell sample comprising isolating DNA from the tissue or cell sample and denaturing the isolated DNA to separate the respective coding and non-coding strands, annealing the denatured DNA with a synthetic oligonucleotide corresponding to 12 or more nucleotides from any of the claimed sequences, incubating the annealed DNA with DNA polymerase 1 to extend the oligonucleotide through the said sequences, if present in the tissue or cell sample; repeating this sequence as many times as desired to amplify levels of target DNA; and subsequently detecting the target DNA in the amplified sample either by:
(a) immobilizing the DNA onto a support matrix, hybridizing the immobilized DNA with a nucleic acid isolate capable of hybridizing only to Y-chromosome specific DNA sequences of ruminants which have been labelled with a detectable marker, under conditions enabling the labelled nucleic acid isolate to bind to complementary sequences, washing unbound isolate from the support matrix, and subsequently detecting binding of the nucleic acid isolate to DNA bound to the support matrix; or
(b) where labelled nucleotide precursors are included in the incubation with the DNA polymerase, fractionating the sample by electrophoresis in a gel matrix, and detecting labelled target DNA sequences which are fractionated in the gel matrix.

Nucleic acid hybridizations are carried out under standard conditions according to the methods of Reed and Mann (14) and Maniatis et al. (9).

According to a further aspect of the invention, a portion of target DNA sequence in the sample of cells may be amplified by "polymerase chain reaction" (PCR). This procedure may be applied to the determination of sex in bovine embryos by using the oligonucleotide primers Pll and P12 defined in Figure 8 to amplify the DNA sequence occurring between these sequences in target DNA. The amplified target sequences may be detected following their fixation to a nylon membrane by using a labelled oligonucleotide such as P13 or P14 (defined in Figure 8) in hybridization analysis. Alternatively, the amplified target sequences may be electrophoresed through a gel matrix then transferred to a nylon membrane for hybridization analysis. The amplified target sequences may also be visualized in the gel matrix following electrophoresis and staining, for example, with a standard silver reagent or with ethidium bromide and visualization under ultraviolet light. Even more particularly, the present invention provides two universal sexing primers, USP1 and USP2 (Figure 12), which can be used for PCR sexing of all three domestic ruminants.

The oligonucleotides used in the foregoing description of PCR sexing of bovine embryos are included by way of example only and may not be construed as being the only oligonucleotides derived from the sequences of Figures 1-12 that are claimed for hybridization analysis or for the application of PCR to the determination of sex in ruminant species.

The nucleic acid isolate of the present invention may comprise or form part of a kit for detecting the presence or absence of Y-chromosome specific sequences in a tissue or cell sample. The nucleic acid isolate may be labelled with detectable markers. Kits may include buffers for diluting reagents, labelled compound(s), and solid supports on which assays may be performed.

According to still a further aspect of the present invention, there is provided a method for the isolation of Y-chromosome specific DNA comprising:
(i) annealing single-stranded ruminant genomic DNA of male and female animals under conditions whereby repeated DNA sequences anneal but "single copy" sequences do not, where the DNA of males has been previously subjected to digestion with a restriction endonuclease generating cohesive termini and the DNA of females has been subjected to random fragmentation;
(ii) ligating the annealed DNA into a replicable vector which has been prepared with cohesive termini complementary to those of the male genomic DNA fragments;
(iii) transforming host cells with the replicable vector containing the annealed DNA; and
(iv) hybridizing the DNA of transformed host cells with a labelled DNA probe prepared from female ruminant genomic DNA, and identifying those host cells which contain ruminant genomic DNA which does not hybridize with the labelled probe.

### DESCRIPTION OF THE DRAWINGS

The present invention will now be described, by way of example only, with reference to the following non-limiting drawings and examples, wherein:

**FIGURE 1** shows the double-stranded DNA sequence of ovine repeat element OY1.1 comprising 3,142 complementary base pairs. The sequence is written in single-letter code with the upper strand (that sequence immediately beneath numerals indicating number of base pairs) written as 5'-->3' and the complementary (lower) strand written 3'-->5' according to standard practice, where A refers to deoxyadenosine-5'-phosphate, C refers to deoxycytidine-5'-phosphate, G refers to deoxyguanosine-5'-phosphate, and T refers to deoxythymidine-5'-phosphate. The underlined sequence (base numbers 2,171-2,475 inclusive) is similar to the sequence of bovine repeat element BRY.1 described in International Patent Application PCT/US87/00254.

**FIGURE 2** shows the double-stranded DNA sequence of ovine repeat element OY4.1-4.2.

**FIGURE 3** shows the double-stranded DNA sequence of ovine repeat element OY9.2-9.5.

**FIGURE 4** shows the double-stranded DNA sequence of ovine repeat element OY11.1 comprising 3,983 base pairs. The sequences in double underline (base numbers 1,251-1,270 inclusive of the complement of base numbers 1,360-1,380 inclusive) are identical to the sequences of PCR primers P12 and P11, respectively; the sequences in single underline (the complements of base numbers 1,272-1,288 inclusive and 1,304-1,320 inclusive) are identical to those of the hybridization oligonucleotides P13 and P14, respectively.

**FIGURE 5** shows the double-stranded DNA sequence of bovine repeat element BRY.4a.

**FIGURE 6** shows the double-stranded DNA sequence of bovine repeat element BRY.4c.

**FIGURE 7** shows a comparison of sequences subtended by oligonucleotide primers used for genetic sex detemination by PCR.

**FIGURE 8** shows the double-stranded DNA sequence of caprine repeat element GRY.1a(a) comprising 384 base pairs. The sequences in double underline (base numbers 65-85 inclusive and the complement of base numbers 174-194 inclusive) are identical to the sequences of PCR primers P12 and P11, respectively; the sequences in single underline (the complements of base numbers 87-102 inclusive and 118-134 inclusive) are identical to those of the hybridization oligonucleot4ides P13 and P14, respectively.

**FIGURE 9** shows the double-stranded DNA sequence of caprine repeat element GRY.1b(a) comprising 448 base pairs. The sequences in double underline (base numbers 68-88 inclusive and the complement of base numbers 178-198 inclusive) are identical to the sequences of PCR primers P12 and P11, respectively; the sequences in single underline (the complements of base numbers 90-106 inclusive and 122-138 inclusive) are identical to those of the hybridization oligonucleotides P13 and P14, respectively.

**FIGURE 10** shows the double stranded DNA sequence of caprine repeat elements GRY.1a and GRY.1b.

**FIGURE 11** shows the double-stranded DNA sequence of bovine repeat element BRY.4a(d) comprising 545 base pairs, written as for Figure 1. The sequences in double underline (base numbers 178-198 inclusive and the complement of base numbers 289-309 inclusive) define the sequences of PCR primers P12 and P11, respectively; the sequences in single underline (the complements of base numbers 200-216 inclusive and 232-248 inclusive) define the sequences of hybridization oligonucleotides P13 and P14, respectively.

**FIGURE 12** shows the double-stranded DNA sequence of bovine repeat element BRY.4c(i) comprising 484 base pairs. The sequences in double underline (base numbers 285-305 inclusive and the complement of base numbers 395-415 inclusive) define the sequences of PCR primers P12 and P11, respectively; the sequences in single undeline (the complements of base numbers 307-323 inclusive and 339-355 inclusive) define the sequences of hybridization oligonucleotides P13 and P14, respectively.

**FIGURE 13** shows a comparison of repeat elements BRY4a, OY11.1 and GRY1a with bovine testes cDNA BCY11a.

### DEFINITIONS AND ABBREVIATIONS

- DNA -: deoxyribonucleic acid
- RNA -: ribonucleic acid
- cDNA -: complementary DNA (enzymatically synthesized from a mRNA sequence)
- mRNA -: messenger RNA
- A -: Adenine
- T -: Thymine
- G -: Guanine
- C -: Cytosine
- U -: Uracil
- Tris -: Tris (hydroxymethyl) aminomethane
- EDTA -: Ethylenediaminetetracetic acid
- SDA -: Sodium dodecylsulphate
- psi -: pounds per square inch
- K.MES -: potassium morpholinoethane-sulphonate
- polynucleotide -: single or double-stranded DNA or RNA

### EXAMPLE 1:

### Preparation of Genomic Libraries:

Portions of liver from male and female ruminants (e.g. cattle, sheep or goats) were collected at an abbatoir and immediately frozen in liquid nitrogen. Samples (0.8g) of frozen liver from individual animals were homogenised in 8ml of HB (0,1M NaCl, 10mM Tris-HCl (pH 7.5), 1mM EDTA). To each homogenate was added, with thorough gentle mixing, 2.2ml of 0.5M EDTA and 1.22ml of 20% (w/v) Sarcosyl. The suspensions were heated at 65°C for 15 min with occasional gentle mixing, 11.6g of solid CsCl was dissolved in each, then lml of ethidium bromide (10mg/ml) was added and mixed. The suspensions were transferred into 13.5ml centrifuge tubes which were placed in a Beckman Ti80 rotor and centrifuged in a Beckman L8-80 ultracentrifuge at 45,000 rpm for 60h at 25°C.

The pink band of concentrated DNA near the middle of the tube was recovered by side puncture (with an 18g needle affixed to a 1ml plastic syringe) and transferred to a capped tube. Ethidium bromide was removed by repeated extraction with butanol (previously equilibrated with saturated NaCl solution) and the resultant clear DNA solution was dialysed against 3 x 2 litres of TE (10mM Tris-HCl, pH 8.0, 1mM EDTA) at 4°C.

### Preparation of Genomic DNA

Between 9µg and lmg of DNA isolated from the liver was digested to completion with restriction endonuclease Sau3Al under standard conditions, then extracted successively with phenol and ether. It was recovered by the addition of ammonium acetate to 2M and ethanol to 70% (v/v), mixing thoroughly, freezing in liquid nitrogen, thawing briefly, and centrifuging for 15 min at 4°C. The pellet of digested male DNA was then rinsed with 70% (v/v) ethanol and dried briefly in vacuo.

### Preparation of Cloning Vector (plasmid pUC9) (15):

The cloning vector was prepared by digestion of 100 µg of plasmid pUC9 with restriction endonuclease BamHI, followed by the addition of SDS to 0.5% (w/v) and 1/6 volume of 1M Tris-HCl, pH 9.0, and incubation at 37°C for lh with 40 µg of calf intestinal alkaline phosphatase (Boehringer ELISA grade). This solution was treated with phenol and DNA recovered by sodium acetate/ethanol precipitation as hereinbefore described. The restricted DNA was dissolved in TE/0.2% (w/v) Sarcosyl and layered onto a cold (4°C) linear gradient of 2-25% (w/v) sucrose dissolved in TE/0.2% (w/v) Sarcosyl in a Beckman SW41 ultracentrifuge tube. The tube was centrifuged in a Beckman L8-80 ultracentrifuge at 36,000 rpm for 20h at 4°C, then fractions of 0.5ml were collected using a gradient fractionator (ISCO).

Samples (10 µl) of each fraction were electrophoresed in a 1.5% (w/v) agarose gel containing ethidium bromide (0.5 µg/ml) then photographed under ultraviolet illumination (302 nm) to allow visual identification of fractions containing linearised plasmid.

DNA was recovered from 20 µl samples of each gradient fraction above by precipitation with ammonium acetate/ethanol as described above. To these samples was added 50 µl of competent JM83 cells (see below), the suspensions were kept on ice for 30 min, heated at 42°C for 90 see, then mixed with 0.1ml of 2YT medium and incubated at 37°C for 1h. Cells were recovered by centrifugation, resuspended in 0.2ml of 2YT medium, plated on 2YT agar (2YT medium with 1.5% (w/v) agar) containing 50 µg/ml ampicillin and incubated at 37°C overnight. Counting the number of colonies on each plate allowed identification of gradient fractions containing circular (uncut) plasmid.

Gradient fractions #13-16 (numbering started from top of gradient) containing a majority of linear, phosphatased plasmid and little circular plasmid (estimated from transforming ability) were pooled and the DNA recovered by precipitation with sodium acetate-ethanol as hereinbefore described.

### Preparation of Competent Cells:

Competent bacterial cells (Escherichia coli strain JM83) were prepared (16) by first inoculating 100ml of 2YT medium (sterile 1.6% (w/v) Bacto-tryptone, 1% (w/v) yeast extract, 0.5% (w/v) NaCl, pH 7.4) with JM83 cells and incubating with vigorous shaking until the optical absorbance of the culture at 600nm was 0.4. The culture flask was chilled in ice/water for 5 min and the cells harvested by centrifugation. The pellet was resuspended in 50ml of 50mM CaCl₂, allowed to stand on ice for 20 min, then recentrifuged. The pellet was resuspended in 5ml of cold 50mM CaCl₂ and allowed to stand on ice for 24h before being used for transformation assays.

### Preparation of Library

Samples of genomic DNA (200 µg) and gradient-purified linear vector DNA (10 µg) were combined in 5ml of ligation buffer (66mM Tris-HCl, pH 7.5, 6.6mM MgCl₂, 1mM ATP, 100 µg/ml BSA (bovine serum albumin, Boehringer nuclease-free), 10mM dithiothreitol, and 50 units of T4 DNA ligase (New England BioLabs)) and incubated overnight at 14°C.

Stocks of JM83 cells were prepared by dilution streaking a sample of cells onto LM agar (sterile 1% (w/v) Bacto-tryptone, 0.5% (w/v) yeast extract, 10mM NaCl, 10mM MgSO₄, 1.5% (w/v) agar) and incubating overnight at 37°C. A few isolated colonies were inoculated into 20ml of pre-warmed SOB medium (sterile 2% (w/v) Bacto-tryptone, 0.5% (w/v) yeast extract, 10mM NaCl, 2.5mM KCl, 20mM MgSO₄), the culture grown with vigorous aeration to an optical absorbance of 0.5 at 550 nm and diluted with 20ml of sterile SOB:glycerol (60:40 by volume). Samples (0.1ml) were transferred to 1.5ml sealable tubes and chilled on ice for 10 min then frozen in dry ice/ethanol and stored at -70°C.

Competent JM83 cells were prepared from single colonies by first scraping a lump of frozen stock cell suspension onto LM agar, dilution streaking and incubating overnight at 37°C. Ten 2.5mm (diameter) colonies were each suspended in lml of pre-warmed SOB medium by vortex mixing, the suspensions pooled in 90ml of pre-warmed SOB medium, and the culture incubated with vigorous aeration at 37°C until the optical absorbance at 550nm was 0.5. The culture was transferred to 2 x 50ml centrifuge tube, chilled on ice/water for 15 min, then the cells harvested by centrifugation at 4°C (2,500 rpm for 12 min). The cell pellet was resuspended in 33ml of ice-cold TEB (sterile 10mM K-MES (Sigma), pH 6.3, 100mM RbCq, 45mM MgCl₂, 10mM CaCl₂, 3mM hexamine cobalt trichloride (Fluka)) and allowed to stand on ice for 15 min. The suspension was centrifuged at 4°C (2,500 rpm for 10 min) and the pellet resuspended in 8ml of ice-cold TEB. To the suspension was added 0.28ml of DMSO (dimethyl sulphoxide, Merck spectroscopic grade) and the mixture swirled and allowed to stand on ice for 5 min, then 0.28ml of 2.25M dithiothreitol (Calbiochem; sterile solution in 40mM potassium acetate, pH6) was added and the cells again swirled and allowed to stand on ice for a further 10 min. Finally, 0.28ml of DMSO was added and mixed in gently and cells again allowed to stand on ice for 5 min.

Samples of cells (210 µl) were transferred into 24 chilled polypropylene tubes with 10 µl of ligated DNA and the tubes were mixed gently and allowed to stand on ice for 30 min. The tubes were then placed in a 42°C water bath for 90 see, transferred to ice/water for 2 min, and 0.8ml of SOC medium (sterile SOB medium containing 20mM glucose) added and the suspension incubated at 37°C for 1h with gentle shaking. The suspensions in 4 tubes were pooled and the cells spread uniformly onto an 82mm diameter nitrocellulose filter disc (Schleicher & Schull) by vacuum filtration on a modified Buchner funnel. The disc was placed onto 2YT agar containing ampicillin (50 µg/ml) and incubated at 37°C overnight. This procedure was repeated for all samples.

Approx. 1,500 colonies of transformed cells were evident on each filter following overnight incubation, giving a "library" complexity of approx. 9,000 for the 240 µl of genomic DNA used for transformation (equivalent to a total potential complexity of 187,500 transformants for the 5ml of ligation mixture). The cells on the six filters were gently suspended in 10ml of SOB medium containing ampicillin (50 µg/ml), the suspension made 20% (v/v) in glycerol, and lml aliquots dispensed into sealed tubes, frozen in liquid nitrogen and stored at -70°C as the genomic library.

### EXAMPLE 2

### Hybridization:

Samples of genomic libraries prepared as in Example 1, each containing approx. 2,000 transformed cells, were diluted in 4ml of 2YT agar/ampicillin at 37°C. Each filter ("master") was then used to prepare a replicate screening filter by placing it, colony surface uppermost, onto a pad of 2 sheets of filter paper (Schleicher & Schull 3MM) and covering it with a fresh nitrocellulose disc (that had been pre-wetted by placing it onto 2YT agar/ampicillin) followed by 2 sheets of 3MM filter paper. The "sandwich" was pressed firmly with a smooth, velvet-covered aluminium block and the upper 2 sheets of 3MM filter paper were then removed. The sandwich of two nitrocellulose discs (master and replica) was "keyed" by piercing it a number of times with a 22g needle attached to a syringe containing waterproof black ink. The filters were then carefully peeled apart and the master filter returned to its 2YT agar/ampicillin plate for storage at 4°C. The replica filter Was placed, colony surface uppermost, onto 2YT agar/ampicillin and incubated at 37°C for approx. 4-6h (until colonies had grown to a diameter of approx. 0.5mm) then transferred to 2YT agar containing chloramphenicol (200 µg/ml) and incubated overnight a 37°C to arrest cell growth without inhibiting plasmid replication (chloramphenicol amplification of plasmid copy number).

The replica filters were then transferred (colonies uppermost) for 5 min onto a pad of 4 sheets of 3MM filter paper that had been wetted uniformly with 0.5M NaOH, 1.5M NaCl. Excess liquid was blotted from the filters by transferring them to dry 3MM filter paper (taking care to maintain them in a horizontal orientation at all times to minimise spreading of plasmid DNA liberated from colonies by alkaline treatment) and the alkaline treatment repeated. The blotted filters were then treated twice successively with 0.5M Tris-HCl (pH 7.5), 1.5M NaCl for 5 min similarly to above. After brief blotting, they were allowed to stand on 3MM filter paper for 30 min. DNA liberated from colonies was fixed to the neutralised filters by heating them in a vacuum oven at 80°C for 2h. Cellular debris was then removed from the filters by washing them in 5 x SSC (1 x SSC is 0.15M NaCl, 15mM Na₃.Citrate), 0.5% SDS at 50°C for 1h with continuous gentle shaking.

The washed filters were prehybridized overnight with continuous gentle agitation at 42°C in hybridization solution (50% (v/v) formamide, 5 x SSPE (1 x SSPE is 0.18M NaCl, 10mM sodium phosphate (pH 7.7), 1mM EDTA), 1% (w/v) SDS, 0.5 mg/ml heat-denatured, sheared salmon sperm DNA (Sigma, dissolved at 10 mg/ml in distilled water and autoclaved), 0.5% (w/v) skim milk powder). The prehybridized filters were transferred to fresh hybridization solution containing radioactive probe DNA at 10 mg/ml and incubated at 42°C overnight with continuous gentle agitation.

The radioactive probe DNA (e.g. BRY1 or OY11.1) was prepared by nick translation. The sample of DNA (0.2 µg) was pre-incubated with 25 mg/ml DNase I (E.coli deoxyribonuclease I) in a total volume of 40 µl containing 50mM Tris-HCl 9 pH 7.5), 7.5mM MgCl₂, 5mM dithiothreitol, 0.1 mg/ml BSA, 20 µM dATP, 20µM dGTP, 20 µM dTTP, and 100 µCi α-³²P dCTP (Amersham; approx. 3,000 Ci/mmol) at 15°C for 20 min. During this time a minute sample (approx. 0.2 µl) was removed and applied to the origin mark on a small sheet (approx. 4 x 8 cm) of PEI-cellulose thin layer chromatography materil (Merck). Following the pre-incubation, DNA polymerase I (Boehringer; approx. 5 units) was added and incubation continued at 15°C for 20 min. The nick translation reaction was stopped by the addition of SDS to 1% (w/v) and EDTA to 20mM, and a second minute sample removed and applied adjacent to the first on the PEI cellulose sheet. The thin layer chromatogram was developed in 0.75M potassium phosphate, pH 3.5, resulting in a separation of dCTP from DNA. The chromatogram was exposed to a sheet of X-ray film (Fuji RX) for 15 min. Development of the X-ray film allowed visual estimation of the efficiency of incorporation of α-³²P dCTP into DNA. This was subsequently quantitated by scintillation counting of appropriate areas of the chromatogram. Before its addition to the hybridization solution, the labelled DNA was recovered by precipitation with ammonium acetate-ethanol as described above then dissolved in distilled water and denatured by heating at 100°C for 5 min.

Following hybridization, the nitrocellulose filters were rinsed briefly in 2 x SSC at room temperature then washed in 2 x SSC, 0.1% (w/v) SDS at room temperature for 15 min. with moderate agitation, followed by washing in 0.5 x SSC, 1% SDS at 68°C for 15 min., again with moderate agitation. They were finally rinsed briefly at room temperature in 0.5 x SSC, blotted dry, wrapped in Gladwrap, and exposed to X-ray film (Fuji RX) with a DuPont Cronex "Lightning Plus" (Trademark) intensifying screen at -70°C for 3 days.

Autoradiography signals on the developed X-ray film correspond to hybridization of recombinant plasmids in discrete colonies with nick translated DNA.

When appropriate "alkaline Southern blotting" was by the method of Reed and Mann (14).

Dot Blot hybridization was as follows: 8 µg of genomic DNA was diluted into 1ml of 0.4M NaOH, 20mM EDTA and heated at 100°C for 10 min. One-half of this sample (0.5ml) was transferred into 0.5ml of 0.4M NaOH, 20mM EDTA, and after mixing 0.5ml of this dilution was again transferred into 0.5ml of 0.4M NaOH, 20mM EDTA, and the serial dilution repeated to generate 7 dilutions for each sample. The first, third, fifth and seventh samples (0.5ml of each, containing respectively 4, 1, 0.25 and 0.0625 µg of DNA) were applied to a sheet of Zeta-Probe membrane using a dot-blot micro-filtration manifold (Bio-Rad). Following vacuum filtration, the sample wells were rinsed with 0.4ml of 0.4M NaOH and the membrane neutralised and hybridized with nick-translated DNA as described above.

### EXAMPLE 3

### Sequence Analysis:

Sequence analysis of the Y-chromosome-specific DNA's was performed as follows:

The inserts in plasmids were excised by digestion with the restriction endonucleases and purified by electrophoresis of the digest in low melting temperature (LMT) agarose (Marine Colloids "Sea-Plaque") followed by excision of an agarose slice containing the band of insert DNA, visualised under ultraviolet illumination following ethidium bromide staining as hereinbefore described. DNA was isolated from the gel slice by melting the agarose at 65°C for 10 min, extracting twice with redistilled phenol, concentrating the solution with repeated butanol extractions, and precipitating the DNA with ammonium acetate/ethanol (procedures as described above).

The recovered inserts were ligated into the plasmid vectors pTZ18u and pTZ19u (Bio-Rad) which had been prepared by digestion with restriction endonucleases BamHI and HindIII followed by treatment with alkaline phosphatase (as described above). Samples of the ligation reactions were used to transform E. coli strain JPA101 (made competent with CaCl₂ treatment as described above) and the cells plated on 2YT agar/ampicillin for overnight incubation at 37°C. Colonies containing the desired recombinant plasmids were identified by restriction enzyme analysis, electrophoresis, alkaline Southern blotting hybridization (described above).

Single colonies were used to inoculate 5ml cultures of 2YT medium/ampicillin (50 µg/ml). The bacteria were infected with bacterophage M13 strain K07 (25 µl of phage stock containing 10⁸ pfu (plaque-forming units) and incubated for 1h at 37°C. Kanamycin was added (70 µg/ml) and incubation continued overnight at 37°C with vigorous aeration. The cultures were then centrifuged and the supernatants removed and treated with ribonuclease A (5 µg/ml) for 30 min. at 37°. Packaged single-stranded plasmid DNA was precipitated by the addition of 0.3ml of polyethylene glycol (PEG) 6,000 (Kock-Light) in 2.5M NaCl. After mixing thoroughly and allowing to stand at room temperature for 15 min, the precipitates were recovered by centrifugation and the supernatants removed completely. The pellets were resuspended in 0.1ml of TE and centrifuged briefly to remove particulate matter, then extracted with phenol and DNA recovered by precipitation from sodium acetate/ethanol as described above. The rinsed and dried pellets were used as templates for Sanger dideoxy sequencing reations (17).

The nucleotide sequences shown in Figures 1-13 are given in single letter code according to standard practice, where individual deoxyribonucleotides are denoted by the following letters: C = deoxycytidine-5'-phosphate; G = deoxyguanosine-5'-phosphate; T = deoxythymidine-5'-phosphate; A = deoxyadanosine-5'-phosphate.

### EXAMPLE 4

### Genetic Sex Determination by PCR:

Table 1 provides the nucleotide sequence and properties of oligonucleotide primers used for genetic sex determination by PCR. The molecular weight of oligonucleotides is calculated from the molecular weight of individual bases as in DNA (sodium salt) with the addition of 1Na and 1OH for the 5'- and 3'- termini.

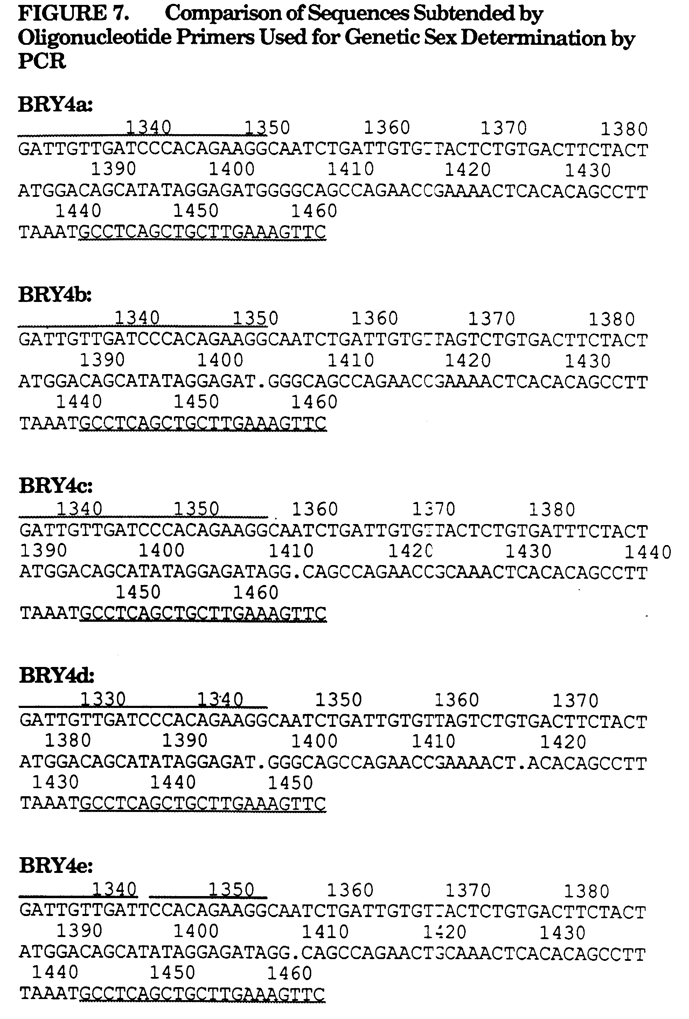

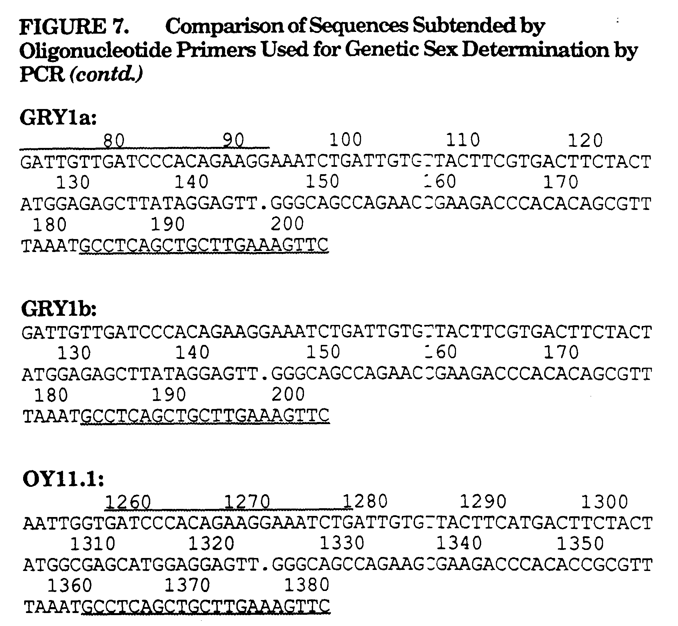

The procedure used for sex determination using PCR is similar for cells derived from all domestic ruminant species (cattle, sheep, goats). The only differences are in the species of origin of the control and test cells and in the sequence of sexing and control oligonucleotide primers. Primers appropriate to each of the three different species are detailed in Figure 7. The example described below is for cattle. Throughout, "tube" refers to the standard 1.5 ml Eppendorf polypropylene microcentrifuge tube with hinged lid.
1. All reagents are prepared previously and aliquots are stored frozen in liquid nitrogen.
(a) Control cells: lymphocytes are prepared from peripheral blood of male and female animals by standard methods and are diluted to a concentration of 2 x 10³/ml in PBS (phosphate buffered saline). The cell suspension is heated at 100°C for 10 min then aliquoted at 20 µl per tube and frozen in liquid nitrogen.
(b) 2 x reaction mix:

| | |
|---|---|
| deionised water | 0.5 µl |
| 4 x buffer | 12.5 µl |
| dNTP mix | 8.0 µl |
| sexing primer SP11 (0.1 mM) | 0.5 µl |
| sexing primer SP12 (0.1 mM) | 0.5 µl |
| control primer CP16 (0.01 mM) | 0.5 µl |
| control primer CP22 (0.01 1M) | 2.0 µl |

The mix is aliquoted for freezing at 25 µl per tube.
Individual components are as follows:

| | |
|---|---|
| 4 x buffer | 200 mM KCl |
| | 10 mM MgCl₂ |
| | 200 mM Tris |
| | 20 mM β-mercaptoethanol |
| | 4% (w/v) dextran T500 (Pharmacia) |
| | pH is adjusted to 8.6 with HCl |

| | |
|---|---|
| dNTP mix | 1.25 mM dATP |
| | 1.25 mM dCTP |
| | 1.25 mM dGTP |
| | 1.25 mM dTTP |

where each of the four deoxynucleotide triphosphate solutions is prepared and frozen separately as a 10 mM stock in 10 mM Tris-HCl/0.5 mM EDTA. The pH of the final dNTP mix is adjusted to 8.5 with NaOH.
"Sexing primers" and "control primers" are described in Table 1 and Figure 7.
"Taq DNA polymerase" is obtained from Biotech International Ltd. (Perth, W.A.).
2. Deionised water (25 µl) is added to negative control tubes (no sample controls).
3. Deionised water (20 µl) is added to male and female control tubes with 5 µl of frozen, heated lymphocyte suspension of the appropriate sex (refer above).
4. Deionised water (20 µl) is added to biopsy sample tubes and to each one is added a 5-10 cell biopsy of an embryo in 5 µl of culture medium.
5. Paraffin oil (65 µl) is added to all tubes.
6. Biopsy sample tubes are immediately heated at 100°C for 10 min. During heating, 2 x reaction mix aliquots sufficient for all tubes are thawed and mixed thoroughly.
7. Following removal of biopsy tubes from 100°C, 25 µl of 2 x reaction mix is added to all tubes (above paraffin layer to minimise possibility of cross-contamination).
8. All tubes are centrifuged for 2-3 see to consolidate and mix aqueous solutions.
9. All tubes are placed in a suitable rack and the rack is transferred to a 94°C water bath for 1 min.
10. The rack is transferred immediately to a 65°C water bath for 2.5 min.
11. Sequential incubation at these two temperatures is continued for a total of 35 cycles.
12. On completion of cycling the rack is removed to room temperature.
13. A sample (40 µl) of the aqueous phase in each tube is removed into a tube containing 11 µl of 5 x stop solution.

| | |
|---|---|
| 5 x stop solution | 50 mM tris-HCl, pH 8.0 |
| | 100 mM EDTA |
| | 1% (w/v) Sarcosyl (Sigma) |
| | 7.5% (w/v) Ficoll 400 (Pharmacia) |
| | 0.05% (w/v) xylene cyanol. |

14. PCR reaction products are analysed by electrophoresis in an agarose gel that has been prepared during preparation of sample biopsies. The system used in the example is the bio-Rad wide mini-subcell with two (tandem) 20-slot sample combs. The gel is 100 ml of 3% (w/v) agarose (Sigma low EEO) in TAE bufer (40 mM Tris, 2 mM EDTA, pH adjusted to 8.0 with acetic acid) containing 0.5 µg/ml ethidium bromide. The tank buffer is identical to the gel buffer.
15. An aliquot (25 µl) of a solution containing 1 µl of DNA fragments of defined size is loaded into a single well of both the upper and lower sets of sample slots in the agarose gel.
DNA electrophoresis size markers: a sample of a plasmid pTZ19u (Bio-Rad) is digested to completion with the restriction endonuclease AluI under conditions recommended by the supplier and is mixed with 1/4 volume of 5 x stop solution (above) which also contains 0.05% (w/v) bromophenol blue.
16. An aliquot (25 µl) of each quenched control and test sample is loaded into a sample slot of the agarose gel.
17. Samples are electrophoresed at 100V for 5 min, then at 200V until the bromophenol blue in the marker slots has migrated 2-3 cm.
18. The gel is removed from the electrophoresis tank and is subjected to 304 nm ultraviolet irradiation on a transilluminator where it is photographed under standard conditions.
19. A band of pink fluorescence indicates the presence of DNA, the size of which is estimated by comparison with the size of the DNA marker fragments.

Negative control samples show no discrete band. Female positive control samples (lymphocytes) show a discrete band at approx. 230 bp. Male positive control samples show two discrete bands: one of approx. 230 bp and one of approx. 130 bp. The former results from PCR amplification of an autosomal satellite sequence common to the genomic DNA of animals of both sexes; the latter results from PCR amplification of a sequence that occurs in multiple copies only in the genomic DNA of male animals.

The sex of an ebryo can be determined if the assay of its biopsy yields a discrete DNA band of approx. 230 bp. If this is the only discrete band observable, the sex of the embryo is female; if in addition to this band there is one of approx. 130 bp, the sex of the embryo is male.

Results of the control sample assays and of the "internal controls" (autosomal sequence amplification in biopxy sample) are critical to interpretation of the data. In the absence of a band at 230 bp in any sample, no determination can be made for that sample. If discrete bands at 230 bp and/or 130 bp are visible in the negative control sample, no determination can be made for any biopsy sample. If either or both of the positive control samples do not yield the anticipated result (refer above), no determination can be made for any biopsy sample.

This example should be interpreted to include the use of alternative procedures for thermal cycling and for PCR product identification. The former can be achieved through the use of programmable heating blocks (e.g. the Thermal Cycler sold by Cetus Perkin-Elmer and the Intelligent Heating Block sold by Hyb-Aid), although their optimal implementation requires modification of cycling temperatures and times according to the thermal gradients generated within the assay tubes. The latter includes the application of nucleic acid hybridization procedures, with or without prior electrophoresis of the PCR reaction products.

Variations in the number of cells used for assay and in the number of temperatures of thermal cycles may, of course, be made without effecting the efficiency of this method for sex determination.

### Example of PCR Application to Cattle Embryo Sexing:

Bovine embryos (late morula state) were asymmetrically divided into four sections. Different cell numbers per section permitted examination of assay sensitivity. Each section was assigned an arbitrary code number and assays were performed in double blind trials. Polymerase chain reaction as described above was used to amplify a bovine male-specific repeated DNA sequence; a control repeated autosomal sequence was amplified simultaneously, allowing for verification of both PCR amplification and successful transfer of biopsy cells into the assay tubes. Each sexing assay trial reuquired approximately 3 hr to complete. Data are presented in the table below.

| TRIAL No. | EMBRYO No. | SECTION No. (a) | | | | ESTIMATED CELL No. (b) | | | | ASSAYED SEX (c) | | | |
|---|---|---|---|---|---|---|---|---|---|---|---|---|---|
| 1 | 1 | 3 | 4 | 18 | 22 | 4 | 6 | 6 | 6 | M | M | M | M |
| 1 | 2 | 1 | 7 | 13 | 15 | 8 | 10 | 8 | 6 | W | M | M | W |
| 1 | 3 | 8 | 17 | 23 | 25 | 12 | 8 | 14 | 4 | F | F | F | F |
| 1 | 4 | 2 | 10 | 29 | 31 | 6 | 7 | 9 | 7 | F | F | F | L |
| 1 | 5 | 11 | 16 | 20 | 28 | 2 | 12 | 8 | 12 | W | M | M | M |
| 1 | 6 | 6 | 9 | 26 | 32 | 6 | 12 | 12 | 12 | F | F | W | F |
| 1 | 7 | 5 | 12 | 27 | 30 | 7 | 10 | 12 | 10 | F | F | F | F |
| 1 | 8 | 14 | 19 | 21 | 24 | 10 | 10 | 10 | 12 | F | F | F | F |
| 2 | 1 | 1 | 13 | 15 | 31 | 12 | 14 | 12 | 10 | M | M | M | M |
| 2 | 2 | 2 | 5 | 11 | 23 | 12 | 14 | 8 | 14 | F | F | F | F |
| 2 | 3 | 6 | 16 | 18 | 26 | 10 | 14 | 7 | 20 | M | M | M | M |
| 2 | 4 | 4 | 17 | 19 | 24 | 14 | 7 | 29 | 15 | F | F | F | F |
| 2 | 5 | 8 | 22 | 16 | 28 | 20 | 20 | 16 | 4 | M | M | M | M |
| 2 | 6 | 20 | 21 | 25 | 29 | 12 | 8 | 15 | 15 | F | F | F | F |
| 2 | 7 | 12 | 14 | 30 | 32 | 15 | 12 | 12 | 6 | M | M | M | M |
| 2 | 8 | 3 | 7 | 9 | 10 | 4 | 9 | 12 | 20 | F | F | F | F |

| | | | | | | | | | | | | | |
|---|---|---|---|---|---|---|---|---|---|---|---|---|---|
| (a) Number of cells per coded section is presented respectively in adjacent columns; | | | | | | | | | | | | | |
| (b) Estimated cell number is ± 20%; | | | | | | | | | | | | | |
| (c) Assay results are presented as F = female, M = male; W = weak signal, L = lost section. | | | | | | | | | | | | | |

Some blood in trial 1 produced signals too weak to interpret. An additional amplification cycle was used in trial 2 resulting i much stronger signals. The results of trial 2 demonstrate unequivocally the accuracy of the assay (p = 6 x 10⁻⁸;χ²).

To date, the assay described has yielded unequivocal data with embryo biopsies and lymphocytes from a variety of domestic breeds of cattle (including Hereford, Poll Hereford, Aberdeen Angus, Holstein/Friesian, Murray Grey, Limousin and Shorthorn) and sheep (Merino, Border Leicester and Dorset); with goats, only lymphocytes have been analysed (angora, Chashmere and feral) with all assays similarly yielding unequivocal accurate data.

Advantages of the PCR technique described herein as a genreal procedure (not confined to sex determination) are:
(i) Constitution of the PCR reaction buffer, in particular the inclusion of the polysaccharide dextran (or similar). This novel component results in three unique advantages: firstly, its inclusion results in more efficient amplification leading to markedly higher sensitivity and specificity. Secondly, it allows the complete reaction mix (including the enzyme Taq DNA polymerase) to be prepared and stored frozen in a single tube resulting in a marked improvement in simplicity and quality control, with reduction in the possibility of on-site contamination. Thirdly, it eliminates the necessity for inclusion of an "inert" protein to stabilise the enzyme. Cognate PCR reaction mixes contain either gelatin or bovine serum albumin; since both are of animal origin there is a strong possibility that they may be contaminated with trace amounts of animal genomic DNA. Our experience has shown that either of these proteins from a variety of sources introduces artifacts into the PCR sexing assays.
(ii) Direct, simple analysis of embryo (any cell) biopsies wherein the sole sample preparation consists of heating the cells in water.
(iii) Judicious selection of oligonucleotide primer sequences such that the melting temperture (Tₘ) of hybrids formed between the primers and their complementary target sequences is approx. 70°C in the solution used for PCR amplication. This has two major effects: firstly, PCR amplification is achieved by cycling for short periods between just two temperatures. The higher temperatures (94°C) causes denaturation of complementary hybrids, and the lower temperature (65°C in the application described above) allows primer annealing to target sequences at a temperature that is near the optimum for activity of Taq NA polymerase (approx. 72°C). Secondly, annealing is done at high stringency, approx. 5°C below the Tₘ for primer target hybrids in the reaction solution. This effectively prohibits non-specific priming, resulting in markedly increased efficiency of specific target amplification with negligible background resulting from non-specific amplification.
(iv) The preceding features, in conjunction with the use of a higher than usual concentration of the enzyme Taq DNA polymerase, allow rapid and simple analysis of PCR reaction products by direct visualisation of amplified DNA following its electrophoresis and staining with a fluorescent dye.

### BCY 11a:

A cDNA library was constructed from the polyadenylated RNA (poly(A)RNA) fraction of bull testis.
1. RNA was extracted from a sample (1 g) of adult bull testis (that had been stored frozen in liquid nitrogen since biopsy) as described by Cathala *et al* (1983).
2. Poly(A)RNA was isolated from this RNA by affinity adsorption to ologo(dT)cellulose according to the procedure described by Nakazato and Edmonds (1974).
3. Single-stranded complementary DNA (ss cDNA) was prepared from a sample of the poly(A)RNA as follows:
A stock of concentrated reaction mix was prepared immediately before use by transferring into a sterile, chilled Eppendorf tube:

| | *Final conc.* |
|---|---|
| 10 µl 1M tris-HCl, pH 8.3 | 50 mM |
| 8 µl 0.5M dithiothreitol | 20 mM |
| 1.5 µl 1M magnesium acetate | 7.5 mM |
| 10 µl 20 mM dATP | 1 mM |
| 10 µl 20 mM dGTP | 1 mM |
| 10 µl 20 mM dTTP | 1 mM |
| 2 µl 20 mM dCTP | 0.2 mM |
| 8 µl 5 mg/ml bovine serum albumin (nuclease-free; Boehringer) | 0.2 mg/ml |

Poly(A)RNA was recovered by centrifugation and the pellet rinsed with 70% ethanol (room temp), dried briefly, and dissolved in sterile water at 1 mg/ml. A template/ primer solution was prepared by transferring to a chilled, sterile Eppendorf tube:

| | |
|---|---|
| 19 µl sterile deionized water | |
| 5 µl 1 mg/ml poly(A) RNA | 0.1 mg/ml |
| 5 µl 0.2 mg/ml oligo(dT) (Boehringer) | 0.02 mg/ml |

The template was denatured by heating the mixture at 80°C for 90 sec then immediately chilling in ice-water.
Remaining stock poly(A)RNA was immediately reprecipitated by adding 1/14 vol of sterile 3M sodium acetate (pH 5.5) and 2 vol of ethanol and storing at -20°C.
To the denatured, chilled template/primer solution was immediately added:

| | |
|---|---|
| 15 µl reaction mix | |
| 1 µl [α-³²P] dCTP | 0.1 mCi/ml |
| (5 mCi/ml, 3,000 Ci/mmol; Amersham Radiochemical Centre) | |

The contents were mixed and centrifuged very briefly, and to it was added:

| | |
|---|---|
| 5 µl AMV reverse transcriptase (12,000-15,000 units/ml; Life Sciences) | 1,200 - 1,500 units/ml |

The contents were again mixed and centrifuged very briefly, then incubated at 42°C for 60 min.
The reaction was quenched by adding:

| | |
|---|---|
| 2 µl 0.5M EDTA | 0.02 M |
| 1 µl 20% SDS | 0.4 % |

The solution was extracted with an equal volume of equilibrated phenol, centrifuged and the upper aqueous phase removed into a clean tube. The aqueous phase was extracted with IAC (isoamyl alcohol:chloroform:1:24), centrifuged and recovered. The cDNA was precipitated by adding:

| | |
|---|---|
| 50 µl 4M ammonium acetate | 2 M |
| 200 µl ethanol | 2 vol |

The solution was frozen solid in liquid nitrogen, then thawed at room temperature and innediately centrifuged for 15 min at 4°C. The pellet was disolved in TE buffer and the cDNA was reprecipitated with ammonium acetate/ethanol as above.
4. Double-stranded cDNA (ds cDNA) was synthesised from the ss cDNA as follows (similar in principle to the procedure of Gubler and Hoffman (1983)):
Immediately before use, a stock of concentrated reaction mix was prepared by transferring into a sterile, chilled Eppendorf tube:

| | *Final conc.* |
|---|---|
| 365 µl sterile deionised water | |
| 20 µl 1M tris-HCl, pH 7.5 | 20 mM |
| 5 µl 1M MgCl₂ | 5 mM |
| 10 µl 1M (NH₄)₂SO₄ | 10 mM |
| 50 µl 2M KCl | 100 mM |
| 10 µl 20 mM dATP | 0.2 mM |
| 10 µl 20 mM dCTP | 0.2 mM |
| 10 µl 20 mM dGTP | 0.2 mM |
| 10 µl 20 mM dTTP | 0.2 mM |
| 10 µl 5 mg/ml bovine serum albumin (nuclease-free; Boehringer) | 0.05 mg/ml |

The doubly-precipitated product of first strand synthesis (ss cDNA/poly(A)RNA hybrid) was collected by centrifugation, rinsed with 70% ethanol, dried briefly, and redissolved in 42 µl of 50 mM Tris-HCl, pH 7.5. This gave a solution containing approx. 50 µg of as cDNA/ml (*i.e.* 100 µg of hybrid/ml).
To a chilled, sterile Eppendorf tube was transferred:

| | |
|---|---|
| 50 µl reaction mix | |
| 42 µl cDNA/RNA template/primer solution | 0.04 mg/ml |
| 1 µl [α-³²P] dCTP (5 mCi/ml, 3,000 Ci/mmol; Amersham Radiochemical Centre) | 0.1 mCi/ml |

The contents were mixed and centrifuged very briefly, and to them was added:

| | |
|---|---|
| 3 µl *E. coli* Ribonuclease H (1,250 units/ml; Boehringer) | 30 units/ml |
| 2.5 µl *E. coli* DNA Polymerase I (5,000 units/ml, nuclease-free; Boehringer) | 125 units/ml |

The contents were again mixed and centrifuged very briefly, then incubated for 1 h at 12°C followed by 1 h at 16°C. To the tube was then added:

| | |
|---|---|
| 1.5 µl *E. coli* Ribonuclease H (1,250 units/ml; Boehringer) | 30 units/ml |

and incubation was continued at 16°C for a further 30 min.
The termini of the ds cDNA were then 'blunt-ended' by limited digestion with S1 nuclease:
To the tube was added 300 µl of freshly prepared
S1 nuclease mix, containing
54 mM potassium acetate, pH 4.6
450 mM NaCl
1.8 mM ZnSO₄
9% (v/v) glycerol
90 units/ml S1 nuclease (Sigma)
and the tube was incubated for 60 min at 10°C.
Reaction was quenched by adding:

| | |
|---|---|
| 16 µl 0.5M EDTA | 0.02 M |
| 8 µl 20% Sarcosyl | 0.4 % |

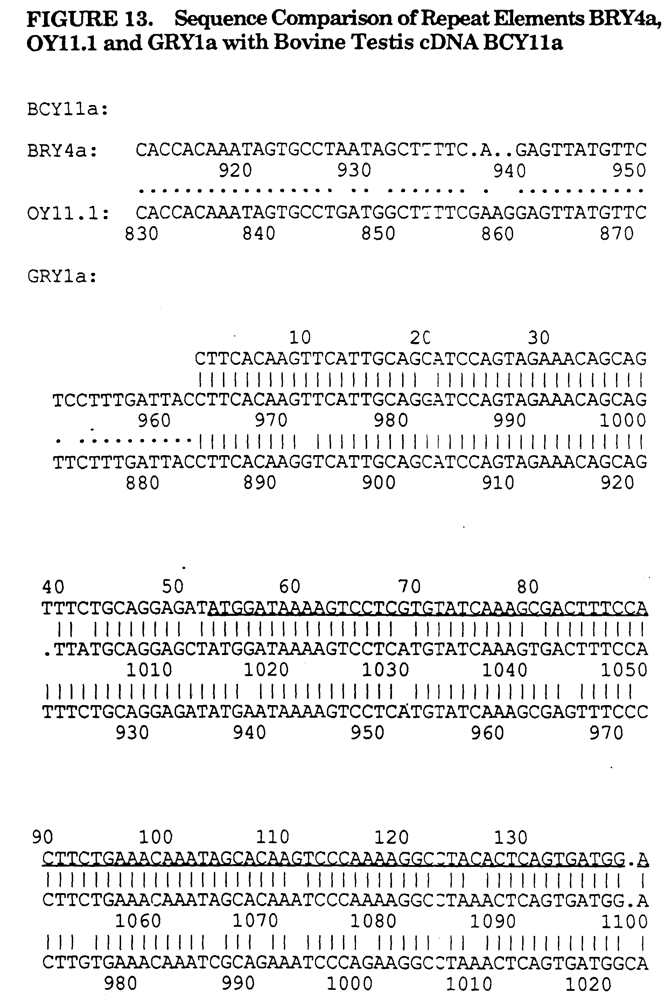

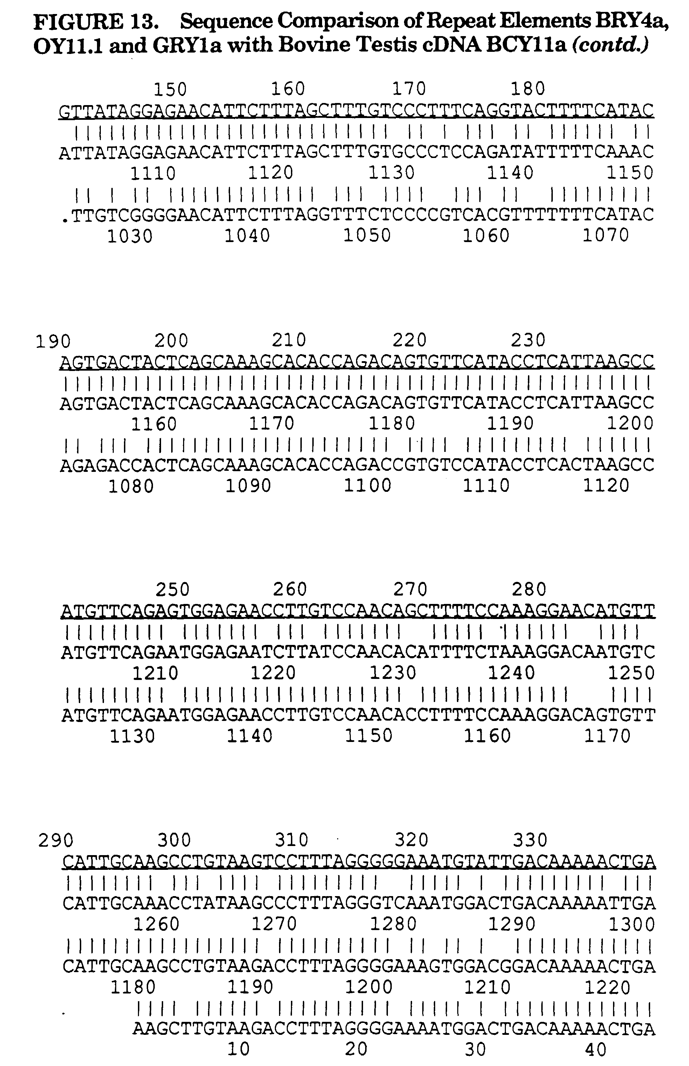

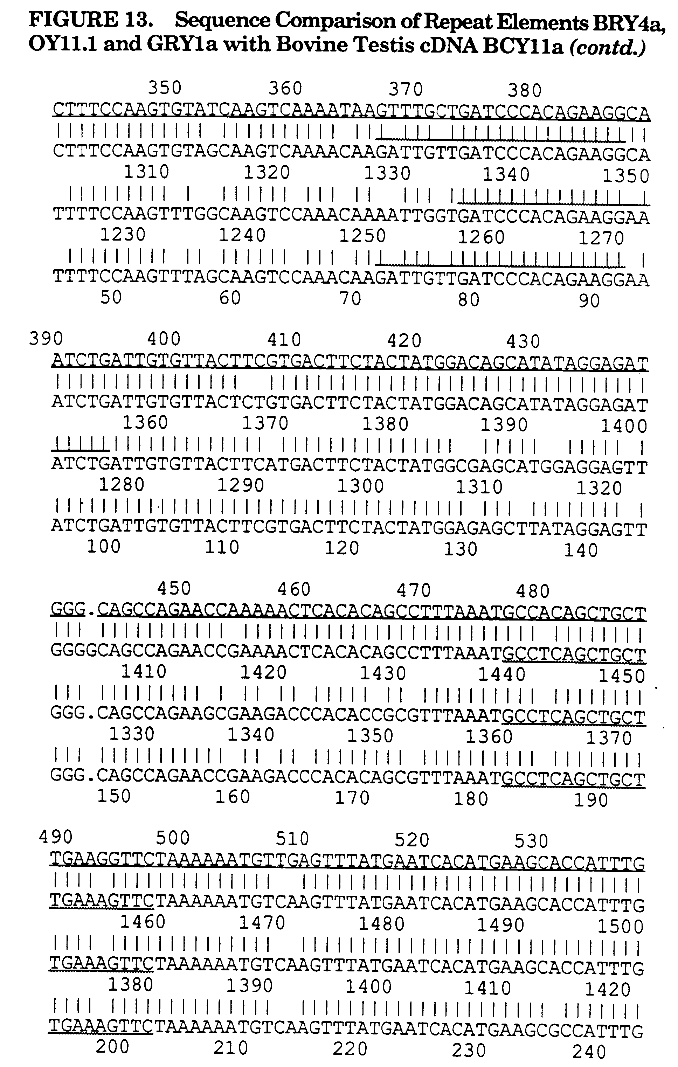

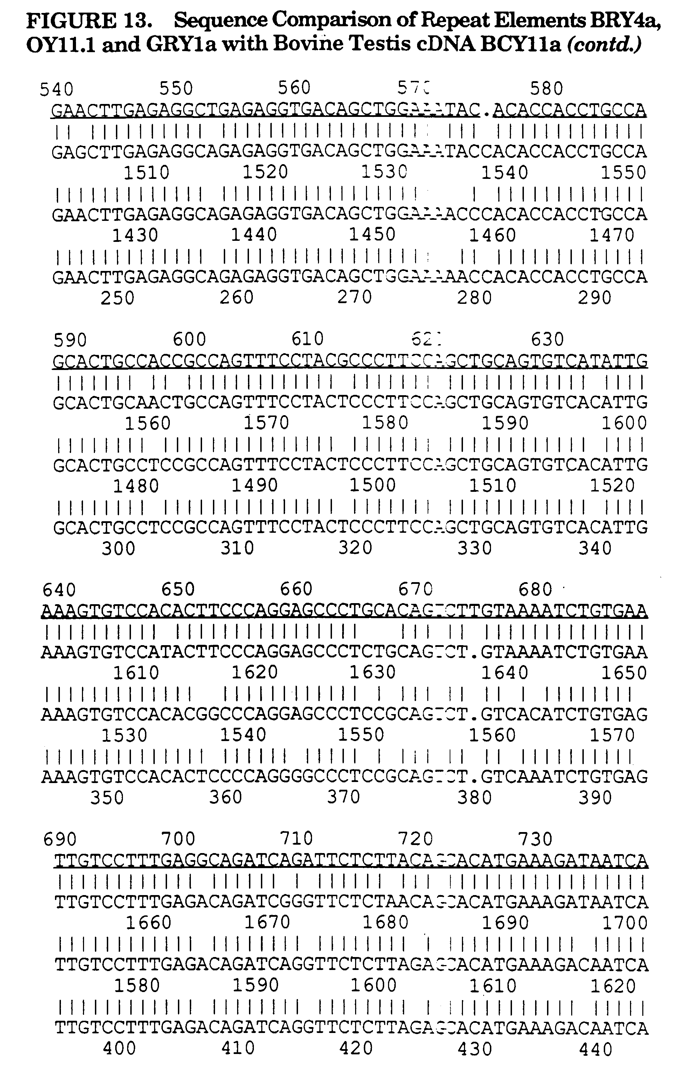

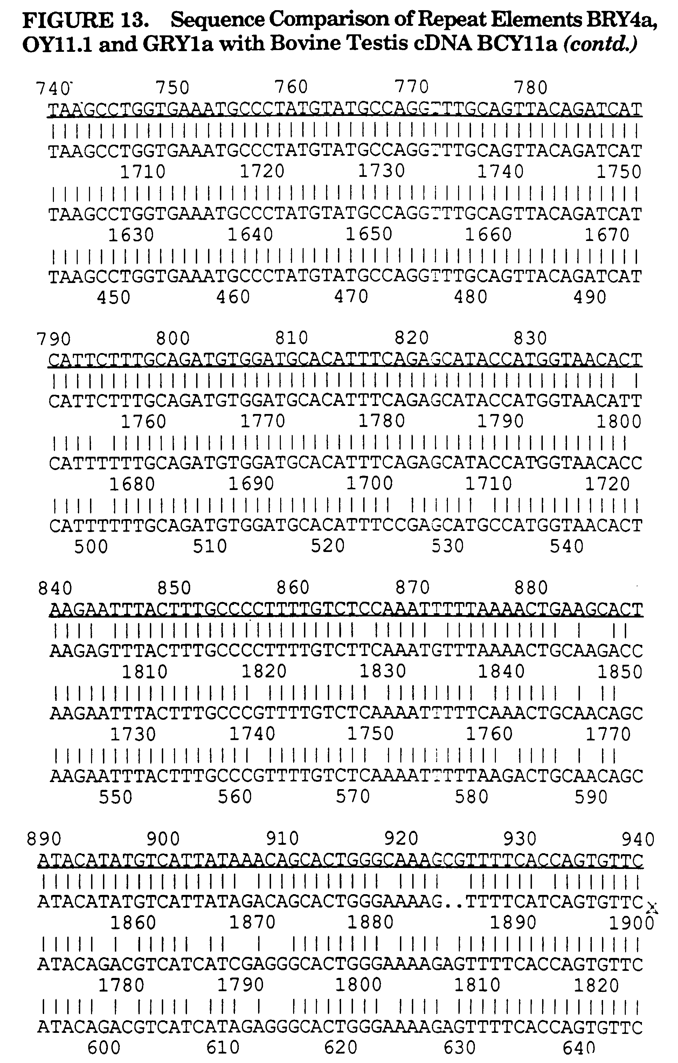

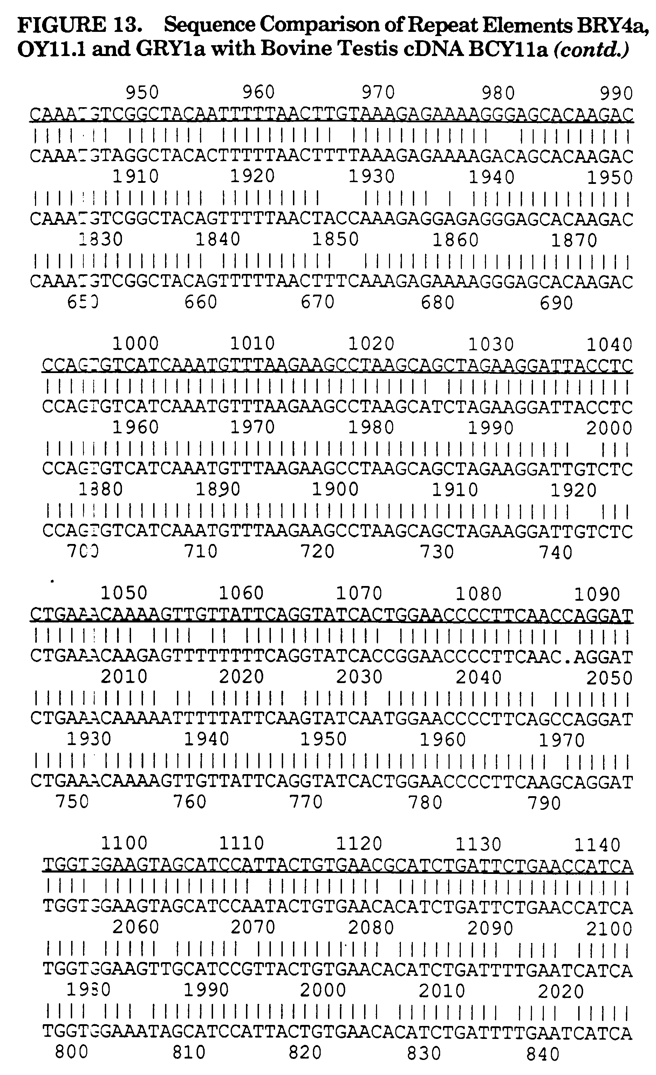

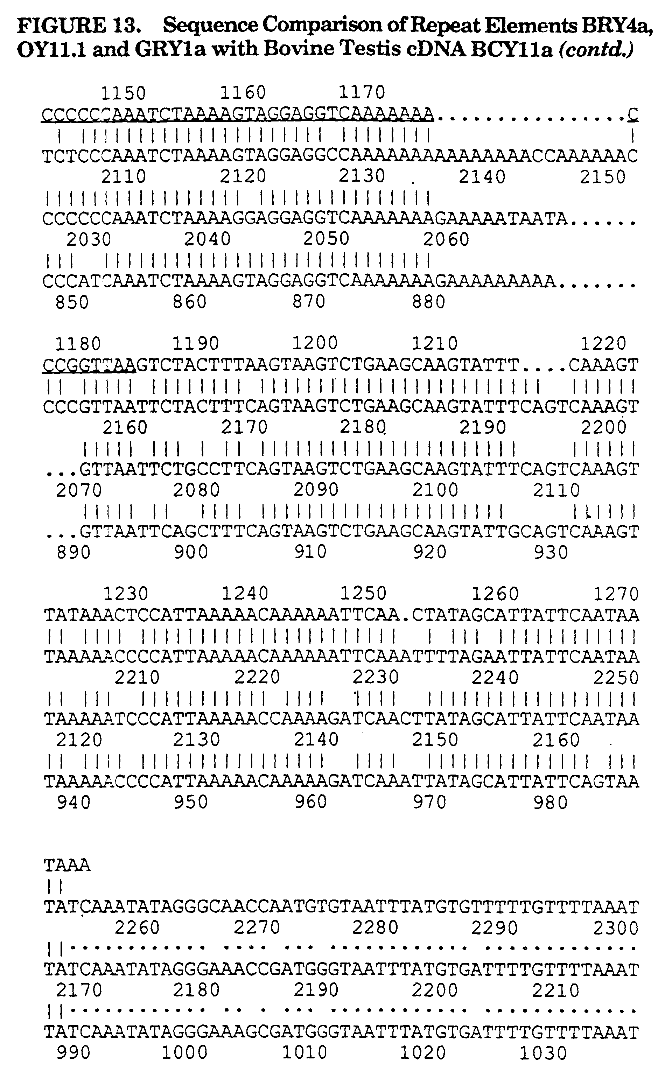

The solution was extracted with an equal volume of phenol and centrifuged to separate the phases. The upper aqueous phase was removed into a clean tube, extracted with IAC and centrifuged.
The aqueous phase was removed and concentrate to approx. 100 µl by repeated extraction with butan-1-ol. Excess butanol was removed by extraction with ether and the ds cDNA precipitated as above.
The pellet was dissolved in TE buffer and reprecipitated with ammonium acetate/ethanol. The cDNA was collected by centrifugation, rinsed with 70% ethanol, dried *in vacuo,* and dissolved in 50 µl of TE.
5. A sample (5 µg) of the plasmid vector BlueScript M13+ (Stralagene) was digested with the restriction endonuclease *SmaI* and treated with alkaline phosphatase. The blunt-ended, linearised, phosphatased vector was then purified by electrophoresis in a gel of low melting temperature agarose (Marine Colloids 'Sea Plaque' agarose) from which it was recovered by excision of a gel slice which was melted and extracted twice with phenol. The solution was concentrated with butanol and the purified DNA precipitated from the aqueous phase with acetate and ethanol as above.
6. A sample of the blunt-ended ds cDNA (approx*.* 0.2 µg) was treated with *E. coli* DNA polymerase I (Boehringer) and T4 polynucleotide kinase (Boehringer) under standard conditions (including the four dNTPs and ATP) to ensure that all fragments terminated in ligatable blunt ends. Following purification by phenol extraction and acetate/ethanol precipitation as described above, this material was ligated with prepared vector DNA.
Ligation mix (50 µl) was prepared as:
0.2 µg ds cDNA
1 µg linearised BlueScript vector DNA
50 mM Tris-HCl, pH 7.5
10 mM MgCl₂
7.5 mM dithiothreitol
100 µg/ml bovine serum albumin (nuclease-free; Boehringer)
1 mM ATP
1 mM spermidine (Sigma)
4 units T4 DNA ligase (BRL)
4 units T4 RNA ligase (BRL)
and incubated at 8°C for three days. The product was purified by phenol extraction and acetace/ethanol precipitation as above, and redissolved in 10 µl of TE.
7. The purified ligation product was used to transforms *E. coli* MC1061.1 cells by electrotransformation (Bill Dower, Bio-Rad; personal communication) .
Competent cells were prepared by growing *E. coli* MC1061.1 in LB-broth with vigorous shaking at 37°C, to A₆₀₀ of 0.5 to 1. The culture flask was chilled briefly on ice/water and the cells were harvested by centrifugation at 4,000 x gₘₐₓ for 15 min at 4°C.
The cell pellet from 1 litre of culture was resuspended in 1 litre of cold deionised water and centrifuged as above, then resuspended in 500 ml of cold deionised water and recentrifuged. It was then resuspended in 20 ml of cold 10% glycerol, recentrifuged, and resuspended to approx. 3 ml (final volume) in cold 10% glycerol. This represented approx.300-fold concentration from the culture, to a density of approx. 3 x 10¹⁰ cells/ml. The final suspension was distributed in 100 µl aliquots, frozen in liquid nitrogen and stored at -70°C
For transformation, an aliquot of the concentrated cells was thawed at room temperature and placed on ice. A sample (40 µl) was removed into a chilled Eppendorf tube and to this was added 1 µl of purified ligated DNA and the suspension mixed vigorously by flicking the tube.
The cell/DNA mixture was transferred into an ice cold 'GenePulser' (Bio-Rad) micro-cuvette (2 mm path length), and the cuvette was positioned in the chilled safety chamber of the GenePulser. The GenePulser was modified to include a 20 Ω resistor in series and a 200 Ω resistor in parallel with the sample. The instrument was set to 25 µF capacitance, 2.5 KV and pulsed once, generating a pulse of 12.5 KV/cm with a time constant of slightly less than 5 msec.
Immediately following the pulse, the cell suspension was removed from the cuvette and mixed into 1-2 ml of SOC medium (2% tryptone, 0.5% yeast extract, 10 mM NaCl, 2.5 mM KCl, 20 mM MgSO₄, 20 mM glucose) in a 17 x 100 mm polypropylene tube. The suspension was incubated with shaking (@ 225 rpm) for 1 h at 37°C.
8. The cells were plated on selective medium (LB agar containing 20 µg/ml ampicillin) and incubated overnight at 37°C. The resultant colonies (approx. 50,000 total) were suspended in 20 ml of LB broth containing 20% glycerol and distributed in 1 ml aliquots into sealed tubes which were frozen in liquid nitrogen and stored at -70°C. This comprised the amplified library of adult bull testis cDNA.
9. Approx. 50,000 cells of the amplified library were spread onto six nitrocellulose filters on LB agar plates containing 20 µg/ml ampicillin and the plates incubated overnight at 37°C. Colonies were replicated for hybridisation screening according to the method of Hanahan and Messelson and duplicate replica filters were screened by hybridisation with plasmid pOY11.1.
Positive colonies (three) were isolated by repeated screening and subcloning. Plasmids contained within them were isolated according to Maniatis *et al* (1982) and were linearised by digestion with the restriction endonuclease *BamHI* and subjected to electrophoresis in an agarose gel.
The insert of the plasmid which contained the largest insert of the three recombinants recovered (approx. 1,400 bp; pBCY11a) was subjected to DNA sequence analysis (Figure 13), The strong sequence similarity between this bovine cDNA (BCY11a) and the ovine genomic repeat element OY11.1 that led to its identification by hybridisation is evident from comparison of the two base sequences in Figure 13.
By definition, BCY11a is the coding sequence of a structural gene that is transcribed in adult bull testis, a conclusion that is reinforced by its including a long open reading frame (Figure 13). While its sequence is very similar to the sequence of genomic repeat elements associated with the DNA of male cattle, sheep and goats, it nevertheless shows significant differences from those sequences (Figure 13). These include not only base substitutions but multiple frame shifts (deletions and insertions) which destroy the integrity of the open reading frame(s). From this it must be concluded that the similar genomic repeat elements that we have sequenced to date are not functional genes. This is reinforced by the linear similarity between BCY11a and the sequenced genomic elements: the majority of structural genes contain non-coding regions (introns) interspersed with coding regions (exons).

### EXAMPLE 6:

### Universal Sexing Primers:

Two oligomers designated USP1 (5'-CCCTTCCAGCTGCAGTGTCA-3'; n=20; T_{H} = 59°C; A₂₆₀ = 192.9) and USP2 (5'-GATCTGTAACTGCAAACCTGGC-3'; n = 22; T_{H} - 61°C; A₂₆₀ = 236.2) were selected for total homology between cloned BCY11a cDNA and all sequenced cattle, sheep and goat genomic repeats (see Figure 13). These oligomers are used for PCR sexing of all three domestic ruminant species (cattle, sheep, goats). Amplification with these primers of target sequences is possible at 68-70°C as well as 65°C and the total length of amplified segment is 173 base pairs.

It should be noted that the assay developed depends for its sensitivity on the detection of DNA sequences that are repeated hundreds of times in the DNA of the male genome, so that not one but hundreds of target sequences in each cell of the assay sample are amplified. Since non-functional repeated DNA elements tend to display substantially sequence variations (refer to Figure 13), it is important to select as targets particular sequences in which variation is minimal. The identification of such regions, which are identical not only between non-functional genomic repeats but also with the analogous region of a functional gene from which those repeats presumably arose, suggests both the possibility of a cellular mechanism that minimises variation in these regions and the probability of these regions being strongly conserved within and between domestic ruminant species.

### REFERENCES

1. Kunkel et al., Science 191, 1189-1190 (1976)
2. Bishop et al., Nature 303, 831 (1983)
3. Barone, et al. Nucleic Acids Res. 12, 4051-4061 (1984)
4. Caruthers, et al. Cold Spring Harbor Sym. Quant. Bio. 47, 411-418 (1982)
5. Cathala, et al. (1983) DNA 2, 329-335.
6. Rigy, et al., J. Mol. Biol. 113, 237-251 (1977)
7. Taylor, et al. Biochim. Biophys. Acta 442, 324-330 (1976)
8. Melton, et al. Nucleic Acids Res. 12, 7035-7056 (1984)
9. Lowary, et al. "Structure and Dynamics of RNA, NATO ASI Series 110" pp. 69-76, ed. van Knippeuberg, P.H. and Hilbers, C.W., Plenum Press, New York (1986)
10. Maniatis et al. (1982) Molecular Cloning, A Laboratory Manual, Cold Spring Harbor Laboratory
11. Swanstrom, R. and Shank, P.R. Anal. Biochem. 86, 184-192 (1978)
12. Saiki, et al. Science 230, 1350-1354 (1986)
13. Saiki, et al. Nature 324, 163-166 (1986)
14. Lederman et al. Anal. Biochem. 117, 158-163 (1981)
15. Reed, K.C. and Mann, D.A. Nucl. Acids Res. 13, 7207-7221 (1985)
16. Norgard, M.V., Tocci, M.J. and Monahan, J.J. (1980) J. Biol. Chem. 255, 7665-7672
17. Dagert, M. and Ehrlich, S.D. (1979) Gene 6, 23-28
18. Sanger, F., Nicklen, S. and Coulson, R. (1977) Proc. Natl. Acad Sci. USA 74, 5463-5467.
19. Gubler, U. and Hoffman, B.J. (1983) Gene 25, 263-269.
20. Maniatis, T., Fritxch, E.F. and Sambrook, J. (1982) "Molecular Cloning. A Laboratory Manual" (Cold Spring Harbor Laboratory) pp. 368-369.
21. Nakazato, H. and Edmonds, M. (1974) Meths. Enzymol. 29, 431-443.

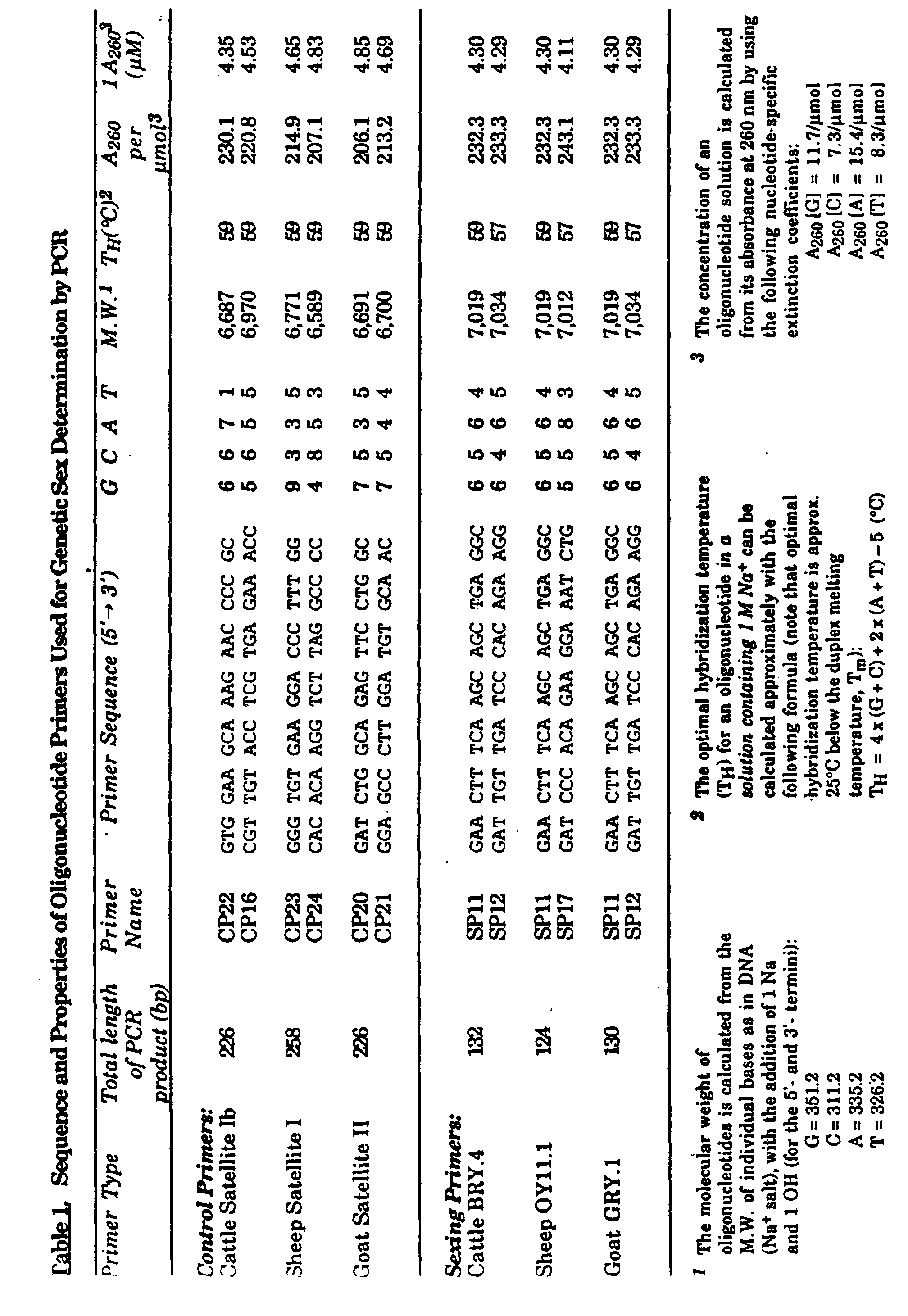

## Claims

1. A nucleic acid isolate capable of hybridizing only to Y-chromosome-specific DNA sequences of cattle, sheep and goats comprising one or both strands of:
OY1.1, a 3142 base pair BamHI-EcoRI sequence as shown in Figure 1;
OY4.1, a 2552 base pair Hind III-BamHI sequence as shown in Figure 2;
OY4.2, a 1076 base pair BamHI-BamHI sequence as shown in Figure 2;
OY9.1, a 2555 base pair Hind III-BamHI sequence as shown in Figure 3;
OY9.2, a 1076 base pair BamHI-BamHI sequence as shown in Figure 3;
OY9.5, a 6933 base pair EcoRI-BamHI sequence as shown in Figure 3;
OY11.1, a 3983 base pair EcoRI-EcoRI sequence as shown in Figure 4;
BRY4a, a sequence as shown in Figure 5;
BRY4b, a sequence as shown in Figure 7;
BRY4c, a sequence as shown in Figure 6;
BRY4d, a sequence as shown in Figure 7;
BRY4e, a sequence as shown in Figure 7;
BRY4a(d), a 545 base pair sequence as shown in Figure 11;
BRY4c(i), a 484 base pair sequence as shown in Figure 12;
GRY.1a, a sequence as shown in Figure 10;
GRY.1a(a), a 384 base pair sequence as shown in Figure 8;
GRY.1b, a sequence as shown in Figure 10;
GRY.1b(a), a 448 base pair sequence as shown in Figure 9; or
BCY11a, a sequence as shown in Figure 13;
or any contiguous portion of 12 or more nucleotides thereof.

2. A nucleic acid isolate according to claim 1 comprising RNA corresponding to one or both strands of the said DNA sequences.

3. A nucleic acid isolate according to claim 1 or 2, which is labelled with a detectable marker.

4. A nucleic acid isolate according to claim 3 wherein the detectable marker is selected from ³²P, ¹⁴C, ³H, ¹²⁵I, biotin or bromodeoxyuridine.

5. A replicable vector comprising a nucleic acid isolate according to claim 1 or 2 or any contiguous portion of 12 or more nucleotides thereof.

6. A method for the determination of the sex chromosome constitution of a tissue or cell sample and hence the sex of said sample comprising, isolating DNA from said tissue or cell sample, immobilising the isolated DNA onto a support matrix, hybridizing the immobilised DNA with a nucleic acid isolate as claimed in claim 3 or 4, under conditions enabling the nucleic acid isolate to bind two complementary sequences, washing unbound nucleic acid isolate from the support matrix, and subsequently detecting nucleic acid isolate binding to DNA bound to the support matrix.

7. A method for determining the presence or absence of a Y-chromosome in fixed cells or interphase or metaphase chromosome spreads comprising, hybridizing said fixed cells or interphase or metaphase chromosomes with a nucleic acid isolate as claimed in claim 3 or 4, under conditions enabling the nucleic acid isolate to bind to complementary sequences, washing away unbound nucleic acid isolate, and detecting binding of the nucleic acid isolate to the fixed cells or metaphase chromosomes.

8. A method for determining the sex chromosome constitution of a tissue or cell sample comprising, isolating DNA from the tissue or cell sample and denaturing the isolated DNA to separate the respective coding and non-coding strands, annealing the denatured DNA with one or more synthetic oligonucleotides corresponding to 12 or more nucleotides of a nucleic acid isolate according to claim 1 or 2, incubating the annealed DNA with DNA polymerase to extend the polynucleotide(s) through the nucleic acid isolate DNA sequence if present in the tissue or cell sample; repeating this sequence as many times as is desired to amplify levels of the nucleic acid isolate and subsequently detecting the nucleic acid isolate sequences in the amplified sample, either by:
(a) immobilizing the amplified DNA onto a support matrix, hybridizing the immobilized DNA with a labelled nucleic acid isolate according to claim 3 or 4 under conditions enabling the nucleic acid isolate to bind to complementary sequences, washing unbound nucleic acid isolate from the support matrix, and subsequently detecting binding of the nucleic acid isolate to DNA bound to the support matrix; or
(b) where labelled nucleotide precursors are included in the incubation with DNA polymerase, fractionating the sample by electrophoresis in a gel matrix, and detecting labelled nucleic acid isolate DNA sequences.

9. A method as claimed in any one of claims 6 to 8 where the tissue or cell sample is obtained from an embryo, a foetus, or from sperm or a fraction of sperm.

10. A kit for detecting the presence or absence of Y-chromosome specific sequences in a tissue or cell sample, comprising a nucleic acid isolate according to any one of claims 1 to 4.

11. A kit as claimed in claim 10, additionally containing buffers for the dilution of reagents.

12. An oligomer for PCR sexing of cattle, sheep and goats comprising the sequence 5'-CCCTTCCAGCTGCAGTGTCA-3'.

13. An oligomer for PCR sexing of cattle, sheap and goats comprising the sequence 5'-GATCTGTAACTGCAAACCTGGC-3'.

## Patentansprüche

1. Isolierte Nucleinsäure, die nur mit Y-Chromosom-spezifischen DNA-Sequenzen von Rindern, Schafen und Ziegen hybridisieren kann, enthaltend einen oder beide Stränge von:
OY1.1, eine BamHI-EcoRI-Sequenz mit 3142 Basenpaaren, wie in Figur 1 gezeigt;
OY4.1, eine wie in Figur 2 gezeigte Hind III-BamHI-Sequenz mit 2552 Basenpaaren ;
OY4.2, eine wie in Figur 2 gezeigte BamHI-BamHI-Sequenz mit 1076 Basenpaaren;
OY9.1, eine wie in Figur 3 gezeigte Hind III-BamHI-Sequenz mit 2555 Basenpaaren;
OY9.2, eine wie in Figur 3 gezeigte BamHI-BamHI-Sequenz mit 1076 Basenpaaren;
OY9.5, eine wie in Figur 3 gezeigte EcoRI-BamHI-Sequenz mit 6933 Basenpaaren;
OY11.1, eine wie in Figur 4 gezeigte EcoRI-EcoRI-Sequenz mit 3983 Basenpaaren;
BRY4a, eine wie in Figur 5 gezeigte Sequenz;
BRY4b, eine wie in Figur 7 gezeigte Sequenz;
BRY4c, eine wie in Figur 6 gezeigte Sequenz;
BRY4d, eine wie in Figur 7 gezeigte Sequenz;
BRY4e, eine wie in Figur 7 gezeigte Sequenz;
BRY4a(d), eine wie in Figur 11 gezeigte Sequenz mit 545 Basenpaaren;
BRY4c(i), eine wie in Figur 12 gezeigte Sequenz mit 484 Basenpaaren;
GRY.1a, eine wie in Figur 10 gezeigte Sequenz;
GRY.1a(a), eine wie in Figur 8 gezeigte Sequenz mit 384 Basenpaaren;
GRY.1b, eine wie in Figur 10 gezeigte Sequenz;
GRY.1b(a), eine wie in Figur 9 gezeigte Sequenz mit 448 Basenpaaren;
oder
BCY11a, eine wie in Figur 13 gezeigte Sequenz;
oder irgendeinen nicht unterbrochenen Abschnitt von 12 oder mehr Nucleotiden daraus.

2. Isolierte Nucleinsäure gemäß Anspruch 1, die die zu einem oder beiden Strängen der DNA-Sequenzen korrespondierende RNA enthält.

3. Isolierte Nucleinsäure gemäß Anspruch 1 oder 2, die mit einem nachweisbaren Marker markiert ist.

4. Isolierte Nucleinsäure gemäß Anspruch 3, wobei der nachweisbare Marker unter ³²P, ¹⁴C, ³H, ¹²⁵I, Biotin oder Bromdeoxyuridin ausgewählt ist.

5. Replizierbarer Vektor, der eine isolierte Nucleinsäure gemäß Anspruch 1 oder 2 oder irgendeinen nicht unterbrochenen Abschnitt von 12 oder mehr Nucleotiden daraus enthält.

6. Verfahren zur Bestimmung der Struktur des Geschlechtschromosoms eines Gewebes oder einer Zellprobe und damit des Geschlechts dieser Probe, welches das Isolieren von DNA aus dem Gewebe oder der Zellprobe, Immobilisieren der isolierten DNA auf eine Trägermatrix, Hybridisieren der immobilisierten DNA mit isolierter Nucleinsäure gemäß Anspruch 3 oder 4 unter Bedingungen, die eine Bindung der isolierten Nucleinsäure an komplementäre Sequenzen erlauben, Waschen der nicht gebundenen isolierten Nucleinsäure von der Trägermatrix und nachfolgendes Nachweisen der Bindung der isolierten Nucleinsäure an die an die Trägermatrix gebundene DNA umfaßt.

7. Verfahren zur Bestimmung des Vorhandenseins oder Nichtvorhandenseins eines Y-Chromosoms in fixierten Zellen oder in ausgebreiteten Interphasen- oder Metaphasenchromosomen, das Hybridisieren der fixierten Zellen oder der Interphasen- oder der Metaphasenchromosomen mit einer isolierten Nucleinsäure gemäß Anspruch 3 oder 4 unter Bedingungen, die eine Bindung der isolierten Nucleinsäure an komplementäre Sequenzen erlauben, Abwaschen der nicht gebundenen isolierten Nucleinsäure und Nachweisen der Bindung der isolierten Nucleinsäure an die fixierten Zellen oder Metaphasenchromosomen umfaßt.

8. Verfahren zur Bestimmung der Struktur des Geschlechtschromosoms eines Gewebes oder einer Zellprobe, welches das Isolieren der DNA aus dem Gewebe oder der Zellprobe und Denaturieren der isolierten DNA, um die kodierenden und nicht-kodierenden Stränge zu trennen, Assoziieren der denaturierten DNA mit einem oder mehreren synthetischen Oligonucleotiden, die 12 oder mehr Nucleotiden der isolierten Nucleinsäure gemäß Anspruch 1 oder 2 entsprechen, Inkubieren der assoziierten DNA mit DNA-Polymerase, um das oder die Polynucleotid(e), wenn im Gewebe oder der Zellprobe vorhanden, mittels der isolierten Nucleinsäure-DNA-Sequenz zu verlängern; Wiederholen dieser Abfolge sooft wie gewünscht, um die isolierte Nucleinsäure zu amplifizieren, und nachfolgendes Nachweisen der isolierten Nucleinsäuresequenzen in der amplifizierten Probe entweder durch:
(a) Immobilisieren der amplifizierten DNA auf eine Trägermatrix, Hybridisieren der immobilisierten DNA mit einer markierten isolierten Nucleinsäure gemäß Anspruch 3 oder 4 unter Bedingungen, die eine Bindung der isolierten Nucleinsäure an komplementäre Sequenzen erlauben, Waschen der ungebundenen isolierten Nucleinsäure von der Trägermatrix und nachfolgendes Nachweisen der Bindung der isolierten Nucleinsäure an die an die Trägermatrix gebundene DNA; oder,
(b) wenn markierte Nucleotidvorläufer während der Inkubation mit DNA-Polymerase vorhanden sind, durch Fraktionieren der Probe mittels Elektrophorese in einer Gelmatrix und Nachweisen der markierten isolierten Nucleinsäure-DNA-Sequenzen,
umfaßt.

9. Verfahren gemäß einem der Ansprüche 6 bis 8, wobei das Gewebe oder die Zellprobe von einem Embryo, einem Fötus oder von Sperma oder einer Spermafraktion erhalten wird.

10. Kit zum Nachweisen des Vorhandenseins oder Nichtvorhandenseins von Y-Chromosom-spezifischen Sequenzen in einem Gewebe oder einer Zellprobe, enthaltend isolierte Nucleinsäure gemäß einem der Ansprüche 1 bis 4.

11. Kit gemäß Anspruch 10, das zusätzlich Puffer zur Verdünnung der Reagenzien enthält.

12. Oligomer zur Geschlechtsbestimmung von Rindern, Schafen und Ziegen mittels PCR, enthaltend' die Sequenz 5'-CCCTTCCAGCTGCAGTGTCA-3'.

13. Oligomer zur Geschlechtsbestimmung von Rindern, Schafen und Ziegen mittels PCR, enthaltend die Sequenz 5'-GATCTGTAACTGCAAACCTGGC-3'.

## Revendications

1. Isolat d'acide nucléique ne pouvant s'hybrider qu'à des séquences d'ADN spécifiques du chromosomes Y de bovins, moutons et chèvres, comprenant l'un ou les deux brins de :
OY1.1, séquence BamHI-EcoRI de 3142 paires de bases présentée dans la figure 1 ;
OY4.1, séquence HindHI-BamHI de 2552 paires de bases présentée dans la figure 2 ;
OY4.2, séquence BamHI-BamHI de 1076 paires de bases présentée dans la figure 2 ;
OY9.1, séquence HindHI-BamHI de 2555 paires de bases présentée dans la figure 3 ;
OY9.2, séquence BamHI-BamHI de 1076 paires de bases présentée dans la figure 3 ;
OY9.5, séquence EcoRI-BamHI de 6933 paires de bases présentée dans la figure 3 ;
OY11.1, séquence EcoRI-EcoRI de 3983 paires de bases présentée dans la figure 4 ;
BRY4a, séquence présentée dans la figure 5 ;
BRY4b, séquence présentée dans la figure 7 ;
BRY4c, séquence présentée dans la figure 6 ;
BRY4d, séquence présentée dans la figure 7 ;
BRY4e, séquence présentée dans la figure 7 ;
BRY4a(d), séquence de 545 paires de bases présentée dans la figure 11 ;
BRY4c(i), séquence de 484 paires de bases présentée dans la figure 12 ;
GKY.1a, séquence présentée dans la figure 10 ;
GRY.1a(a), séquence de 384 paires de bases présentée dans la figure 8 ;
GRY.1b, séquence présentée dans la figure 10 ;
GRY.1b(a), séquence de 448 paires de bases présentée dans la figure 9 ; ou
BCY11a, séquence présentée dans la figure 13 ;
ou l'une quelconque de leurs portons contiguës de 12 nucléotides ou plus.

2. Isolat d'acide nucléique conforme à la revendication 1, comprenant de l'ARN correspondant à l'un ou aux deux brins desdites séquences d'ADN.

3. Isolat d'acide nucléique conforme à la revendication 1 ou à la revendication 2, qui est marqué par un marqueur détectable.

4. Isolat d'acide nucléique conforme à la revendication 3, dans lequel le marqueur détectable est choisi parmi ³²P, ¹⁴C, ³H, ¹²⁵I, la blotine ou la bromodésoxyuridine.

5. Vecteur de réplication comprenant un isolat d'acide nucléique conforme à la revendication 1 ou à la revendication 2, ou l'une quelconque de ses portions contiguës de 12 nucléotides ou plus.

6. Procédé de détermination de la constitution du chromosome sexuel d'un échantillon de tissu ou de cellules, et par conséquent du sexe dudit échantillon, comprenant l'isolement de l'ADN dudit échantillon de tissu ou de cellules, l'immobilisation de l'ADN isolé sur une matrice support, l'hybridation de l'ADN immobilisé avec un isolat d'acide nucléique tel que revendiqué dans la revendication 3 ou la revendication 4, dans des conditions permettant à l'isolat d'acide nucléique de se fixer aux deux séquences complémentaires, le lavage de l'isolat d'acide nucléique non fixé de la matrice de support, et ensuite la détection de l'isolat d'acide nucléique fixé à l'ADN lié à la matrice support.

7. Procédé de détermination de la présence ou de l'absence d'un chromosome Y dans des cellules fixées ou dans des étalements de chromosomes en interphase ou en métaphase, comprenant l'hybridation desdites cellules fixées ou desdits chromosomes en interphase ou en métaphase avec un isolat d'acide nucléique tel que revendiqué dans la revendication 3 ou la revendication 4, dans des conditions permettant à l'isolat d'acide nucléique de se fixer aux séquences complémentaires, l'élimination par lavage de l'isolat d'acide nucléique non fixé, et la détection de la fixation de l'isolat d'acide nucléique aux cellules fixées ou aux chromosomes en métaphase.

8. Procédé de détermination de la constitution du chromosome sexuel d'un échantillon de tissu ou de cellules, comprenant l'isolement de l'ADN de l'échantillon de tissu ou de cellules et la dénaturation de l'ADN isolé afin de séparer les brins codants et non codants respectifs, l'hybridation de l'ADN dénaturé avec un ou plusieurs oligonucléotides synthétiques correspondant à 12 nucléotides ou plus d'un isolat d'acide nucléique conforme à la revendication 1 ou à la revendication 2, l'incubation de l'ADN hybridé avec de la polymérase d'ADN afin d'allonger le ou les polynucléotides sur la séquence d'ADN de l'isolat d'acide nucléique si elle est présente dans l'échantillon de tissu ou de cellules, la répétition de cette séquence autant de fois qu'on le souhaite pour amplifier les teneurs en isolat d'acide nucléique, et ensuite la détection des séquences d'isolat d'acide nucléique dans l'échantillon amplifié, par :
(a) immobilisation de l'ADN amplifié sur une matrice support, hybridation de l'ADN immobilisé avec un isolat d'acide nucléique marqué conforme à la revendication 3 ou à la revendication 4 dans des conditions permettant à l'isolat d'acide nucléique de se fixer à des séquences complémentaires, lavage de l'isolat d'acide nucléique non fixé de la matrice support, et ensuite détection de la fixation de l'isolat d'acide nucléique à l'ADN lié à la matrice support ; ou par
(b) lorsque les précurseurs de nucléotides marqués sont inclus lors de l'incubation avec la polymérase d'ADN, fractionnement de l'échantillon par électrophorèse dans une matrice de gel, et détection de séquences d'ADN d'isolat d'acide nucléique marqué.

9. Procédé tel que revendiqué dans l'une quelconque des revendications 6 à 8, dans lequel l'échantillon de tissu ou de cellules est obtenu à partir d'un embryon, d'un foetus, ou à partir de sperme ou d'une fraction de sperme.

10. Nécessaire pour la détection de la présence ou de l'absence de séquences spécifiques du chromosome Y dans un échantillon de tissu ou de cellules, comprenant un isolat d'acide nucléique conforme à l'une quelconque des revendications 1 à 4.

11. Nécessaire tel que revendiqué dans la revendication 10, contenant en outre des tampons pour la dilution des réactifs.

12. Oligomère pour la détermination du sexe par RCP, de bovins, de moutons et de chèvres, comprenant la séquence 5'-CCCTTCCAGCTGCAGTGTCA-3'.

13. Oligomère pour la détermination du sexe par RCP, de bovins, de moutons et de chèvres, comprenant la séquence 5'-GATCTGTAACTGCAAACCTGGC-3'.
